# FASCICULE DE BREVET EUROPEEN

(11) **EP 1 315 709 B1**
(45) Date de publication et mention de la délivrance du brevet: **26.05.2010**
(21) Numéro de dépôt: 01963113.4
(22) Date de dépôt: 21.08.2001
(51) Int. Cl.: C07D 307/84, C07D 307/80, C07D 407/10, C07D 409/06, C07D 407/04, C07D 471/04, A61K 31/34

(54) **AMINOALKYKBENZOYL-BENZOFURANES OU BENZOTHIOPHENES, LEUR PROCEDE DE PREPARATION ET LES COMPOSITIONS LES CONTENANT**
AMINOALKYKBENZOYL-BENZOFURANE ODER BENZOTHIOPHENE, VERFAHREN ZU IHRER HERSTELLUNG UND DIE ENTHALTENDE ZUSAMMENSETZUNGEN
AMINOALKYLBENZOYL-BENZOFURAN OR BENZOTHIOPHENE DERIVATIVES, METHOD FOR PREPARING SAME AND COMPOSITIONS CONTAINING SAME

(30) Priorité: 23.08.2000 FR 0010834
(43) Date de publication de la demande: 04.06.2003
(73) Titulaire: Sanofi-Aventis, 75013 Paris (FR)
(72) Inventeur: ASSENS, Jean-Louis, F-34790 Grabels (FR); BERNHART, Claude, F-34980 Saint-Gely-du-Fesc (FR); CABANEL-HAUDRICOURT, Frédérique, F-34570 Pignan (FR); GAUTIER, Patrick, 34660 Cournonterral (FR); NISATO, Dino, F-34680 Saint Georges d'Orques (FR)
(74) Mandataire: Weber, Mathieu
(86) Numéro de dépôt international: PCT/FR2001/002640
(87) Numéro de publication internationale: WO 2002/016339

(56) Documents cités:
- EP-A- 0 617 030
- EP-A- 0 835 871
- WO-A-90/02743
- WO-A-94/29289
- WO-A-95/10513
- US-A- 3 248 401
- US-A- 4 806 663
- MARTIN M J ET AL: "Versatile Raloxifene Triflates" BIOORGANIC & MEDICINAL CHEMISTRY LETTERS, OXFORD, GB, vol. 7, no. 7, 8 avril 1997 (1997-04-08), pages 887-892, XP004136150 ISSN: 0960-894X

## Description

La présente invention se rapporte, d'une manière générale, à de nouveaux dérivés hétérocycliques ainsi qu'à leur procédé de préparation.

La demande de brevet WO 9510513 décrit des composés benzothiophènes possédant des propriétés agonistes des oestrogènes.

La demande de brevet WIO 9429289 concerne des dérivés benzoylbenzofuranes pour le traitement de l'arythmie cardiaque. Ces composés comportent une fonction ester ou amide en position 2 du bicycle et une fonction éther directement sur le groupe phényle de la chaîne en position 3 du bicycle.

Le brevet EP 0835871 décrit des composés benzothiophènes pour le traitement de la perte osseuse, de l'hyperlipidémie et des cancers estrogène-dépendants. Ces composés comportent un groupe phényle substitué en position 2 du bicycle, une fonction éther directement sur le groupe phényle de la chaîne en position 3 du bicycle et un substituant en position 6.

La publication de Martin et col. (Bioorganic & Medecinal Chemistry Letters, Vol. 7, N°7, pages 887-892) concerne des dérivés benzothiophènes du raloxifène pour le traitement de la perte osseuse, de la dépression, de la schizophrénie, de maladies cardiovasculaires, de la maladie d'Alzheimer. Ces composés comportent un groupe phényle en position 2 du bicycle, une fonction éther directement sur le groupe phényle de la chaîne en position 3 du bicycle et un substituant en position 6.

L'invention concerne ainsi des nouveaux dérivés de benzofurane ou de benzothiophène de formule générale : 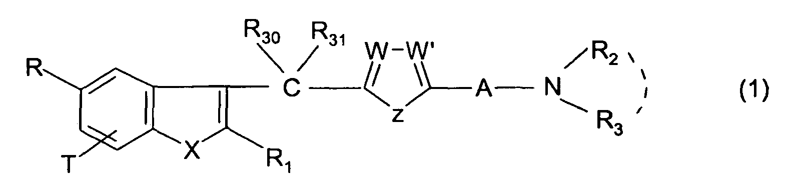 ainsi que leurs sels pharmaceutiquement acceptables, dans laquelle :
A représente un groupement alkylène, linéaire ou ramifié, en C₁-C₅, éventuellement substitué par un groupement hydroxyle, ou A représente un groupement de formule générale :

   -R₁₉-O-R₂₀- (h)

   dans laquelle R₁₉ et R₂₀, identiques ou différents, représentent chacun un groupement alkylène en C₁-C₄, linéaire ou ramifié,
R₃₀ et R₃₁, pris ensemble, représentent un groupement carbonyle avec le carbone sur lequel ils sont fixés, ou représentent un groupement de formule générale :

   -O-R₂₉-O- (m)

   dans laquelle R₂₉ représente un groupement alkylène en C₁-C₄,
T représente l'hydrogène ou un radical alkyle en C₁-C₄,
R représente :
   - le groupement cyano, formyle ou tétrazolyle,
   - un groupement ester de formule générale: 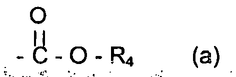 dans laquelle R₄ représente un groupement alkyle en C₁-C₆ , linéaire ou ramifié, ou cycloalkyle en C₃-C₆,
   - un groupement carboxyle de formule générale : 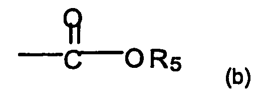 dans laquelle R₅ représente l'hydrogène ou un atome de métal alcalin,
   - un groupement amide de formule générale : 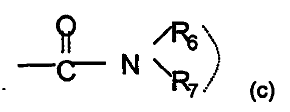 dans laquelle R₆ et R₇, identiques ou différents, représentent l'hydrogène ou un radical alkyle, linéaire ou ramifié, en C₁-C₄ ou R₆ et R₇, lorsqu'ils sont pris ensemble, représentent une chaîne alkylène en C₂-C₆,
   - un groupement cétone de formule générale : 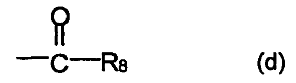 dans laquelle R₈ représente un groupement alkyle en C₁-C₄,
   - un groupement oxime de formule générale :

      -CH=N-OR₉ (e)

      dans laquelle R₉ représente l'hydrogène ou un radical alkyle en C₁-C₄,
   - un groupement carboxyle de formule générale : 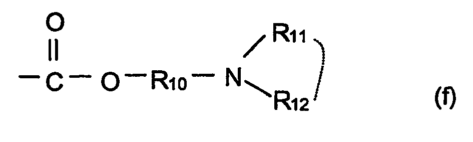 dans laquelle R₁₀ représente un groupement alkylène, linéaire ou ramifié, en C₁-C₄, R₁₁ représente l'hydrogène ou un radical alkyle en C₁-C₄, R₁₂ représente un radical alkyle en C₁-C₄ ou R₁₁ et R₁₂, lorsqu'ils sont pris ensemble, représentent une chaîne alkylène en C₂-C₆,
   - un groupement de formule générale : 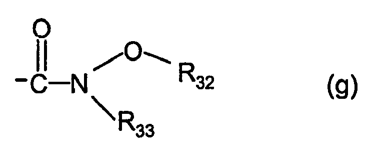 dans laquelle R₃₂ et R₃₃, identiques ou différents, représentent un groupement alkyle en C₁-C₄, linéaire ou ramifié,
R₁ représente un groupement alkyle en C₁-C₆ linéaire ou ramifié ou R₁ représente un groupement de formule générale :

   -R₂₃-OH (j)

   dans laquelle R₂₃ représente un groupement alkylène en C₁-C₆, linéaire ou ramifié,
R₂ et R₃, identiques ou différents, représentent l'hydrogène, un groupement alkyle linéaire ou ramifié en C₁-C₆ , éventuellement substitué par un ou plusieurs atomes d'halogène ou par un groupement pyrrolidinyle, un groupement cycloalkyle en C₃-C₆, un groupement de formule générale :

   -R₂₄-O-R₂₅ (k)

   dans laquelle R₂₄ représente un groupement alkylène en C₁-C₄, linéaire ou ramifié, et R₂₅ représente un groupement alkyle en C₁-C₄, linéaire ou ramifié,
   ou R₂ et R₃, lorsqu'ils sont pris ensemble, représentent un groupement alkylène en C₃-C₁₀, linéaire ou ramifié, ou représentent avec l'atome d'azote sur lequel ils sont fixés un groupement de formule générale : 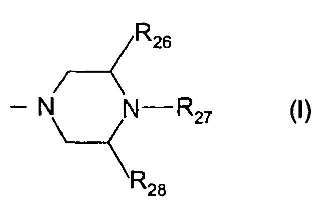 dans laquelle :
   - R₂₆, R₂₇, et R₂₈, identiques ou différents, représentent l'hydrogène ou un groupement alkyle en C₁-C₄, linéaire ou ramifié, ou
   - R₂₆ représente l'hydrogène ou un groupement alkyle en C₁-C₄, linéaire ou ramifié, et R₂₇ et R₂₈ , lorsqu'ils sont pris ensemble, représentent un groupement alkylène en C₁-C₄, linéaire ou ramifié,
W, W' et Z sont tels que :
   - lorsque W et W', identiques représentent CH, Z représente -O- ou -S-
   - lorsque W représente CH et W' représente C-R₁₃, Z représente
   - CH=C(R₁₄)-, R₁₃ et R₁₄ étant identiques ou différents et représentant l'hydrogène, un atome d'halogène, un radical alkyle en C₁-C₄, ou un radical alkoxy en C₁-C₄,
X représente -O- ou -S-,
ces dérivés de benzofurane ou de benzothiophène étant sous forme d'isomères optiques individuels ou de mélanges de ceux-ci.

En particulier, les dérivés de benzofurane ou de benzothiophène selon l'invention sont **caractérisés en ce que** A représente un groupement alkylène, linéaire ou ramifié, en C₁-C₅ éventuellement substitué par un groupement hydroxyle.

Des classes de composés préférés de l'invention peuvent être représentés par les composés de formule (1):
- dans laquelle R représente un groupement isopropoxycarbonyle
- ou dans laquelle R₁ et/ou R₂ et/ou R₃ représentent le groupement n-butyle
- ou dans laquelle X représente - O -.

Une autre classe de composés préférés de formule (1) est celle dans laquelle, lorsque R₃₀ et R₃₁ pris ensemble représentent un groupement carbonyle avec le carbone sur lequel ils sont fixés, : 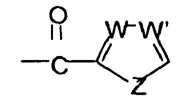 représente le radical benzoyle.

Enfin, les composés de formule (1) dans laquelle R₁ représenté n-butyle, A représente le groupement propylène et R₂ et R₃, identiques, représentent le groupement n-butyle peuvent être considérés comme préférés.

Des composés de formule (1) peuvent se présenter sous forme d'isomères optiques ou géométriques, par exemple les composés en question dans lesquels R₂ et R₃, pris avec l'atome d'azote auxquels ils sont attachés, représentent un groupement diéthylpipéridino ou dans lesquels R représente un groupement (e).

En conséquence, l'invention se rapporte à la fois aux isomères individuels des composés de formule (1) ainsi qu'à leurs mélanges notamment le mélange racémique.

L'invention se rapporte également aux sels pharmaceutiquement acceptables des composés de formule (1) formés à partir d'un acide organique ou inorganique.

Comme exemples de sels organiques de ce genre, on peut citer les oxalate, maléate, fumarate, méthanesulfonate, benzoate, ascorbate, pamoate, succinate, hexamate, bisméthylènesalicylate, éthanedisulfonate, acétate, propionate, tartrate, salicylate, citrate, gluconate, lactate, malate, cinnamate, mandélate, citraconate, aspartate, palmitate, stéarate, itaconate, glycolate, p-aminobenzoate, glutamate, benzènesulfonate, p-toluènesulfonate et théophylline acétate ainsi que les sels formés à partir d'un acide aminé tel que le sel de lysine ou l'histidine.

Comme sels inorganiques de ce genre, on peut citer les chlorhydrate, bromhydrate, sulfate, sulfamate, phosphate et nitrate.

On a trouvé que les composés de l'invention possèdent de remarquables propriétés pharmacologiques notamment des propriétés anti-arythmiques puisqu'ils se sont révélés capables de supprimer ou de prévenir les troubles du rythme ventriculaire et auriculaire. La plupart des composés de l'invention ont des propriétés électrophysiologiques des classes 1,2,3 et 4 de la classification de Vaughan-Williams qui confèrent des propriétés bradycardisantes, anti-hypertensives et anti-adrénergiques α et β non compétitives. De plus, la plupart des composés ont également révélé des propriétés antioxydants, une affinité pour les récepteurs sigma et une capacité à augmenter la synthèse du NO.

Par ailleurs, ces composés de l'invention manifestent des propriétés inhibitrices de différents agents hormonaux tels que par exemple l'angiotensine II, l'arginine vasopressine, le neuropeptide Y ou l'endothéline.

Ces propriétés sont capables de rendre les composés en question très utiles dans le traitement de certains syndromes pathologiques du système cardio-vasculaire en particulier dans le traitement de l'angine de poitrine, de l'hypertension, de l'arythmie en particulier atriale, ventriculaire ou supraventriculaire, de l'insuffisance circulatoire cérébrale. De même, les composés de l'invention pourront être utilisés dans le traitement de l'insuffisance cardiaque, de l'infarctus du myocarde compliqué ou non d'insuffisance cardiaque ou pour la prévention de la mortalité post-infarctus.

Dans le domaine antitumoral, les composés de l'invention pourront être utiles comme potentialisateurs d'anticancéreux. En conséquence, l'invention se rapporte également à un médicament, **caractérisé en ce qu**'il comprend un composé dérivé de benzofurane ou de benzothiophène, ou un sel pharmaceutiquement acceptable de ce dernier, selon l'invention.

En conséquence, l'invention se rapporte également à des compositions pharmaceutiques ou vétérinaires contenant comme principe actif, au moins un composé de l'invention, en association avec un véhicule pharmaceutique ou excipient approprié.

Selon la voie d'administration choisie, la posologie journalière pour un être humain pesant 60 kg se situera entre 2 et 2000 mg de principe actif, en particulier entre 50 et 500 mg de principe actif.

Les composés de formule (1) peuvent être préparés selon les méthodes suivantes :
**A.** - Dans le cas où R représente le groupement cyano, le groupement formyle, un groupement (a), un groupement (d), un groupement (e) ou un groupement (f), en faisant réagir un composé de formule générale : 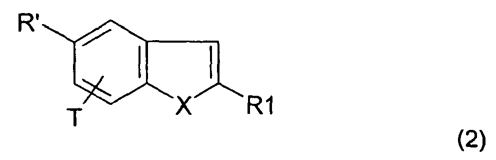 dans laquelle R' représente le groupement cyano, le groupement formyle, un groupement (a), un groupement (d), un groupement (e) ou un groupement (f) et R₁, T et X ont la même signification que précédemment, avec un halogénure de formule générale : 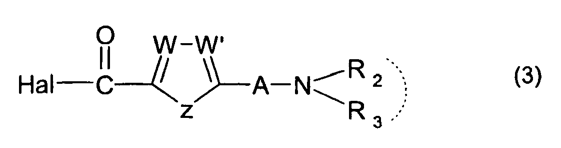 dans laquelle A, R₂, R₃, W, W' et Z ont la même signification que précédemment et Hal représente un atome d'halogène tel que par exemple chlore ou brome, la réaction ayant lieu en présence d'un acide de Lewis tel que le chlorure d'aluminium, le chlorure stannique, le chlorure ferrique ou le trifluorométhanesulfonate d'argent, ce qui fournit les composés désirés de formule (1) sous forme de base libre.
   Habituellement, la réaction ci-dessus se déroule dans un solvant apolaire tel qu'un composé halogéné, par exemple le dichlorométhane ou le dichloroéthane et à une température comprise entre 5°C et la température de reflux.
**B.** - Dans le cas où R représente un groupement (b), en saponifiant en présence d'un agent basique à savoir un hydroxyde de métal alcalin, par exemple l'hydroxyde de sodium, un composé de formule (1) ci-dessus dans laquelle R représente un groupement (a) c'est-à-dire un composé de formule générale: 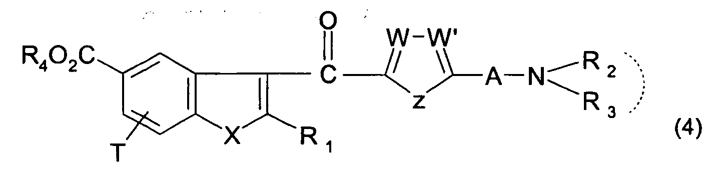 dans laquelle A, R₁, R₂, R₃, R₄, T, W, W', X et Z ont la même signification que précédemment, ce qui fournit, sous forme de base libre, les composés de formule (1) dans laquelle R₅ représente un atome de métal alcalin, composés que l'on traite, si nécessaire, avec un acide fort, par exemple l'acide chlorhydrique, ce qui fournit, sous forme de base libre, les composés désirés de formule (1) dans laquelle R₅ représente l'hydrogène.
**C.** - Dans le cas où R représente le groupement hydroxyméthyle, en déprotégeant un cétal de formule générale : 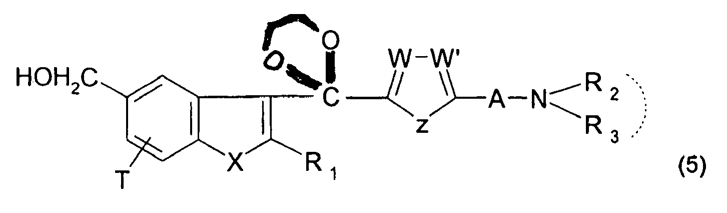 dans laquelle A, R₁, R₂, R₃, T, X, W, W' et Z ont la même signification que précédemment et ce, au moyen de pyridine p-toluènesulfonate et de préférence à la température de reflux, ce qui fournit les composés désirés de formule (1) sous forme de base libre.
   Alternativement, des composés de formule (1) peuvent être obtenus par mise en oeuvre des procédés suivants :
**D.** - Dans le cas où R représente le groupement cyano, le groupement formyle, un groupement (a), un groupement (d), un groupement (e) ou un groupement (f), et R₂ et R₃, qui sont identiques, représentent chacun l'hydrogène, en traitant, avec la triphénylphosphine, un azide de formule générale : 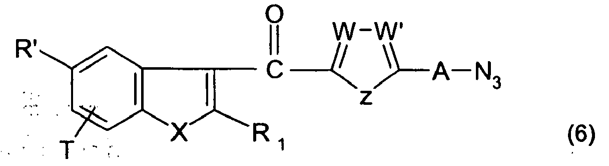 dans laquelle A, R', R₁, T, W, W', X et Z ont la même signification que précédemment, pour former les composés dérivés de formule (1) sous forme de base libre.
**E**. - Dans le cas où R représente le groupement cyano, le groupement formyle, un groupement (a), un groupement (d), un groupement (e) ou un groupement (f), et A représente un groupement alkylène, linéaire ou ramifié, en C₃-C₅ ou un groupement (h), en faisant réagir un composé cétonique de formule générale : 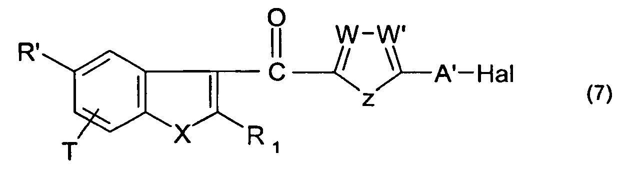 dans laquelle A' représente un groupement alkylène, linéaire ou ramifié, en C₃-C₅, et Hal, R', R₁, T, W, W', X et Z ont la même signification que précédemment, avec un composé de formule générale : 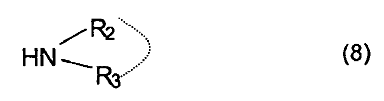 dans laquelle R₂ et R₃ ont la même signification que précédemment, la réaction ayant lieu en présence d'un agent basique tel qu'un carbonate ou hydroxyde de métal alcalin ou un excès de composé de formule (8) sous forme basique, ce qui fournit les composés désirés de formule (1) sous forme de base libre.
   Généralement, la réaction se déroule à une température comprise entre la température ambiante et la température de reflux du solvant et dans un solvant polaire tel que le N,N-diméthylformamide, l'acétonitrile, la méthyléthylcétone ou le diméthylsulfoxyde ou un solvant apolaire tel que le benzène ou le toluène.
   D'autre part, cette réaction est habituellement conduite en présence d'un catalyseur de préférence l'iodure de sodium ou de potassium.
   Enfin, dans la variante où A représente un groupement alkylène en C₃-C₅ substitué par un groupe hydroxyle, on fait réagir à la place du composé (7) un composé de formule générale : 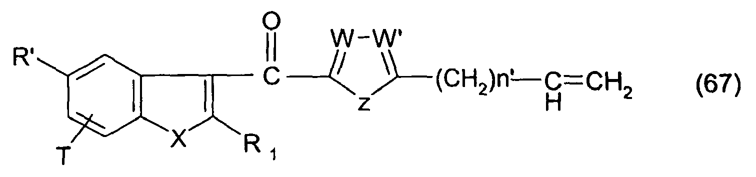 dans laquelle n' vaut 0 ou est un entier compris entre 1 et 3, avec de l'acide métachloroperbenzoïque pour obtenir l'époxyde de formule générale : 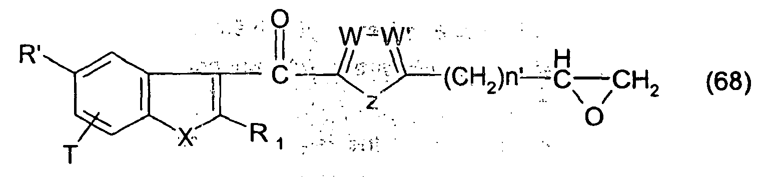 que l'on fait ensuite réagir avec une amine de formule (8) comme décrit ci-dessus pour le composé (7). On obtient ainsi le composé de formule : 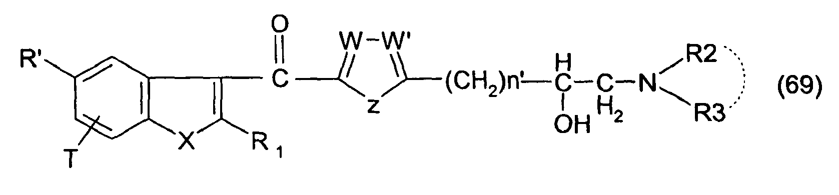
**F.** - Dans le cas où R représente le groupement cyano, le groupement formyle, un groupement (a), un groupement (d), un groupement (e) ou un groupement (f), R₂ représente l'hydrogène ou un groupement alkyle, linéaire ou ramifié, en C₁-C₆ et R₃ représente un groupement alkyle, linéaire ou ramifié, en C₁-C₆, en faisant réagir une amine de formule générale : 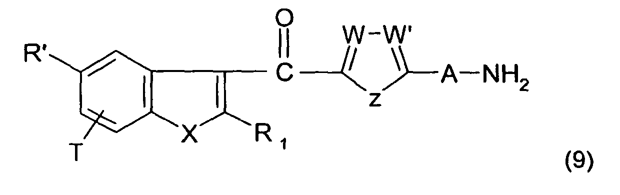 dans laquelle A, R', R₁, T, X, W, W' et Z ont la même signification que précédemment, avec une aldéhyde de formule générale : 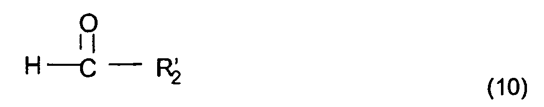 dans laquelle R'₂ représente l'hydrogène ou un radical alkyle en C₁-C₅ et ce, en présence de triacétoxyborohydrure de sodium, pour fournir les composés désirés de formule (1) sous forme de base libre.
   Les dérivés benzofuraniques ou benzothiophéniques de formule (1), qui répondent également à la formule générale : 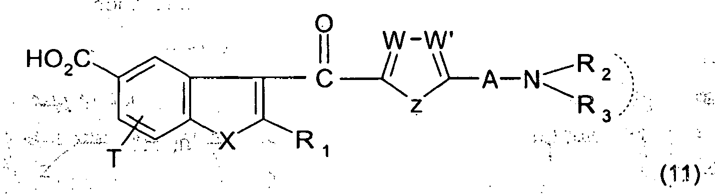 dans laquelle A, R₁, R₂, R₃, T, W, W', X et Z ont la même signification que précédemment, sont eux-mêmes intermédiaires de synthèse pour la préparation d'autres composés de formule (1).
   A cet effet, on peut mettre en oeuvre les méthodes suivantes au départ des composés de formule (11) en question pour obtenir les composés souhaités de formule (1), c'est-à-dire :
**G.** - Dans le cas où R représente un groupement (c), en faisant réagir un composé de formule (11) après protection de la fonction amine lorsque R₂ et/ou R₃ représentent l'hydrogène et ce, de préférence dans un hydrocarbure halogéné comme solvant et généralement à la température de reflux, avec un agent halogénant tel que le chlorure de thionyle, le phosgène ou le chlorure d'oxalyle pour obtenir un halogénure d'acyle que l'on traite ensuite, de préférence à la température ambiante, avec un composé de formule générale: 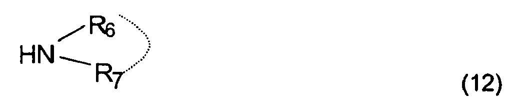 dans laquelle R₆ et R₇ ont la même signification que précédemment, puis on déprotège, si nécessaire, le composé formé, ce qui fournit les composés désirés de formule (1) sous forme de base libre.
**H.** - Dans le cas où R représente un groupement (a), en faisant réagir un composé de formule (11) après protection de la fonction amine lorsque R₂ et/ou R₃ représentent l'hydrogène et ce, de préférence dans un hydrocarbure halogéné comme solvant et généralement à la température de reflux, avec un agent halogénant tel que le chlorure de thionyle, le phosgène ou le chlorure d'oxalyle pour obtenir un halogénure d'acyle que l'on traite ensuite, de préférence à une température comprise entre la température ambiante et la température de reflux, avec un alcool de formule générale :

   R₄-OH (13)

   dans laquelle R₄ a la même signification que précédemment, puis on déprotège, si nécessaire, le composé formé, ce qui fournit les composés désirés de formule (1) sous forme de base libre.
**I.**- Dans le cas où R représente un groupement (f) :
   a) si ce groupement (f) est du type dialkylaminoalkyle primaire, on fait réagir, de préférence dans un solvant polaire tel que le N,N-diméthylformamide et habituellement à une température comprise entre 30 et 50°C, un composé de formule (11) après protection de la fonction amine lorsque R₂ et/ou R₃ représentent l'hydrogène et ce, avec un alcool de formule générale: 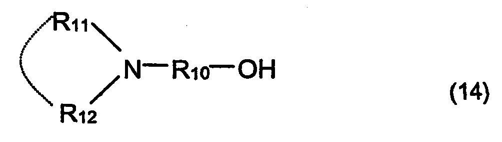 dans laquelle R₁₁ et R₁₂ ont la même signification que précédemment et R₁₀ représente un groupement alkylène linéaire en C₁-C₄, la réaction ayant lieu en présence de carbonyl diimidazole et de 1,8-diazabicyclo[5.4.0]undec-7ène, puis on déprotège, si nécessaire, le composé formé, ce qui fournit, sous forme de base libre, les composés désirés de formule (1),
   b) si ce groupement (f) est du type dialkylaminoalkyle secondaire ou tertiaire, on fait réagir, de préférence dans un solvant aprotique tel qu'un hydrocarbure halogéné et généralement à la température de reflux du milieu, un composé de formule (11) après protection de la fonction amine lorsque R₂ et/ou R₃ représentent l'hydrogène et ce, avec un agent halogénant tel que le chlorure de thionyle, pour obtenir un halogénure d'acyle que l'on traite ensuite, de préférence à la température ambiante, avec un alcool de formule (14) ci-dessus dans laquelle R₁₁ et R₁₂ ont la même signification que précédemment et R₁₀ représente un groupement alkylène secondaire ou tertiaire en C₂-C₄, puis on déprotège, si nécessaire, le composé formé, ce qui fournit les composés de formule (1) sous forme d'halohydrate ou sous forme de base libre, halohydrate que l'on peut traiter, si nécessaire, avec un agent basique tel qu'un hydroxyde de métal alcalin ou un carbonate de métal alcalin, pour obtenir les composés désirés sous forme de base libre.
      Dans les procédés G, H et I ci-dessus, la protection de la fonction amine du composé de formule (10), c'est-à-dire la protection envisagée lorsque R₂ et/ou R₃ représentent l'hydrogène, peut être obtenue par exemple par traitement au moyen d'un composé permettant la fixation d'un groupement aisément éliminable notamment au moyen de 9-fluorenylméthylchloroformiate et la déprotection s'opère par la suite par traitement avec une amine secondaire, par exemple la pipéridine ou la diéthylamine et ce, dans un solvant approprié par exemple le N,N-diméthylformamide.
      I-1. Dans le cas où R représente un groupement (g), en faisant réagir un composé de formule générale : 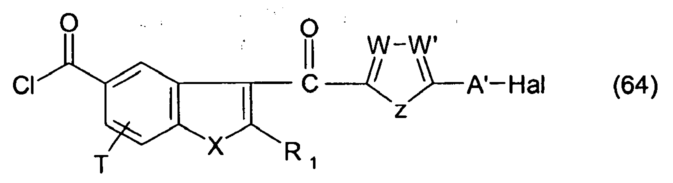 dans laquelle A', Hal, R₁, T, W, W', X et Z ont la même signification que précédemment, avec un composé de formule générale :

         R₃₃-NHO-R₃₂ (65)

         pour obtenir le composé de formule générale : 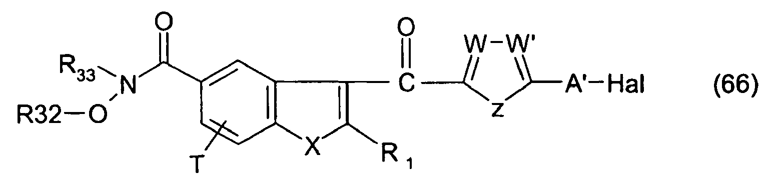 que l'on fait ensuite réagir avec une amine de formule (8) comme décrit ci-dessus pour le composé (7).

   D'autres composés de formule (1) peuvent être utilisés comme intermédiaires de synthèse de composés de l'invention notamment les dérivés cyano qui répondent également à la formule générale : 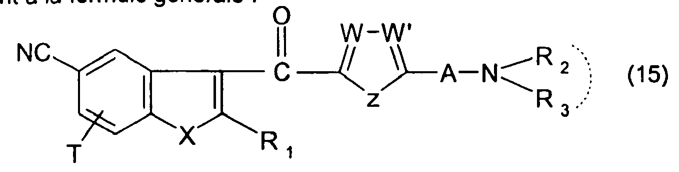 dans laquelle A, R₁, R₂, R₃, T, W, W', X et Z ont la même signification que précédemment.
   Ainsi, on peut mettre en oeuvre les méthodes suivantes, c'est-à-dire :
**J.** - Dans le cas où R représente un groupement (c) dans lequel R₆ et R₇ représentent chacun l'hydrogène, on hydrolyse un composé de formule (15) en présence d'un acide fort tel que par exemple l'acide sulfurique et généralement à la température ambiante, ce qui fournit, sous forme de base libre, les composés désirés de formule (1).
**K.** - Dans le cas où R représente le groupement tétrazolyle, on fait réagir de préférence dans un solvant aprotique tel qu'un hydrocarbure aromatique, par exemple le benzène ou le toluène et habituellement à la température de reflux du milieu, un composé de formule (15) avec un (trialkyl en C₁-C₄) azido étain par exemple le tributylazido étain, ce qui fournit, sous forme de base libre, les composés désirés de formule (1).
   D'une autre manière, on peut obtenir les composés de formule (1) dans laquelle R représente le groupement cyano, le groupement formyle, un groupement (a), un groupement (d), un groupement (e) ou un groupement (f), et dans laquelle R₂ et R₃, qui sont différents, représentent chacun un groupement alkyle, linéaire ou ramifié, en C₁-C₆ ou un groupement cycloalkyle en C₃-C₆, en transformant une amine secondaire comportant un groupement -NHR₃ dans lequel R₃ est différent d'hydrogène en amine tertiaire, par réaction d'un composé de formule générale : 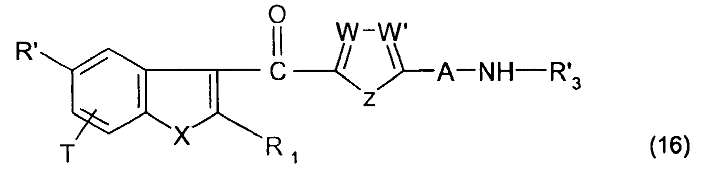 dans laquelle A, R₁, R', T, X, W, W' et Z ont la même signification que précédemment et R'₃ représente un groupement alkyle, linéaire ou ramifié, en C₁- C₆ ou cycloalkyle en C₃-C₆, avec un halogénure de formule générale :

   Hal - R"₂ (17)

   dans laquelle Hal représente un atome d'halogène, de préférence brome et R"₂ représente un groupement alkyle, linéaire ou ramifié, en C₁-C₆ ou un groupement cycloalkyle en C₃-C₆, la réaction ayant lieu en présence d'un agent basique tel qu'un carbonate ou hydroxyde de métal alcalin et de préférence à la température de reflux, ce qui fournit les composés désirés de formule (1) sous forme de base libre.
   Les méthodes décrites précédemment permettent d'obtenir les composés de formule (1) sous forme de mélanges d'isomères lorsqu'un ou plusieurs carbones asymétriques sont présents.
   Toutefois, ces isomères peuvent être produits sous forme séparée par mise en oeuvre de méthodes connues telles que par exemple chromatographie ou précipitation.
**L.** - Les composés de formule (1) obtenus sous forme de base libre selon l'une ou l'autre des méthodes décrites ci-dessus, peuvent ensuite être transformés, si nécessaire, en sels pharmaceutiquement acceptables par réaction avec un acide organique ou inorganique approprié par exemple l'acide oxalique, maléique, fumarique, méthanesulfonique, benzoïque, ascorbique, pamoïque, succinique, hexamique, bisméthylènesalicylique, éthanedisulfonique, acétique, propionique, tartrique, salicylique, citrique, gluconique, lactique, malique, cinnamique, mandélique, citraconique, aspartique, palmitique, stéarique, itaconique, glycolique, p-aminobenzoïque, glutamique, benzènesulfonique, p-toluènesulfonique, théophylline acétique, la lysine ou l'histidine ou encore l'acide chlorhydrique, bromhydrique, sulfurique, sulfamique, phosphorique ou nitrique.

Les composés de formule (2) peuvent être obtenus selon différentes méthodes en relation avec leur structure chimique et plus particulièrement en relation avec la position du groupement R'.
**A.** - Les composés de formule (2) dans laquelle R' est situé en position 5 et représente le groupement cyano, un groupement (a) ou un groupement (f), et R₁ est situé en position 2 peuvent être préparés selon la suite d'étapes ci-après :
   a) **soit,** on traite un dérivé cyano de formule générale : 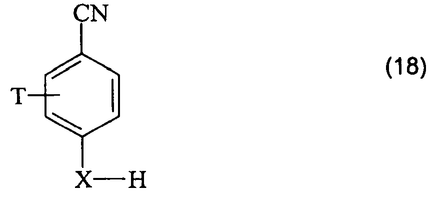 dans laquelle T et X ont la même signification que précédemment, avec l'iode en présence d'ammoniaque, pour former un dérivé iodo de formule générale : 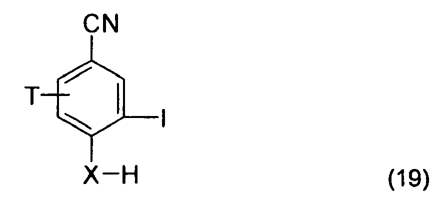 dans laquelle T et X ont la même signification que précédemment,
      **soit,** on traite un dérivé d'acide benzoïque de formule générale : 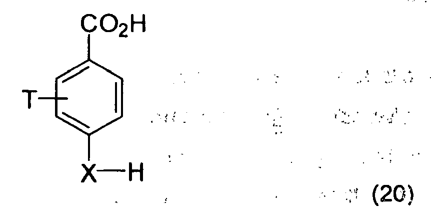 dans laquelle T et X ont la même signification que précédemment, d'abord avec un iodure de métal alcalin et un agent oxydant tel qu'un hypochlorite de métal alcalin, par exemple l'hypochlorite de sodium, ensuite avec un alcool de formule générale :

      R₄-OH (21)

      dans laquelle R₄ a la même signification que précédemment, ce qui fournit un dérivé iodo de formule générale : 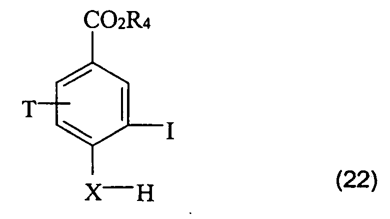 dans laquelle R₄, T et X ont la même signification que précédemment,
   b) on fait réagir le dérivé iodé de formule (19) ou (22) avec un dérivé acétylénique de formule générale :

      HC≡C-R₁ (23)

      dans laquelle R₁ a la même signification que précédemment et ce, en présence d'un catalyseur approprié tel qu'un dérivé de palladium, par exemple le tétrakis (triphénylphosphine)palladium et d'iodure cuivreux, ce qui fournit les composés désirés de formule (2).

   Si nécessaire, la préparation de l'ester de formule (22) peut être réalisée selon la méthode décrite ci-dessus mais au départ d'un acide de formule (20) sous forme d'halogénure d'acyle obtenu après traitement du dérivé d'acide benzoïque de formule (20) au moyen d'un agent halogénant, par exemple le chlorure de thionyle, le phosgène ou le chlorure d'oxalyle.
**B.** - Les composés de formule (2) dans laquelle R' est situé en position 6 et représente le groupement cyano ou un groupement (a) ou un groupement (f), et R₁ est situé en position 2 peuvent être obtenus comme suit :
   a) on fait réagir un composé de formule générale : 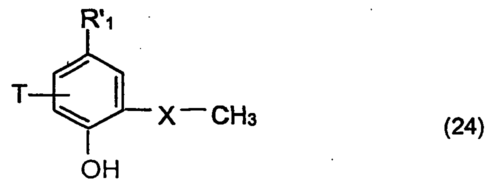 dans laquelle T et X ont la même signification que précédemment et R'₁ représente le groupement cyano, un groupement (a) ou un groupement (f), avec l'anhydride de l'acide trifluorométhanesulfonique en présence de pyridine, pour obtenir un composé de formule générale: 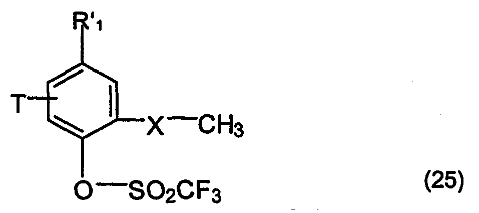 dans laquelle R'₁, T et X ont la même signification que précédemment,
   b) on fait réagir le composé ainsi formé avec un dérivé acétylénique de formule (22) et ce, en présence d'un catalyseur approprié, par exemple un dérivé de palladium tel que le dichloro bis-(triphénylphosphine)palladium et d'un accepteur d'acide tel que la triéthylamine, pour former les composés de formule générale : 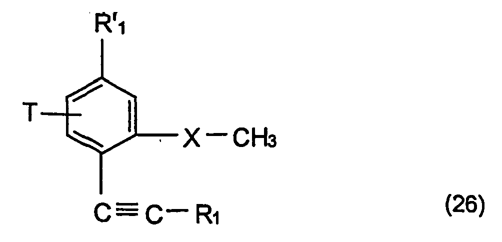 dans laquelle R'₁, R₁, T et X ont la même signification que précédemment,
   c) on cyclise alors ce composé de formule (26) en présence de tribromure de bore à une température inférieure à -50°C, ce qui fournit les composés hétérocycliques de formule générale : 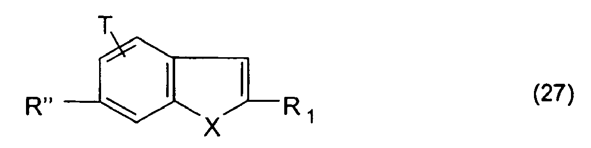 dans laquelle R₁, T et X ont la même signification que précédemment et R" représente le groupement cyano ou carboxylique, ce qui fournit soit des composés désirés de formule (2) lorsque R" représente le groupement cyano soit un acide lorsque R" représente le groupement carboxylique,
   d) on estérifie cet acide avec un alcool de formule (21) ou de formule (14), ce qui fournit des composés désirés de formule (2).
**C.** - Les composés de formule (2) dans laquelle R' est situé en position 4 et représente le groupement cyano, un groupement (a) ou un groupement (f), et R₁ est situé en position 2 peuvent être obtenus comme suit :
   a) on fait réagir un composé de formule générale : 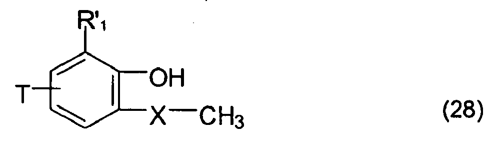 dans laquelle R'₁, T et X ont la même signification que précédemment, avec l'acide trifluorométhanesulfonique en présence de pyridine, pour obtenir un composé de formule générale : 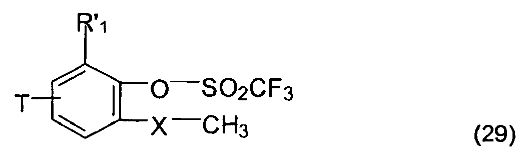 dans laquelle R'₁, T et X ont la même signification que précédemment,
   b) on fait réagir le composé ainsi formé avec un dérivé acétylénique de formule (23) et ce, en présence d'un catalyseur approprié tel qu'un dérivé de palladium par exemple le dichloro bis-(triphénylphosphine)palladium et d'un accepteur d'acide tel que la triéthylamine, pour former les composés de formule générale : 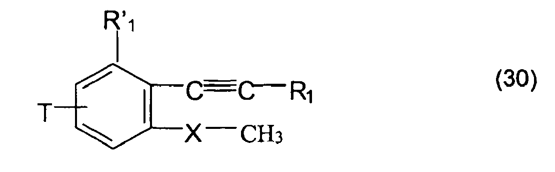 dans laquelle R'₁, R₁ et X ont la même signification que précédemment,
   c) on cyclise alors ce composé de formule (30) en présence de tribromure de bore, ce qui fournit les composés hétérocycliques de formule générale : 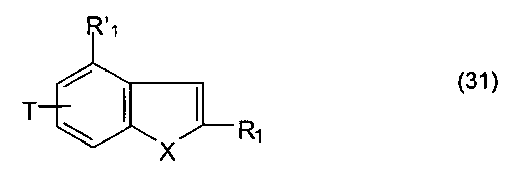
dans laquelle R'₁, R₁, T et X ont la même signification que précédemment qui correspondent aux composés désirés de formule (2).

De manière alternative, les composés de formule (2) dans laquelle R' est situé en position 5 et représente le groupement cyano, un groupement (a) ou un groupement (f), et R₁ est en position 2 peuvent également être préparés comme suit :
I. Lorsque R' représente un groupement (a) :
   a) on traite d'abord un benzoate de formule générale : 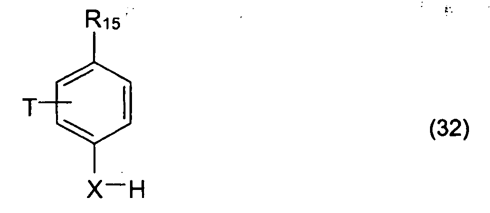 dans laquelle T et X ont la même signification que précédemment, et R₁₅ représente un groupement (a) ou (f), avec l'acide méthanesulfonique en présence de pentoxyde de phosphore et d'hexaméthylènetétramine, pour donner un dérivé formyle de formule générale : 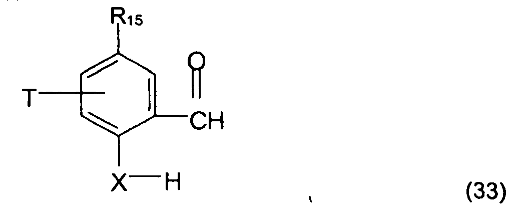 dans laquelle R₁₅, T et X ont la même signification que précédemment,
   b) on fait ensuite réagir ce composé de formule (33) avec un ester de formule générale : 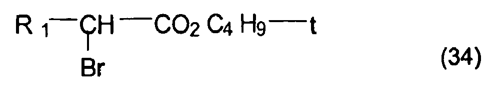 dans laquelle R₁ a la même signification que précédemment, ce qui fournit les composés de formule générale : 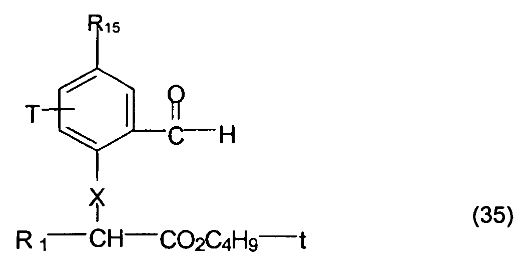 dans laquelle R₁, R₁₅, T et X ont la même signification que précédemment,
   c) on traite cet ester de formule (35) avec l'acide formique ou l'acide trifluoracétique, ce qui fournit les acides de formule générale : 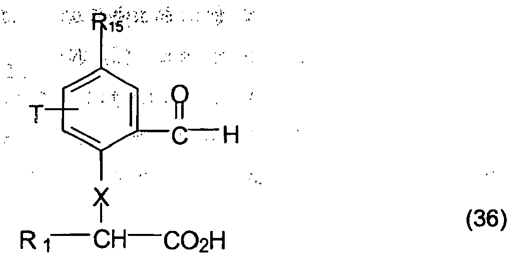 dans laquelle R₁, R₁₅, T et X ont la même signification que précédemment,
   d) on cyclise ce composé (36) en présence de chlorure de benzènesuflonyle
   ou de p-toluènsulfonyle et d'un accepteur d'acide tel que la triéthylamine, ce qui donne les composés souhaités de formule (2).
II. Lorsque R' représente le groupement cyano :
   a) on traite d'abord un dérivé formyle de formule générale : 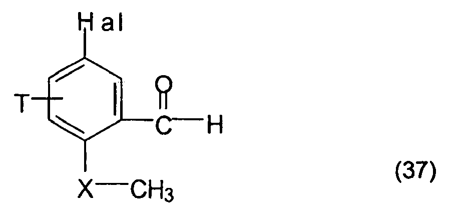 dans laquelle Hal, T et X ont la même signification que précédemment, avec le cyanure de zinc en présence d'un catalyseur approprié, par exemple un dérivé de palladium tel que le tétrakis-(triphénylphosphine)palladium, ce qui fournit les composés de formule générale : 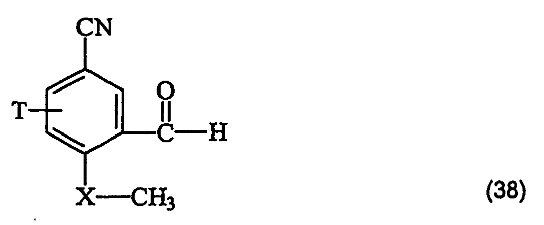 dans laquelle T et X ont la même signification que précédemment,
   b) on déméthyle ensuite ce composé de formule (38) avec le chlorure de lithium, ce qui fournit les composés de formule générale: 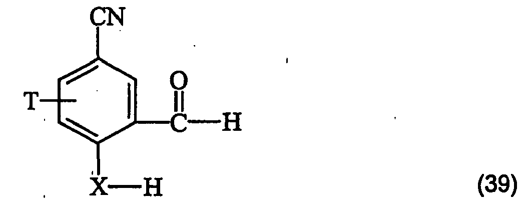 dans laquelle T et X ont la même signification que précédemment,
   c) on traite alors ce composé de formule (39) avec un ester de formule générale : 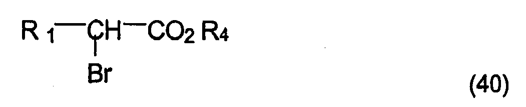 dans laquelle R₁ et R₄ ont la même signification que précédemment et ce, en présence.d'un agent basique tel qu'un carbonate de métal alcalin, ce qui donne les composés de formule générale : 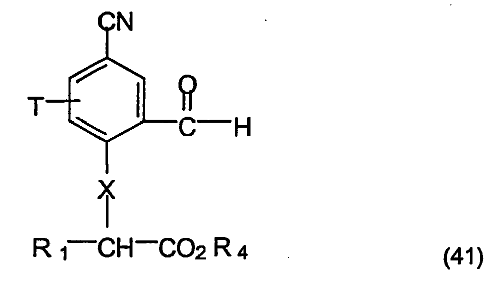 dans laquelle R₁, R₄, T et X ont la même signification que précédemment,
   d) et e) on saponifie cet ester de formule (41) en présence d'un agent basique tel qu'un hydroxyde de métal alcalin et l'on cyclise l'acide ainsi obtenu en présence de chlorure de benzènesulfonyle ou de p-toluènesulfonyle et d'un accepteur d'acide tel que la triéthylamine, ce qui fournit les composés souhaités.

Les composés de formule (2) dans laquelle R' est situé en position 7 et représente le groupement cyano, un groupement (a) ou un groupement (f), et R₁ est situé en position 2 peuvent être obtenus selon la suite d'étapes ci-après :
a) on traite un alcool de formule générale : 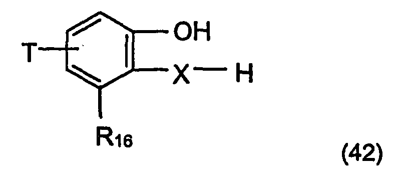 dans laquelle R₁₆ représente un groupement cyano ou formyle et T et X ont la même signification que précédemment et ce, avec l'iodure de méthyle en présence d'un hydrure de métal alcalin pour donner un composé de formule générale : 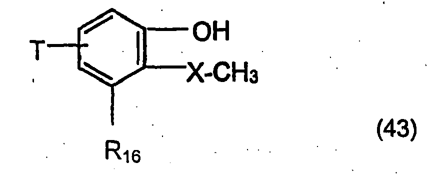 dans laquelle R₁₆, T et X ont la même signification que précédemment,
b) on fait réagir le composé ainsi formé avec l'anhydride trifluorométhanesulfonique pour former un composé de formule générale : 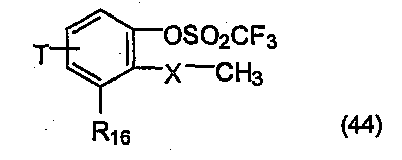 dans laquelle R₁₆, T et X ont la même signification que précédemment,
c) on traite le composé ainsi formé avec un composé de formule (23) en présence d'un catalyseur approprié tel qu'un dérivé de palladium, par exemple le dichloro bis-(triphénylphosphine)palladium, ce qui produit le composé de formule générale : 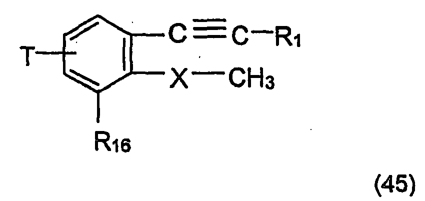 dans laquelle R₁, R₁₆, T et X ont la même signification que précédemment,
d) on fait ensuite réagir le composé de formule (45) ainsi formé :
   - lorsque R₁₆ représente le groupement cyano, avec le chlorure de lithium pour former les composés désirés de formule (2) dans laquelle R' représente le groupement cyano,
   - lorsque R₁₆ représente le groupement formyle, avec un cyanure de métal alcalin en présence d'oxyde manganeux et d'acide acétique pour donner un composé de formule générale : 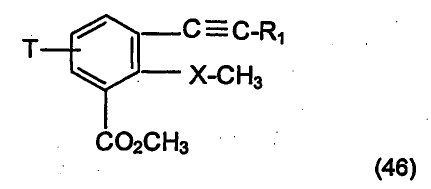 dans laquelle R₁, T et X ont la même signification que précédemment, que l'on cyclise avec le chlorure de lithium pour donner un mélange d'ester et d'acide de formule générale : 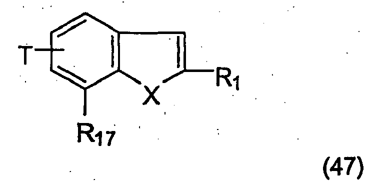 dans laquelle R₁, X et T ont la même signification que précédemment et R₁₇ représente le groupement méthoxycarbonyle ou carboxylique, mélange que l'on traite avec le méthanol en présence d'un acide fort tel que l'acide sulfurique, ce qui fournit les composés désirés de formule (2) dans laquelle R' représente le groupement méthoxycarbonyle.
      Les autres composés de formule (2), c'est-à-dire les composés de formule (2) dans laquelle R' situé en position 7 représente un groupement (a), à l'exception du groupement méthoxycarbonyle, ou un groupement (f), peuvent être obtenus en saponifiant un ester de formule (2) dans laquelle R' situé en position 7 représente le groupement méthoxycarbonyle et ce, en présence d'un agent basique tel qu'un hydroxyde de métal alcalin pour donner un sel que l'on acidifie avec un acide fort tel que l'acide chlorhydrique pour donner un dérivé de 7-carboxy-benzofurane que l'on estérifie avec un alcool de formule (14) ou un alcool de formule générale :

      R'₄-OH (48)

      dans laquelle R'₄ représente un groupement alkyle en C₂-C₆, ou cycloalkyle en C₃-C₆, ce qui fournit des composés désirés de formule (2).
      Les composés de formule (2) dans laquelle R' représente le groupement formyle peuvent être préparés en oxydant avec le chlorure d'oxalyle, un alcool de formule générale : 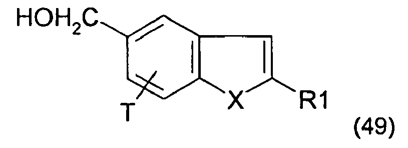 dans laquelle R₁, X et T ont la même signification que précédemment, pour fournir les composés désirés.
      Les composés de formule (2) dans laquelle R' représente un groupement oxime de formule (e) peuvent être obtenus en traitant une aldéhyde de formule générale : 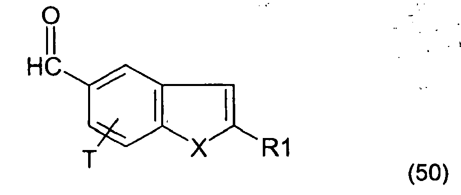 dans laquelle R₁, X et T ont la même signification que précédemment, avec un composé de formule générale:

      R₉-O-NH₂ (51)

      dans laquelle R₉ a la même signification que précédemment, éventuellement sous forme de ses sels, et ce, dans un solvant capteur d'acide, par exemple la pyridine, pour former les composés désirés.

Les composés de formule (2) dans laquelle R' représente un groupement cétone de formule (d) peuvent être obtenus en traitant un acide de formule générale : 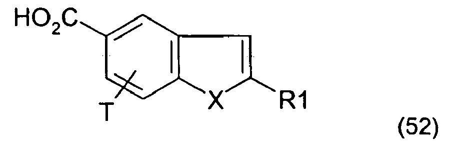 dans laquelle R₁, T et X ont la même signification que précédemment, avec un agent halogénant tel que le chlorure de thionyle ou le chlorure d'oxalyle puis en faisant réagir l'halogénure d'acyle ainsi formé avec un dérivé cadmien de formule générale :

(R₈)₂ Cd (53)

dans laquelle R₈ a la même signification que précédemment, ce qui fournit les composés désirés.

Les composés de formule (3) sont soit des produits connus soit des produits pouvant être préparés par des méthodes connues.

Par exemple, on peut obtenir les composés de formule (3) dans laquelle A représente un radical alkyle en C₃-C₅ moyennant la suite d'étapes ci-après :
a) acylation d'un composé de formule générale : 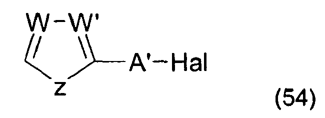 dans laquelle A', Hal, W, W' et Z ont la même signification que précédemment, avec le chlorure d'acétyle et ce, en présence d'un acide de Lewis tel que le chlorure d'aluminium, pour former un composé de formule générale : 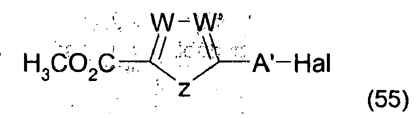 dans laquelle A', Hal, W, W' et Z ont la même signification que précédemment,
b) réaction du composé de formule (55) d'abord avec le brome en présence d'un hydroxyde de métal alcalin et ensuite avec un acide fort tel que l'acide chlorhydrique ou sulfurique, pour obtenir l'acide de formule générale : 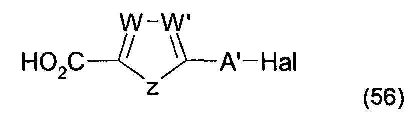 dans laquelle A', Hal, W, W' et Z ont la même signification que précédemment,
c) estérification de l'acide de formule (56) ou d'un halogénure de celui-ci obtenu après traitement par exemple au moyen de chlorure de thionyle, cette estérification étant effectuée au moyen d'un alcool de formule générale :

   R₁₈ - OH (57)

   dans laquelle R₁₈ représente un groupement alkyle en C₁-C₄, pour obtenir un ester de formule générale : 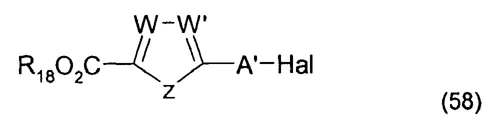 dans laquelle A', Hal, R₁₈, W, W' et Z ont la même signification que précédemment,
d) aminolyse du composé de formule (58) au moyen d'une amine de formule (6) en présence d'un agent basique tel qu'un carbonate de métal alcalin, pour former les composés de formule générale : 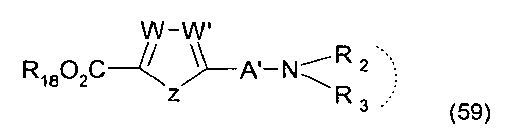 dans laquelle A',R₂,R₃,R₁₈,W , W' et Z ont la même signification que précédemment,
e) saponification du composé de formule (59) au moyen d'un agent basique tel qu'un hydroxyde de métal alcalin, pour former les acides de formule générale : 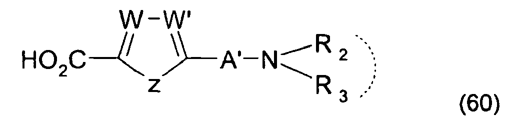 dans laquelle A', R₂, R₃, W, W' et Z ont la même signification que précédemment,
f) réaction au moyen d'un agent halogénant tel que le chlorure de thionyle, le phosgène ou le chlorure d'oxalyle, pour former les composés souhaités.

Les composés de formule (5) peuvent être préparés au départ d'un ester de formule (1) dans laquelle R représente un groupement (a) :
(a) en traitant cet ester de formule (1) à la température de reflux du milieu, au moyen de glycol en présence d'acide p-toluènesulfonique, pour former un cétal de formule générale : 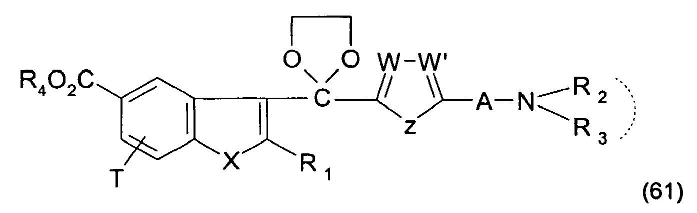 dans laquelle A, R₁, R₂, R₃, R₄, T, X, W, W' et Z ont la même signification que précédemment,
(b) en réduisant ce composé de formule (61) au moyen d'un hydrure de métal alcalin tel que l'hydrure de lithium aluminium et dans un solvant tel qu'un éther pour obtenir les composés désirés.

Les composés de formule (6) peuvent être obtenus en faisant réagir un composé cétonique de formule générale : 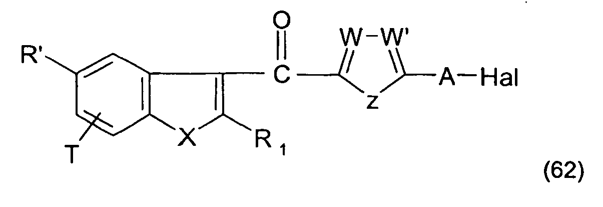 dans laquelle A, Hal, R', R₁, T, X, W, W' et Z ont la même signification que précédemment avec l'azide de sodium en présence d'iodure de tétrabutylammonium pour former les composés désirés.

Les composés de formule (7) peuvent être préparés au départ d'un composé de formule (2) par réaction de ce composé avec un dihalogénure de formule générale : 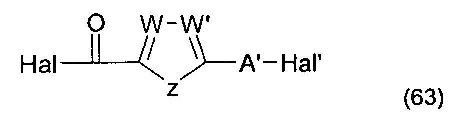 dans laquelle A', Hal, W, W' et Z ont la même signification que précédemment et Hal' représente un atome d'halogène tel que chlore ou brome, de préférence brome, la réaction ayant lieu en présence d'un acide de Lewis tel que le chlorure d'aluminium, le chlorure stannique, le chlorure ferrique ou le trifluorométhanesulfonate d'argent, ce qui fournit les composés souhaités.

De manière alternative, les composés de formule (7) dans laquelle R' représente un groupement (a) ou un groupement (f), peuvent être obtenus en saponifiant un ester de formule (7) dans laquelle R' représente un groupement -CO₂R"₄ dans laquelle R"₄ représente un groupement alkyle en C₁-C₁₀ ou cycloalkyle en C₃-C₆ et ce, en présence d'un agent basique généralement un hydroxyde de métal alcalin, en traitant le sel ainsi formé avec un acide fort tel que l'acide chlorhydrique, en faisant réagir l'acide ainsi formé avec un agent halogénant tel que le chlorure d'oxalyle, et enfin en estérifiant l'halogénure d'acyle ainsi formé avec un alcool de formule (13) ou (14) pour obtenir les composés désirés.

De même, les composés de formule (62) peuvent être préparés de manière identique à celles décrites ci-dessus pour la synthèse des composés de formule (7).

Les composés de formule (63), quant à eux, peuvent être préparés en halogénant un acide de formule (56) au moyen d'un agent approprié tel que le chlorure de thionyle, le phosgène ou le chlorure d'oxalyle, ce qui fournit les composés désirés.

Les autres composés de départ ou intermédiaires intervenant dans les différents procédés décrits précédemment sont pour la plupart des composés connus ou pouvant être préparés par des méthodes connues.

Par exemple, les amines de formule (8) sont connues et décrites dans les brevets US4831054 ou EP471609 ou pouvant être préparées par les méthodes y décrites.

On connaît déjà des dérivés de benzofurane ou de benzothiophène comportant une chaîne aminoalkyl-benzoyle et diversement substitués sur l'homocycle. De tels composés ont été décrits par exemple dans les brevets ou demandes de brevet EP651998, EP657162, W09510513 ou WO9725033 où ils sont présentés comme possédant des propriétés anticholestérolémiantes, inhibitrices de la thrombine, agonistes des oestrogènes ou inhibilitrices de la perte osseuse.

Or, on a maintenant découvert, dans le cadre de l'invention, que des dérivés de benzofurane, ou benzothiophène comportant une chaîne aminoalkylbenzoyle ainsi que d'autres groupements fixés sur l'hétérocycle par l'intermédiaire d'un atome de carbone, présentent de très intéressantes propriétés pharmacologiques notamment des propriétés antiarythmiques tout en offrant une très bonne stabilité métabolique, une solubilité très acceptable et une très bonne biodisponibilité par voie orale.

Les résultats de tests pharmacologiques effectués en vue de déterminer des propriétés des composés de l'invention sur le système cardiovasculaire sont répertoriés ci-dessous.

### I. Arythmies ventriculaires

Le but de ce test est de déterminer la capacité des composés de l'invention à assurer une protection contre les arythmies provoquées par reperfusion. A cet effet, on a utilisé la méthode rapportée par MANNING A.S. et coll. dans Circ. Res. 1984, 55 : 545-548 modifiée comme suit :
On anesthésie d'abord des rats, répartis en lots, avec du pentobarbital sodique (60mg/kg par voie intrapéritonéale) puis on les intube et les maintient sous respiration assistée.
On leur place ensuite une canule pour administration intraveineuse dans la veine jugulaire droite, on administre une dose intraveineuse du composé à étudier et 5 minutes plus tard, on place une boucle de ligature autour de l'artère coronaire descendante antérieure gauche et à proximité immédiate de son origine. On provoque alors l'occlusion de cette artère pendant 5 minutes par traction sur les extrémités de la ligature de manière à induire une reperfusion par relâchement de la tension.
On évalue alors les arythmies induites par cette reperfusion.
Un test analogue a été pratiqué par voie orale. Dans ce cas, le composé à étudier est administré 120 minutes avant la ligature de l'artère coronaire descendante antérieure gauche.
Les résultats de ces tests ont montré que les composés de l'invention protègent les animaux traités de manière significative allant jusqu'à 100% à des doses comprises entre 0,3 et 10mg/kg par voie intraveineuse et 10 à 90mglkg par voie orale.

### II. Propriétés antiadrénergiques

Le but de ce test est de déterminer la capacité des composés de l'invention à réduire l'augmentation de la pression sanguine induite par la phényléphrine (effet anti-α) et l'accélération de la fréquence cardiaque induite par l'isoprénaline (effet anti-β) chez le chien préalablement anesthésié au pentobarbital et chloralose.

On détermine d'abord pour chaque chien la dose de phényléphrine (5 ou 10µg/kg) qui provoque une augmentation de la pression artérielle comprise entre 25 et 40mm Hg et la dose d'isoprénaline (0,9 ou 1µg/kg) qui devra entraîner une augmentation de la fréquence cardiaque comprise entre 60 et 120 battements/minute.

On injecte alternativement toutes les 10 minutes les doses de phényléphrine et d'isoprénaline ainsi déterminées et après obtention de 2 réponses de référence successives, on administre une dose du composé à étudier par voie intraveineuse
- *Effet anti-*α
   On enregistre le pourcentage de réduction, par le composé de l'invention, de l'hypertension provoquée comparativement à l'hypertension de référence obtenue avant injection de ce composé (environ 100mm Hg).
- *Effet anti*-β
   On enregistre le pourcentage de réduction, par le composé à étudier, de l'accélération provoquée de la fréquence cardiaque.

Les résultats de ces tests montrent qu'à des doses variant de 1 à 10 mg/kg, les composés de l'invention présentent des effets anti-α et/ou anti-β se traduisant par des réductions de l'hypertension provoquée et/ou de l'augmentation provoquée de la fréquence cardiaque, allant de 50% à presque 100%.

### III. Fibrillation auriculaire

Le but de ce test est d'évaluer l'efficacité des composés de l'invention vis-à-vis de la fibrillation auriculaire induite par stimulation permanente du nerf vague chez le chien anesthésié selon la méthode décrite dans Circulation 1993 ;88 : 1030-1044.

Les composés à étudier sont administrés aux doses cumulées de 3 et 10 mg/kg en perfusions intraveineuses lentes de 10 minutes pendant un épisode de fibrillation auriculaire soutenu.

A la dose de 10 mg/kg les composés de l'invention convertissent généralement 100% des fibrillations auriculaires en rythme sinusal et en préviennent la ré-induction dans 50 à 100% des cas. A cette dose, on observe des augmentations significatives de la période cardiaque ainsi que des périodes réfractaires effectives auriculaires pour différentes valeurs basales de la période cardiaque.

### IV. Effets inhibiteurs du système neuro-hormonal

Le but de ce test est de rechercher les effets inhibiteurs des composés de l'invention vis-à-vis des effets vasoconstricteurs induits par différents peptides tels que la noradrénaline (NA), l'angiotensine II (A-II), l'arginine vasopressine (AVP), le neuropeptide Y (NPY) et l'endothéline (ET) et également vis-à-vis des effets tachycardes induits par l'isoprénaline (Iso) chez le rat vigile.

Chez des rats mâles Sprague Dawley d'environ 300g, on implante, 24 heures avant le test, un cathéter artériel (artère carotide droite) pour la mesure de la pression artérielle et un cathéter veineux (veine jugulaire droite) pour l'injection des produits à étudier. Le lendemain, on place les rats dans des boîtes cylindriques et on relie le cathéter artériel à un capteur de pression par l'intermédiaire d'un joint tournant sur balancier. Ce capteur de pression est lui-même relié à un polygraphe pour enregistrement de la pression artérielle.

On recherche alors l'action des composés de l'invention, par voie intraveineuse, vis-à-vis des effets vasoconstricteurs induits par la NA (1 µg/kg), l'A-II (100 µg/kg) et l'AVP (40 µg/kg) aux doses respectives de soit 3, 10 et 30 mg/kg soit 1,3 à 10 mg/kg et uniquement à la dose de 10 mg/kg vis-à-vis des effets vasoconstricteurs induits par le NPY (6 µg/kg) et l'ET (0,5 µg/kg) ou des effets tachycardies induits par l'Iso (1 µg/kg).

On solubilise d'abord les différents agonistes peptiques dans du sérum physiologique à 0,9% et le composé à étudier dans un solvant approprié. On injecte ensuite ces peptides en bolus sous un volume de 0,05 ml/kg et ce, 30 et 10 minutes avant l'administration intraveineuse de 0,1 ml/kg d'une solution du composé à étudier ou de solvant. On répète ensuite ces injections de peptide 10, 30, 60 et 120 minutes après l'administration du composé à étudier. En fonction de la durée d'action de composé à tester, on peut éventuellement prolonger ces injections toutes les 30 minutes sans jamais dépasser 5 heures au total.

On évalue alors les variations de la pression artérielle après administration d'un peptide donné en mesurant, à différents temps, la différence entre l'effet maximal induit par l'agoniste peptide et la valeur basale de la pression artérielle.

Les résultats obtenus montrent que la NA, l'A-II, L'AVP, le NPY et l'ET induisent des augmentations respectives de la pression de la fréquence cardiaque de 209±7 battements par minute.

En outre, on observe que les composés de l'invention antagonisent d'une manière dose-dépendante les effets vasoconstricteurs induits par la NA, l'A-II et l'AVP. Ils antagonisent également les effets induits par le NPY et par l'ET et l'augmentation de la fréquence cardiaque induite par l'Iso. Aux doses les plus élevées, l'inhibition maximale obtenue après 15 minutes varie entre 40 et 80% et la durée d'action est au moins supérieure ou égale à 30 minutes.

### V. Toxicité

La toxicité des composés de l'invention s'est révélée compatible avec leur utilisation en thérapeutique.

Les compositions pharmaceutiques selon l'invention peuvent être présentées sous toute forme convenant à l'administration en thérapie humaine ou vétérinaire. Par exemple les compositions pharmaceutiques de la présente invention peuvent être formulées pour l'administration orale, sublinguale, sous-cutanée, intramusculaire, intraveineuse, transdermique ou rectale. Pour ce qui concerne l'unité d'administration, celle-ci peut prendre la forme, par exemple, d'un comprimé, d'une dragée, d'une capsule, d'une gélule, d'une poudre, d'une suspension, d'un sirop ou encore de granules pour l'administration orale, d'un suppositoire pour l'administration rectale ou d'une solution ou suspension pour l'administration parentérale.

Les compositions pharmaceutiques de l'invention pourront comprendre, par unité d'administration, par exemple de 50 à 500mg en poids d'ingrédient actif pour l'administration orale, de 50 à 200mg d'ingrédient actif pour l'administration rectale et de 50 à 150mg d'ingrédient actif pour l'administration parentérale.

Suivant la voie d'administration choisie, les compositions pharmaceutiques ou vétérinaires de l'invention seront préparées en associant au moins un des composés de formule (1) ou un sel pharmaceutiquement acceptable de ce composé avec un excipient approprié, ce dernier pouvant être constitué par exemple d'au moins un ingrédient sélectionné parmi les substances suivantes : lactose, amidons, talc, stéarate de magnésium, polyvinylpyrrolidone, acide alginique, silice colloïdale, eau distillée, alcool benzylique ou agents édulcorants.

Lorsqu'il s'agit de comprimés, ceux-ci peuvent être traités de telle sorte qu'ils présentent une activité prolongée ou retardée et qu'ils libèrent d'une façon continue une quantité prédéterminée de principe actif.

Les Exemples suivants illustrent la préparation des composés et compositions de l'invention :

### EXEMPLE 1

### 2-Butyl-6-méthyl-3-[4-[3-(dibutylamino)propyl]benzoyl]-1-benzofurane-5-carboxylate d'éthyle

### A. (2-méthyl-4-hydroxy-phényl) bromométhylcétone

On introduit 75 g (0,499 mole) de (2-méthyl-4-hydroxy-phényl) méthylcétone dans 500 ml de dioxane, puis, à température inférieure ou égale à 25°C, on additionne 29,7 ml de brome dans 750 ml de dioxanne. On agite à température ambiante durant 2 heures, on concentre et on recristallise dans l'éther diisopropylique.

De cette manière, on obtient 76,82 g de produit désiré.
Rendement : 67,2%
P.F. : 130-131 °C

### B. Acide 2-méthyl-4-hydroxy-benzoïque

On introduit 76,8 g de composé obtenu à l'étape précédente dans 310 ml d'acétate d'éthyle puis on ajoute 70,8 ml de pyridine. La température s'élève progressivement de 20°C à 50°C. On chauffe alors à 70°C pendant une heure puis on laisse revenir le milieu réactionnel à la température ambiante.

On essore puis on reprend le solide isolé dans 710 ml d'hydroxyde de sodium aqueux à 10%. On porte au reflux durant une heure puis on laisse revenir à la température ambiante. On lave la phase aqueuse avec de l'éther diéthylique, on l'acidifie avec de l'acide chlorhydrique concentré et on extrait avec de l'acétate d'éthyle. On lave ensuite les extraits avec de l'eau et une solution saturée de chlorure de sodium. On purifie alors par recristallisation dans l'eau.

De cette manière, on obtient 41,94 g de composé désiré.
Rendement : 82,2%
P.F. : 178-182°C

### C. Acide 2-méthyl-4-hydroxy-5-iodo-benzoïque

On porte au reflux, un mélange de 7 g (46 mmoles) de composé obtenu à l'étape précédente, 125 ml de méthanol, 3,68 g (2 équivalents) d'hydroxyde de sodium et 15,87 g (2,3 équivalents) d'iodure de sodium. Toujours au reflux, on ajoute, en 30 minutes environ, 115 ml d'une solution d'hypochlorite de sodium à environ 4% de chlore actif. On poursuit le reflux pendant 15 minutes puis on laisse revenir à température ambiante. On ajoute 92 ml de thiosulfate de sodium à 10 % puis 20,7 ml d'acide chlorhydrique concentré. On extrait avec de l'éther diisopropylique puis on lave avec de l'eau jusqu'à pH neutre.

De cette manière, on obtient 13,34 g de composé désiré sous forme brute.
Rendement : quantitatif
P.F. : entre 143° C et 150° C

### D. 2-Méthyl-4-hydroxy-5-iodo-benzoate d'éthyle

On mélange 13,3 g de composé obtenu à l'étape précédente dans 250 ml d'éthanol absolu contenant 2,5 ml d'acide sulfurique. On porte au reflux pendant 60 heures, on concentre à sec et on extrait avec de l'acétate d'éthyle. On lave avec de l'eau, avec une solution de bicarbonate de sodium, avec de l'eau et avec une solution de chlorure de sodium. On dissout alors l'extrait à chaud dans l'hexane. On filtre sur papier pour éliminer un insoluble huileux, on refroidit et on essore, ce qui fournit 2,79 g de produit désiré. On concentre le filtrat et on chromatographie sur silice (éluant : dichlorométhane) ce qui fournit 2,12 g supplémentaires de produit attendu.

De cette manière, on recueille 4,91 g du produit souhaité.
Rendement : 34,9 %
P.F. : 131-134° C

### E. 2-Butyl-6-métharl-1-benzofuranne-5-carboxylate d'éthyle

On constitue un milieu réactionnel à partir de 4,91 g (16 mmoles) de composé obtenu à l'étape précédente et de 50 ml de N;N-diméthylformamide auquel on ajoute 2,66 g de n-hexyne, 19 ml de pipéridine, 0,340 g de dichloro bis-(triphénylphosphine)palladium et 0,087 g d'iodure cuivreux. On chauffe à 90° C pendant 2 heures, on refroidit et on concentre. On extrait avec de l'acétate d'éthyle et on lave avec de l'acide chlorhydrique dilué puis avec de l'eau. On purifie ensuite par chromatographie sur silice (éluant : dichlorométhane/heptane 50/50).

De cette manière, on recueille 2,87 g de composé désiré.
Rendement : 68,9 %

### F. 2-Butyl-6-méthyl-3-[4-(3-bromopropyl) benzoyl]-1-benzofurane-5-carboxylate d'éthyle

A 2,68 g (16,5 mmoles) de chlorure ferrique dans 70 ml de dichloréthane, on ajoute, à une température inférieure à 15° C, 2,87 g (11 mmoles) de composé obtenu à l'étape précédente dans 25 ml de 1,2-dichloréthane. Toujours à une température inférieure à 15° C, on additionne alors 4,32 g (16,5 mmoles) de chlorure de 4-(3-bromopropyl) benzoyle dans 25 ml de 1,2-dichloréthane. On agite à température ambiante pendant 18 heures puis on verse sur un mélange glace/eau. On extrait avec du dichlorométhane et on lave d'abord avec de l'eau jusqu'à pH neutre ensuite avec une solution de chlorure de sodium. On purifie alors par chromatographie sur silice (éluant : dichlorométhane/heptane 70/30)

De cette manière, on obtient 2,72 g de composé désiré.
Rendement : 50,9 %

### G. 2-Butyl-6-méthyl-3-[4-[3-(dibutylamino)propyl]benzoyl]-1-benzofurane-5-carboxylate d'éthyle

On dissout 2,7 g (5,56 mmoles) de composé obtenu à l'étape précédente dans 60 ml d'acétonitrile et on ajoute à ce milieu réactionnel 1,43 g (11,1 mmoles) de dibutylamine, 0,833 g (1 équivalent) d'iodure de sodium et 2,31 g (16,7 mmoles) de carbonate de potassium. On porte au reflux durant 18 heures et on verse dans l'eau. On extrait avec de l'acétate d'éthyle puis on lave avec de l'eau et une solution de chlorure de sodium. On purifie alors par chomatographie sur silice (éluant : 98/2/0,1 dichlorométhane/méthanol/ammoniaque à 20 %).

De cette manière, on obtient 2,052 g de composé désiré.
Rendement : 69,14%

De manière analogue à celle décrite précédemment, on a préparé les composés suivants :
2-Butyl-6-méthyl-3-[4-[3-(dibutylamino)propyl]benzoyl]-1-benzofurane-5-carboxylate de méthyle **(Exemple 2).**
   Rendement : 84,4 %
2-Butyl-6-méthyl-3-[4-[3-(dibutylamino)propyl]benzoyl]-1-benzofurane-5-carboxylate d'isopropyle **(Exemple 3).**
   Rendement : 87%

### EXEMPTE 4

### Oxalate de 2-butyl-6-méthyl-3-[4-[3-(dibutylamino)propyl]benzoyl]-1-benzofurane-5-carboxylate d'éthyle

On introduit 1,997 g (3,74 mmoles) de 2-butyl-6-méthyl-3-[4-[3-(dibutylamino) propyl]benzoyl]-1-benzofurane-5-carboxylate d'éthyle dans la quantité nécessaire d'éthanol absolu pour obtenir une dissolution totale. On ajoute alors 0,337 g (1 équivalent) d'acide oxalique. On concentre à sec et on triture dans l'éther diéthylique. On essore et on sèche.

De cette manière, on obtient 2 g d'oxalate désiré.
Rendement : 85,7 %
P.F. : 158-160°C

De manière analogue à celle décrite précédemment, on a préparé les composés ci-dessous :
Oxalate de 2-butyl-6-méthyl-3-[4-[3-(dibutylamino)propyl]benzoyl]-1-benzofurane-5-carboxylate de méthyle **(Exemple 5).**
   Rendement : 83,8%
   P.F. 164-166°C
Oxalate de 2-butyl-6-méthyl-3-[4-[3-(dibutylamino)propyl]benzoyl]-1-benzofurane-5-carboxylate d'isopropyle **(Exemple 6).**
   Rendement: 85%
   P.F. : 104-106°C

### EXEMPLE 7

### Fumarate de 2-butyl-3-[4-[3-(dibutylamino)propyl]benzoyl]-1-benzofurane-5-carboxylate d'isopropyle

On introduit dans 300 ml d'acétonitrile, 14,67 g (environ 30 mmoles) de 2-butyl-3-[4-(3-bromopropyl)benzoyl]-1-benzofurane-5-carboxylate d'isopropyle, 11,72 g (3 équivalents) de dibutylamine, 4,53 g (1 équivalent) d'iodure de sodium et 12,63 g (3 équivalents) de carbonate de potassium.

On porte au reflux durant 18 heures puis on évapore. On reprend dans l'éther diéthylique et on lave ensuite avec de l'eau et avec une solution saturée de chlorure de sodium. On purifie ensuite par chromatographie sur silice (éluant: dichlorométhane/ méthanol/ammoniaque 100/3/0,2), ce qui fournit 11,50 _g (rendement : 72 %) de composé désiré sous forme de base libre.

On dissout alors 0,533 g de produit basique ainsi obtenu, dans 20 ml d'acétone et on ajoute 0,116 g (1 équivalent) d'acide fumarique. On évapore et on reprend dans l'éther diéthylique. On filtre et on sèche sous vide.

De cette manière, on obtient le fumarate désiré.
P.F. : 132-134° C.

De manière analogue à celle décrite précédemment, on a préparé les composés suivants :
Oxalate de 2-butyl-3-[4-[3-(dibutylamino)propyl]benzoyl]-1-benzofurane-5-carboxylate d'isopropyle **(Exemple 8).**
   P.F. : 131-133°C
Oxalate de 2-butyl-6-méthyl-3-[4-[3-(butylamino)propyl]benzoyl]-1-benzofurane-5-carboxylate d'isopropyle **(Exemple 9).**
Spectre R.M.N. (résonance magnétique nucléaire) (200MHz)
Solvant : DMSO (diméthylsulfoxyde) à 2,5 ppm
δ (ppm) :
0,6 à 1,7 ; massif; 20H, 4CH₃, 4CH₂
1,85 ; multiplet ; 2H, CH₂
2,6 ; singulet ; 3H-CH₃
2,6 à 3 ; massif ; 8H, 2CH₂, 2NCH₂
5 ; septuplet ; 1H, OCH
6,1 ; singulet élargi ; 2COOH, DOH
7,2 à 7,9 ; massif ; 6H, ¹H aromatique

### EXEMPLE 10

### 2-Butyl-3-[4-[3-(dibutylamino)propyl]benzoyl]-1-benzofurane-5-carboxylate de méthyle

Dans 100 ml d'acétonitrile, on introduit 5 g (0,01 mole) de 2-butyl-3-[4-(3-bromopropyl)benzoyl]-1-benzofurahe-5-carboxylate de méthyle, 2,58 g (0,02 mole) de dibutylamine, 1,5 g (0,01 mole) d'iodure de sodium et 4,18 g (3 équivalents) de carbonate de potassium. On porte au reflux durant environ 20 heures, on extrait avec de l'éther diéthylique et on lave deux fois avec de l'eau puis avec une solution saturée de chlorure de sodium. On purifie ensuite par chromatographie sur silice (éluant : dichlorométhane/méthanol/ammoniaque 98/2/0,1).

De cette manière, on obtient 4 g de composé désiré.
Rendement : 79%

### EXEMPLE 11

### Chlorhydrate de l'acide 2-butyl-3-[4-[3-(dibutylamino)propyl]benzoyl]-1-benzofurane -5-carboxylique

On maintient sous agitation, à température ambiante et pendant 4 heures, un milieu réactionnel formé de 4 g de 2-butyl-3-[4-[3-(dibutylamino)propyl]benzoyl]-1-benzofurane-5-carboxylate de méthyle, 0,632 g (2 équivalents) d'hydroxyde de sodium, 66 mi de dioxane, 13 ml d'eau et 13 ml de méthanol. On évapore, on reprend dans l'eau et on ajoute de l'acide chlorhydrique dilué jusqu'à pH = 4. On extrait avec du chloroforme et on lave avec de l'eau et une solution saturée de chlorure de sodium.

On purifie alors par chromatographie sur silice (éluant : dichlorométhane/ méthanol 95/5) pour donner 2,3 g (rendement : 59,2 %) du composé désiré.

On dissout alors dans l'acétate d'éthyle 2,3 g du composé, ainsi obtenu et on ajoute goutte à goutte un mélange d'acide chlorhydrique dans l'acétate d'éthyle et ce, jusqu'à pH acide. On évapore l'acétate d'éthyle, on reprend dans l'éther diéthylique et on filtre le précipité.

De cette manière, on obtient 2,18 g du chlorhydrate désiré.
Rendement : 88,2 %
P.F. : 147-149° C.
En utilisant le même procédé que précédemment, on a préparé le composé suivant : Oxalate de l'acide 2-butyl-6-méthyl-3-[4-[3-(dibutylamino)propyl]benzoyl]-1-benzofurane-5-carboxylique **(Exemple 12).**
P.F. : 138°C

### EXEMPLE 13

### Chlorhydrate de 2-butyl-3-[4-[3-(dibutylamino)propyl]benzoyl]-1-benzofurane-5-carboxamide

On dissout 3,5 g (7,1 mmoles) d'acide 2-butyl-3-[4-[3-(dibutylamino) propyl]benzoyl]-1-benzofurane-5-carboxylique dans 50 ml de 1,2-dichloréthane. On ajoute ensuite 10 ml de chlorure de thionyle et on porte à reflux durant 3 heures. On évapore pour obtenir un produit brut que l'on reprend à nouveau dans 50 mi de dichloréthane contenant 10 ml d'ammoniaque.

On maintient le milieu réactionnel à température ambiante durant 15 heures puis on évapore. On reprend avec de l'éther diéthylique puis on lave avec de l'eau puis avec une solution de chlorure de sodium.

On purifie ensuite par chromatographie sur silice (éluant : dichlorométhane/ méthanol/ammoniaque 95/5/0,1) ce qui fournit 1,9 g (rendement : 54 %) de composé désiré sous forme de base libre.

On dissout ensuite, dans l'éther diéthylique, 1,9 g (3,9 mmoles) du composé basique ainsi obtenu, on ajoute une solution de chlorure d'hydrogène dans l'éther diéthylique et on évapore.

De cette manière, on obtient 1,6 g de chlorhydrate désiré sous forme d'une poudre amorphe.
Rendement : 78 %
Spectre R.M.N. (200 MHz)
Solvant DMSO à 2,5 ppm
DOH à 3,33 ppm
δ (ppm) :
0,7 ; triplet ; 3H, CH₃
0,8 ; triplet ; 6H, 2CH₃
1,2 ; multiplet ; 6H, 3CH₂
1,5 ; multiplet ; 6H, 3CH₂
1,95 ; multiplet ; 2H, CH₂
2,7 ; triplet ; 4H, 2CH₂
2,9 ; multiplet ; 6H, 3NCH₂
7 à 8 ; massif ; 9H, CONH₂, 7H aromatique
10,2 ; singulet élargi ; 1H, NH⁺

### EXEMPLE 14

### Oxalate de 2-butyl-3-[4-[3-(dibutylamino)propyl]benzoyl]-N,N-diméthyl-1-benzofurane-5-carboxamide

On dissout 2,8 g (5,3 mmoles) d'acide 2-butyl-3-[4-[3-(dibutylamino)propyl] benzoyl]-1-benzofurane-5-carboxylique dans 50 ml de dichloréthane.

On ajoute 10 ml de chlorure de thionyle et on porte au reflux durant 3 heures.

On évapore, on reprend le chlorure d'acyle formé dans 40 ml de dichlorométhane et on sature à 5° C avec la diméthylamine gaz. On agite à température ambiante durant 15 heures et on reprend dans l'eau. On décante et on extrait avec du dichlorométhane. On lave ensuite avec de l'eau et avec une solution de chlorure de sodium puis on purifie par chromatographie sur silice (éluant : déchlorométhane/méthanol/ammoniaque 98/2/0,2), ce qui fournit 2 g (rendement : 68 %) de composé désiré sous forme de base libre.

On dissout alors, dans l'éthanol absolu, 1,82 g (3,3 mmoles) du composé basique ainsi obtenu et on ajoute 0,296 g (3,3 mmoles) d'acide oxalique dissous dans l'éthanol absolu. On évapore et cristallise dans l'éther diéthylique. On filtre ensuite puis on sèche sous vide.

De cette manière, on obtient 1,65 g d'oxalate désiré.
Rendement : 78 %
P.F. : 77-79° C

### EXEMPLE 15

### 2-Butyl-3-[4-(2-aminoéthyl)benzoyl]-1-benzofurane-5-carboxylate de méthyle A. 2-Butyl-3-[4-(2-azidoéthyl)benzoyl]-1-benzofurane-5-carboxylate de méthyle

On introduit dans 10 ml d'acétonitrile contenant 20 ml de N,N-diméthylformamide, 1,99 g (5 mmoles) de 2-butyl-3-[4-(2-chloroéthyl)benzoyl]-1-benzofurane-5-carboxylate de méthyle, 0,650 g (10 mmoles) d'azide de sodium et 0,180 g (environ 0,5 mmoles) d'iodure de tétrabutylammonium.

On porte au reflux durant 20 heures puis on dilue dans l'eau. On extrait avec de l'éther diéthylique et lave deux à trois fois avec de l'eau puis avec une solution saturée de chlorure de sodium. On purifie alors par chromatographie sur silice en utilisant le dichlorométhane comme éluant.

De cette manière, on obtient 1,80 g de composé désiré.
Rendement : 89 %
P.F. : 60-62° C

### B. 2-Butyl-3-[4-(2-aminoéthyl)benzoyl]-1-benzofurane-5-carboxylate de méthyle

On dissout dans 15 ml de tétrahydrofurane, 1,37 g (environ 3,4 mmoles) du composé obtenu au paragraphe précédent. On refroidit par un mélange glace/eau et on ajoute, par fractions, 0,89 g de triphénylphosphine. On enlève le bain glacé et on agite à température ambiante durant 3 heures. On ajoute 1 ml d'eau et on agite à température ambiante durant 16 heures. On évapore, on reprend avec de l'éther diéthylique et on lave avec de l'eau et avec une solution saturée de chlorure de sodium. On purifie ensuite par chromatographie sur silice (éluant : dichlorométhane/ méthanol/ammoniaque 100/7/0,5)

De cette manière, on obtient 1,17 g de composé désiré.
Rendement : 91 %

### EXEMPLE 16.

### 2-Butyl-3-[4-[2-(dibutyrlamino)éthyl]benzoyl]-1-benzofurane-5-carboxylate de méthyle

Sous argon, on dissout dans 40 ml de dichlorométhane 1,12 g (environ 3 mmoles) de 2-butyl-3-[4-(2-aminoéthyl)benzoyl]-1-benzofurane-5-carboxylate de méthyle et 0,648 g (environ 9 mmoles) de butyraldéhyde. On ajoute en une fois 1,91 g (9 mmoles) de triacétoxyborohydrure de sodium et on agite à température ambiante durant 18 heures. On évapore et on reprend avec de l'éther diéthylique. On lave ensuite avec une solution de bicarbonate sodique, avec de l'eau et finalement avec une solution saturée de chlorure de sodium.

On purifie alors par chromatographie sur silice (éluant: dichlorométhane/ méthanol/ammoniaque 100/2,5/0,15)

De cette manière, on obtient 1,016 g de produit désiré.
Rendement : 69 %

### EXEMPLE 17

### Acide 2-butyl-3-[4-[2-(dibutylamino)éthyl]benzoyl]-1-benzofurane-5-carboxylique

Dans 300 ml de dioxane, on introduit 15,5 g (31,7 mmoles) de 2-butyl-3-[4-[2-(dibutylamino)éthyl]benzoyl]-1-benzofurane-5-carboxylate de méthyle, 60 ml d'eau et 60 ml de méthanol.

On ajoute alors 2,59 g d'hydroxyde de sodium et on agite à température ambiante durant environ 20 heures, ce qui forme le composé désiré sous forme de sel sodique. On évapore, reprend dans l'eau et acidifie jusqu'à pH = 5-6 avec de l'acide chlorhydrique dilué. On extrait avec de l'acétate d'éthyle et on lave avec une solution saturée de chlorure de sodium.

De cette manière, on obtient 18 g de composé désiré sous forme brute.

### EXEMPLE 18

### Oxalate de 2-butyl-3-[4-[2-(dibutylamino)éthyl]benzoyl]-1-benzofurane-5 carboxylate d'isopropyrle

Dans 50 ml de dichloréthane, on introduit 3,33 g d'acide 2-butyl-3-[4-[2-(dibutylamino)éthyl]benzoyl]-1-benzofurane-5-carboxylique et 5 ml de chlorure de thionyle. On porte à reflux durant 2 heures et on évapore. On reprend dans l'éther diéthylique et on évapore à nouveau.

On reprend le résidu dans 50 ml d'isopropanol et on porte à reflux durant 16 heures. On évapore et on reprend dans une solution de bicarbonate sodique. On extrait avec de l'éther diéthylique et on purifie par chromatographie sur silice (éluant: dichlorométhane/méthanol/ammoniaque 100/2/0,1), ce qui fournit 1,63 g (rendement : 53,5%) de composé désiré sous forme basique.

On introduit alors dans l'acétone 1,58 g du composé basique ainsi obtenu, ainsi que 0,270 g d'acide oxalique. On évapore, reprend dans l'éther diéthylique et filtre. On sèche alors sous vide.

De cette manière, on obtient 1,29 g d'oxalate désiré.
Rendement : 68,5%
P. F. : 148-151°C

### EXEMPLE 19

### Oxalate de 2-butyl-3-[4-[3-(dibutylamino)propyl]benzoyl]-1-benzofurane-5-carboxylate d'isobutyle

Dans 30 ml de dichloréthane, on dissout 1,58 g (3 mmoles) d'acide 2-butyl-3-[4-[3-(dibutylamino)propyl]benzoyl]-1-benzofurane-5-carboxylique et on ajoute 6 ml de chlorure de thionyle. On porte au reflux durant 3 heures, on évapore et on reprend trois fois dans l'éther diéthylique.

On reprend à nouveau le produit brut obtenu dans 30 ml d'isobutanol et on porte à reflux pendant 15 heures. On évapore et on reprend avec de l'éther diéthylique. On lave avec une solution diluée de carbonate de potassium, avec de l'eau et finalement avec une solution de chlorure de sodium. On purifie ensuite par chromatographie sur silice (éluant: dichlorométhane/méthanol/ammoniaque 96/4/0,1), ce qui fournit le composé désiré sous forme de base.

On dissout alors dans de l'éthanol absolu, 1,5 g (2,6 mmoles) du composé basique ainsi obtenu et on ajoute 0,231 g d'acide oxalique dans l'éthanol absolu.

On évapore et on reprend dans l'éther diéthylique. On filtre ensuite et sèche sous vide.

De cette manière, on obtient 1,22 g d'oxalate désiré.
Rendement : 73 %
P.F.: 105-107° C.

### EXEMPLE 20

### Oxalate de 2-butyl-3-[4-[3-(dibulylamino)propyl]benzoyl]-1-benzofurane-5-carboxylate de cyclobutyle

On introduit 3,21 g (65,3 mmoles) d'acide 2-butyl-3-[4-[3-(dibutylamino)propyl] benzoyl]-1-benzofurane-5-carboxylique dans 50 ml de toluène. On ajoute alors à température ambiante 2 ml de chlorure d'oxalyle dans 10 ml de toluène.

On agite à température ambiante puis on chauffe à 80° C pendant 2 heures. On concentre à sec et on reprend dans l'éther diéthylique. On concentre de nouveau à sec et on reprend le produit brut obtenu dans 50 ml de dichloréthane. On ajoute alors 0,518 g (71,8 mmoles) de cyclobutanol et 0,568 g (71,8 mmoles) de pyridine.

On agite à température ambiante pendant 72 heures, on concentre à sec et on reprend avec de l'acétate d'éthyle. On lave avec de l'eau et une solution de chlorure de sodium puis on purifie par chromatographie sur silice (éluant : dichlorométhane/ méthanol/ammoniaque 97/3/0,1), ce qui fournit 1,409 g (rendement: 39,5 %) de composé désiré sous forme de base.

On ajoute alors 1,335 g (24,5 mmoles) de composé basique ainsi formé à la quantité nécessaire de méthanol pour obtenir une dissolution totale puis on additionne 0,220 g (24,5 mmoles) d'acide oxalique. On concentre à sec et on triture dans l'éther diéthylique. On essore et on sèche.

De cette manière, on obtient 1,409 g de composé désiré.
Rendement : 90,45 %
P.F.: 134-137°C.

### EXEMPLE 21

### Oxalate de 2-butyl-3-[4-[3-(dibutylamino)propyl]benzoyl]-1-benzofurane-7-carboxylate d'isopropyle

### A. 3-Hydroxy-2-méthoxy-benzaldéhyde

On introduit 13,08 g (0,39 mole) d'hydrure de sodium dans 300 ml de diméthylsulfoxyde puis on ajoute 54 g (0,39 mole) de 2,3-dihydroxy-benzaldéhyde. On maintient le milieu réactionnel durant 1 heure à température ambiante puis on ajoute, goutte à goutte, 55,35 g (26,3 ml ; 0,39 mole) d'iodure de méthyle dans 60 ml de diméthylsulfoxyde. On maintient durant 18 heures à température ambiante puis on dilue avec de l'eau. On ajoute de l'acide chlorhydrique, on extrait avec de l'acétate d'éthyle et on lave avec une solution aqueuse de chlorure de sodium. On purifie ensuite par chromatographie sur silice (éluant : dichlorométhane/acétate d'éthyle 97/3).

De cette manière, on obtient 27,69 g de produit désiré.
P.F. : 116°C

### B. 2-Méthoxy-3-trifluorométhanesulfonyloxy-benzaldéhyde

On dissout 27,69 g (0,182 mole) de 3-hydroxy-2-méthoxy-benzaldéhyde dans 290 ml de dichlorométhane et on ajoute 16 g (0,2 mole) de pyridine. A une température de 0 à 5°C, on additionne ensuite 56,42 g (33,65 ml ; 0,2 mole) d'anhydride trifluorométhanesulfonique dans 290 ml de dichlorométhane. On laisse le milieu réactionnel revenir à la température ambiante et on l'y maintient durant 2 heures. On évapore et on extrait avec de l'éther diéthylique. On lave avec de l'eau, de l'acide chlorhydrique dilué, de l'eau, une solution d'hydrogénocarbonate sodique, de l'eau et une solution de chlorure de sodium. On purifie ensuite par chromatographie sur silice (éluant : dichlorométhane/hexane 90/10).

De cette manière, on obtient 45,1 g de produit désiré.
Rendement : 87 %

### C. 2-Méthoxy-3-(hexyn-1-yl)-benzaldéhyde

Dans 426 ml de N,N-diméthylformamide on dissout 45 g (0,158 mole) de 2-méthoxy-3-trifluorométhanesulfonyloxy-benzaldéhyde et 26 g (36,36 ml ; 0,317 mole) de 1-hexyne. On ajoute 79,58 g (109,6 ml ; 0,788 mole) de triéthylamine et 5,53 g (0,0079 mole) de dichloro bis-(triphénylphosphine)palladium. On chauffe le milieu réactionnel à 90°C durant 2 heures, on dilue avec une solution aqueuse d'acide chlorhydrique et on extrait avec de l'éther diéthylique. On lave avec de l'eau et une solution de chlorure de sodium. On purifie ensuite par chromatographie sur silice (éluant : dichlorométhane/hexane 5/5).

De cette manière, on obtient 22,4 g de produit désiré.
Rendement : 65 %

### D. 2-Méthoxy-3-(hexyn-1-yl)-benzoate de méthyle

On dissout 20 g (0,092 mole) de 2-méthoxy-3-(hexyn-1-yl)-benzaldéhyde dans 840 ml de méthanol et on ajoute 23,9 g de cyanure de sodium ainsi que 9,24 ml d'acide acétique et 187,8 g d'oxyde manganeux. On maintient le milieu réactionnel à température ambiante durant 18 heures, on filtre et on évapore. On extrait avec de l'acétate d'éthyle et on lave à l'eau et avec une solution de chlorure de sodium.

De cette manière, on obtient 20 g de composé désiré.
Rendement : 88 %

### E. 2-Butyl-1-benzofurane-7-carboxylate de méthyle

On dissout 16,57 g (0,0673 mole) de 2-méthoxy-3-(hexyn-1-yl)-benzoate de méthyle dans 500 ml de N,N-diméthylformamide et on ajoute 8,56 g (0,2 mole) de chlorure de lithium. On porte à reflux durant 18 heures puis on dilue avec de l'eau. On ajoute une solution d'hydrogénosulfate de potassium et on extrait avec de l'acétate d'éthyle. On lave ensuite avec de l'eau et avec une solution de chlorure de sodium, ce qui fournit 16,51 g d'un mélange de composé désiré et d'acide 2-butyl-1-benzofurane-7-carboxylique.
Dans 300 ml de méthanol, on dissout alors 17,5 g (0,078 mole) du mélange obtenu précédemment et on ajoute 5 ml d'acide sulfurique. On porte à reflux durant 5 heures et on évapore. On extrait avec de l'acétate d'éthyle et on lave avec de l'eau et une solution de chlorure de sodium. On purifie ensuite par chromatographie sur silice (éluant : dichlorométhane/hexane 6/4).

De cette manière, on obtient 13,24 g de composé désiré.
Rendement : 73%

### F. 2-Butyl-3-[4-(3-bromopropyl) benzoyl]-1-benzofurane-7-carboxylate de méthyle

On introduit 5,29 g (0,0326 mole) de chlorure ferrique dans 122 ml de 1,2-dichloréthane puis, à température inférieure ou égale à 15°C, 5,05 g (0,0217 mole) de 2-butyl-1-benzofurane-1-carboxylate de méthyle dans 61 ml de 1,2-dichloréthane. On ajoute ensuite 8,2 g (0,0326 mole) de chlorure de 4-(3-bromopropyl) benzoyle dans 61 ml de 1,2-dichloréthane et on maintient à température ambiante durant 18 heures. On verse sur un mélange glace/eau et on extrait avec du dichlorométhane. On lave avec de l'eau, de l'acide chlorhydrique dilué, de l'eau, une solution d'hydrogénocarbonate sodique, avec de l'eau et finalement avec une solution saturée de chlorure de sodium. On purifie ensuite par chromatographie sur silice (éluant : dichlorométhane).

De cette manière, on obtient 7,95 g de composé désiré.
Rendement : 82 %

### G. Acide 2-butyl-3-[4-(3-bromopropyl) benzoyl]-1-benzofurane-7-carboxylique

On dissout 9,26 g (0,0207 mole) de 2-butyl-3-[4-(3-bromopropyl) benzoyl]-1-benzofurane-7-carboxylate de méthyle dans 168 ml de dioxanne et on ajoute 1,65 g (0,0414 mole) d'hydroxyde de sodium dans 39 ml d'eau. On ajoute 39 ml de méthanol et on maintient à température ambiante durant 15 heures. On évapore et on reprend dans l'acide chlorhydrique jusqu'à pH=1. On extrait avec de l'acétate d'éthyle et on lave d'abord avec de l'eau jusqu'à pH=7, ensuite avec une solution de chlorure de sodium.

De cette manière, on obtient 8,39 g de composé désiré.
Rendement : 94 %

### H. 2-Butyl-3-[4-(3-bromopropyl)benzoyl]-1-benzofurane-7-carboxylate d'isopropyle

Dans 100 ml de toluène, on dissout 8,39 g (0,0193 mole) d'acide 2-butyl-3-[4-(3-bromopropyl)benzoyl]-1-benzofurane-7-carboxylique et on ajoute, à température ambiante 6,5 ml de chlorure d'oxalyle dans 20 ml de toluène. On porte à reflux durant 2 heures, puis on évapore. On reprend le résidu dans 100 ml d'isopropanol, on reflue durant 15 heures puis on évapore. On purifie ensuite par chromatographie sur silice (éluant : dichlorométhane).

De cette manière, on obtient 4,7 g de composé désiré.
Rendement : 51 %

### I. Oxalate de 2-butyl-3-[4-[3-(dibutylamino)propyl]benzoyl]-1-benzofurane-7-carboxylate d'isopropyle

Dans 80 ml d'acétonitrile, on dissout 4,5 g (0,0095 mole) de 2-butyl-3-[4-(3-bromopropyl)benzoyl]-1-benzofurane-7-carboxylate d'isopropyle. On ajoute alors 3,68 g (0,0285 mole) de dibutylamine puis 1,42 g (0,0095 mole) d'iodure de sodium et 3,94 g (0,0285 mole) de carbonate de potassium. On porte à reflux durant 15 heures puis on évapore. On extrait avec de l'éther diéthylique et on lave avec de l'eau et une solution de chlorure de sodium. On purifie ensuite par chromatographie sur silice (éluant : dichlorométhane/méthanol/ammoniaque 100/3/0,2), ce qui fournit 2,91 g du composé désiré sous forme de base libre (rendement : 57 %).
Dans de l'éthanol absolu, on dissout alors 2,91 g (0,00545 mole) du composé basique ainsi obtenu puis on ajoute une solution de 0,490 g (0,00545 mole) d'acide oxalique dans l'éthanol absolu. On évapore, on reprend dans l'éther diéthylique et on laisse cristalliser. On filtre et sèche.

De cette manière, on obtient 2,65 g du composé désiré.
Rendement : 78 %
P.F. : 118-119°C

### EXEMPLE 22

### Oxalate de 2-butyl-3-[4-[3-(dibutylamino)propyl]benzoyl]-1-benzofurane-6-carboxylate d'isopropyle

### A. 3-Méthoxy-4-trifluorométhanesulfonyloxy-benzoate d'isopropyle.

Dans 20 ml de dichlorométhane, on introduit 2,1 g (0,01 mole) de 3-méthoxy-4-hydroxy-benzoate d'isopropyle et 0,3 g (1,1 équivalent) de pyridine. On ajoute alors goutte à goutte entre 0° et 5°C, une solution de 3,1 g (1,1 équivalent) d'anhydride trifluorométhanesulfonique dans 10 ml de dichlorométhane. On laisse revenir à la température ambiante, on agite durant 0,5 h à cette température et on évapore. On extrait avec de l'acétate d'éthyle et on lave avec de l'eau, de l'acide chlorhydrique dilué, une solution de bicarbonate sodique, de l'eau et enfin une solution saturée de chlorure de sodium.

De cette manière, on obtient 3,22 g de composé désiré.
Rendement : 94%

### B. 3-Méthoxy-4-(hexyn-1-yl)-benzoate d'isopropyle

Dans 25 ml de N,N-diméthylformamide, on introduit 3,18 g (9,3 mmoles) de composé obtenu à l'étape précédente, 1,52 g (2 équivalents) de 1-hexyne, 6,5 ml (environ 5 équivalents) de triéthylamine et 0,325 g (0,05 équivalent) de dichloro bis-(triphénylphosphine)palladium. On chauffe à 90°C durant 2 heures, on dilue avec de l'eau et on ajoute de l'acide chlorhydrique dilué. On extrait avec de l'éther diéthylique et on lave avec de l'eau et une solution saturée de chlorure de sodium. On purifie ensuite par chromatographie sur silice (éluant : dichlorométhane/heptane 1/1).

De cette manière, on obtient 1,35 g de composé désiré.
Rendement : 53%

### C. Acide 2-butyl-1-benzofurane-6-carboxylique

On introduit 9,62 g (35 mmoles) de composé obtenu à l'étape précédente dans 200 ml de dichlorométhane et on refroidit à environ -70°C. On ajoute alors 70 ml (2 équivalents) d'une solution de tribromure de bore 1 M dans le dichlorométhane. On laisse revenir à température ambiante et on agite durant 1 heure à température ambiante. On refroidit au moyen d'un mélange glace/eau et on hydrolyse. On extrait avec du dichlorométhane et on lave avec de l'eau et une solution saturée de chlorure de sodium. On purifie ensuite par chromatographie sur silice (éluant : dichlorométhane/méthanol 100/3).

De cette manière, on obtient 2,5 g de composé désiré.
Rendement : 32,7%
P.F. : 102-104°C

### D. 2-Butyl-1-benzofurane-6-carboxylate de méthyle

On introduit 2,5 g de composé obtenu à l'étape précédente dans 80 ml de méthanol et on ajoute 1ml d'acide sulfurique concentré. On porte au reflux durant 6 heures et on évapore. On extrait avec de l'éther diéthylique et on lave avec de l'eau, une solution de bicarbonate sodique, de l'eau et une solution saturée de chlorure de sodium.

De cette manière, on obtient 2,60 g de composé désiré.
Rendement : 98%

### E. 2-Butyl-3-[4-(3-bromopropyl)benzoyl]-1-benzofurane-6-carboxylate de méthyle

On introduit 7,30 g (1,5 équivalent) de chlorure ferrique dans 70 ml de dichlorométhane. On ajoute alors, à une température inférieure ou égale à 15°C, 6,96 g (0,03 mole) de 2-butyl-1-benzofurane-6-carboxylate de méthyle dissous dans 30 ml de dichlorométhane puis, dans les mêmes conditions, 11,77 g (1,5 équivalent) de chlorure de 4-(3-bromopropyl)benzoyle dissous dans 30 ml de dichlorométhane. On agite à température ambiante durant 18 heures puis on verse sur un mélange glace/eau. On extrait avec du dichlorométhane et lave avec de l'eau et une solution saturée de chlorure de sodium. On purifie ensuite par chromatographie sur silice (éluant : dichlorométhane).

De cette manière, on obtient 7,60 g de composé désiré.
Rendement : 55,4 %

### F. Acide 2-butyl-3-[4-(3-bromopropyl)benzoyl]-1-benzofurane-6-carboxylique

Dans un mélange de 100 ml de dioxanne, 30 ml de méthanol et 30 ml d'eau, on dissout 7,27 g (environ 16 mmoles) de 2-butyl-3-[4-(3-bromopropyl)benzoyl]-1-benzofurane-6-carboxylate de méthyle et 1,28 g (2 équivalents) d'hydroxyde de sodium. On agite durant 8 heures à température ambiante, on évapore et on reprend dans l'eau. On acidifie jusqu'à pH = 1 à 2 et on extrait avec de l'acétate d'éthyle. On lave avec de l'eau et une solution saturée de chlorure de sodium.

De cette manière, on obtient le composé désiré que l'on utilise sous forme brute.

### G. 2-Butyl-3-[4-(3-bromopropyl)benzoyl]-1-benzofurane-6-carboxylate d'isopropyle

Dans 100 ml de toluène, on dissout l'acide 2-butyl-3-[4-(3-bromopropyl)benzoyl]-1-benzofurane-6-carboxylique obtenu sous forme brute à l'étape précédente puis on ajoute, goutte à goutte, 4,5 ml de chlorure d'oxalyle dans 15 ml de toluène. On chauffe le milieu réactionnel à 80°C durant 2 heures puis on évapore. On reprend dans 100 ml d'isopropanol et on porte à reflux durant 2 heures. On évapore, on reprend dans l'eau et on extrait avec de l'éther diéthylique. On lave ensuite avec une solution saturée de chlorure de sodium et on purifie par chromatographie sur silice (éluant : dichlorométhane).

De cette manière, on obtient 5,47 g de composé désiré.
Rendement : 77,6 %

### H. Oxalate de 2-buty3-[4-[3-(dibutylamino)propyl]benzoyl]-1-benzofurane-6-carboxylate d'isopropyle

Dans 50 ml d'acétonitrile, on dissout 2,7 g (environ 6 mmoles) de 2-butyl-3-[4-(3-(bromopropyl)benzoyl]-1-benzofurane-6-carboxylate d'isopropyle, 2,66 g (3 équivalents) de dibutylamine, 1,03 g (1 équivalent) d'iodure de sodium et 2,87 g (3 équivalents) de carbonate de potassium. On porte le milieu réactionnel à reflux durant 40 heures, on évapore et on reprend dans l'eau. On extrait avec de l'éther diéthylique et on lave avec de l'eau et une solution saturée de chlorure de sodium. On purifie alors par chromatographie sur silice (éluant : dichlorométhane/méthanol/ ammoniaque 100/2,5/0,2), ce qui fournit 2,35 g (rendement: 73,5 %) de composé désiré sous forme basique. On mélange ensuite dans l'acétone 2,23 g de composé basique ainsi obtenu et 0,376 g d'acide oxalique. On évapore, on reprend dans l'éther diéthylique, on filtre et on sèche sous vide.

De cette manière, on obtient 2,15 g de composé désiré.
Rendement : 82,5 %
P.F.: 104-105°C

### EXEMPLE 23

### Oxalate de 2-butyl-3-[4-[3-(dibutylamino)propyl]benzoyl]-1-benzofurane-4-carboxylate d'isopropyle

### A. 2-Hvdroxy-3-méthoxy-benzoate de méthyle

On introduit 25,5 g (0,152 mole) d'acide 2-hydroxy-3-méthoxy-benzoique dans 250 ml de méthanol contenant 1ml d'acide sulfurique. On porte l'ensemble au reflux pendant 4 jours, on concentre à sec puis on extrait avec de l'acétate d'éthyle. On lave ensuite avec de l'eau, une solution de carbonate de sodium à 10%, de l'eau et une solution de chlorure de sodium.

De cette manière, on obtient 25,2 g de composé désiré.
Rendement : 91,2%
P.F. : 68-69°C

### B. 2-Trifluorométhanesulfonyloxy-3-méthoxy-benzoate de méthyle

On introduit dans 200 ml de dichlorométhane 25 g (0,137 mole) de composé obtenu à l'étape précédente et on ajoute 11,93 g (0,151 mole) de pyridine. On additionne alors, à une température de 0°C à 5°C, un mélange de 42,6 g (0,151 mole) d'anhydride triflique dans 200 ml de dichlorométhane. On agite à température ambiante pendant 3 heures, on concentre à sec et on extrait à l'éther diéthylique. On lave alors avec de l'eau, de l'acide chlorhydrique dilué, de l'eau, une solution diluée de bicarbonate sodique, de l'eau et une solution de chlorure de sodium. On purifie alors par chromatographie sur silice (éluant : dichlorométhane/heptane 5/5).

De cette manière, on obtient 19,36 g de composé désiré.
Rendement : 54,9%

### C. 2-(Hexvn-1-yl)-3-méthoxy-benzoate de méthyle

On introduit 19,26 g (74,9 mmoles) de composé obtenu à l'étape précédente dans 200ml de N,N-diméthylformamide puis on ajoute 12,3 g (16,85 ml ; 149,7 mmoles) de 1-hexyne, 37,94 g (52,2ml; 375 mmoles) de triéthylamine et 2,62 g (3,74 mmoles) de dichloro bis-(triphénylphosphine)palladium. On chauffe à 90°C pendant 3 heures, on dilue alors avec de l'eau et on extrait avec de l'éther diéthylique. On lave alors avec de l'acide chlorhydrique dilué, de l'eau et une solution de chlorure de sodium. On purifie alors par chromatographie sur silice (éluant : dichlorométhane/heptane 5/5 puis dichlorométhane).

De cette manière on obtient 6,1 g de composé souhaité.
Rendement: 33,1%

### D. 2-Butyl-1-benzofurane-4-carboxylate de méthyle

On introduit 5,45 g (22,1 mmoles) de composé obtenu à l'étape précédente, dans 100 ml de dichlorométhane. A une température d'environ -5°C, on ajoute alors 44,5 ml d'une solution molaire de tribromure de brome dans du dichlorométhane. On agite à température ambiante pendant 4 heures puis on ajoute de l'eau avec précaution tout en maintenant la température inférieure à 30°C. On extrait ensuite avec du dichlorométhane et on purifie par chromatographie sur silice (éluant : dichlorométhane).

De cette manière, on obtient 2 g du composé souhaité.
Rendement : 39%

### E. 2-Butyl-3-[4-(3-bromopropyl)benzoyl]-1-benzofurane-4-carboxylate de méthyle

On introduit 5,23 g (32,2 mmoles) de chlorure ferrique dans 150 ml de 1,2-dichloréthane puis on ajoute, goutte à goutte, et à une température inférieure à 15°C, une solution de 5,0 g (21,5 mmoles) de 2-butyl-1-benzofurane-4-carboxylate de méthyle dans 50 ml de 1,2-dichloréthane. On additionne alors, goutte à goutte et à une température inférieure à 15°C, une solution de 8,45 g (32,2 mmoles) de chlorure de 4-(3-bromopropyl)benzoyle dans 50 ml de 1,2-dichloréthane et on agite le milieu réactionnel durant 18 heures à température ambiante. On verse sur un mélange eau/glace et on extrait avec du dichlorométhane. On lave avec de l'eau jusqu'à neutralité puis avec une solution de chlorure de sodium. On purifie alors par chromatographie sur silice (éluant : dichlorométhane).

De cette manière, on obtient 7,4 g de composé désiré.
Rendement : 75,3 %

### F. 2-Butyl-3-[4-[3-(dibutylamino)propyl]benzoyl]-1-benzofurane-4-carboxylate de méthyle

On introduit 7,4 g (16,2 mmoles) de 2-butyl-3-[4-(3-bromopropyl)benzoyl]-1-benzofurane-4-carboxylate de méthyle dans 200 ml d'acétonitrile puis on ajoute 2,3 g (17,8 mmoles) de dibutylamine, 2,67 g (17,8 mmoles) d'iodure de sodium et 4,48 g (32,4 mmoles) de carbonate de potassium. On porte le milieu réactionnel à reflux durant 18 heures, on concentre et on extrait avec de l'éther diéthylique. On lave avec de l'eau et avec une solution de chlorure de sodium. On purifie ensuite par chromatographie sur silice (éluant: dichlorométhane/méthanol/ammoniaque 97/3/0,1).

De cette manière, on obtient 5,24 g de composé désiré.
Rendement : 63,96 %

### G. Acide 2-butyl-3-[4-[3-(dibulylamino)propyl]benzoyl]-1-benzofurane-4-carboxylique

On introduit 5,24 g (10,36 mmoles) de 2-butyl-3-[4-[3-(dibutylamino)propyl]benzoyl]-1-benzofurane-4-carboxylate de méthyle dans un mélange de 60 ml de dioxane et de 10 ml de méthanol. On ajoute alors 0,829 g (20,72 mmoles) d'hydroxyde de sodium et 10 ml d'eau. On agite à température ambiante durant 18 heures, on concentre à sec et on reprend dans l'eau. On acidifie avec de l'acide chlorhydrique dilué jusqu'à pH=6 et on extrait avec de l'acétate d'éthyle. On lave alors avec de l'eau et une solution de chlorure de sodium.

De cette manière, on obtient 4,48 g de composé désiré.
Rendement : 88 %

### H. Oxalate de 2-butyl-3-[4-[3-(dibutylamino)propyl]benzoyl]-1-benzofurane-4-carboxylate d'isopropyle

On introduit 4,48 g (9,11 mmoles) d'acide 2-butyl-3-[4-[3-(dibutylamino)propyl] benzoyl]-1-benzofurane-4-carboxylique dans 50 ml de toluène puis on ajoute une solution de 4,5 ml de chlorure d'oxalyle dans 20 ml de toluène. On agite le milieu réactionnel à température ambiante puis on le porte à 80°C pendant 2 heures. On concentre à sec, on reprend dans l'éther diéthylique et on concentre. On introduit alors dans 300 ml d'isopropanol le chlorure d'acyle ainsi formé et on porte le milieu réactionnel à reflux durant 48 heures. On concentre à sec et on extrait avec de l'éther diéthylique. On lave alors avec de l'eau, une solution d'hydrogénocarbonate sodique, de l'eau et une solution saturée de chlorure de sodium. On purifie ensuite par chromatographie sur silice (éluant : dichlorométhane/méthane/ammoniaque 97/3/0,1), ce qui fournit 1,183 g (rendement : 24,3 %) de composé désiré sous forme de base libre.
On dissout alors dans du méthanol 1,137 g (2,13 mmoles) de composé basique ainsi obtenu et on ajoute 0,192 g (1 équivalent) d'acide oxalique. On concentre à sec, on triture dans l'éther diéthylique, on essore et on sèche.

De cette manière, on obtient 1,094 g de composé désiré.
Rendement : 80 %
P.F. : 120-122°C

### EXEMPLE 24

### Oxalate de 2-butyl-3-[4-[4-(dibutylamino)butyl]benzoyl]-1-benzofurane-5-carboxylate d'isopropyle

### A. 1-Chloro-4-(4-acétylphényl)butane

Dans un ballon tricol, on introduit 58 ml de dichlorométhane puis on ajoute 16,21 g (0,122 mole) de chlorure d'aluminium et 9,8 g (0,125 mole) de chlorure d'acétyle. On additionne ensuite goutte à goutte et à une température de 0° à 5°C, 20 g (0,118 mole) de 4-chlorobutyl-benzène et 20,3 g (0,26 mole) de chlorure d'acétyle. On agite durant 2 heures dans un bain de glace puis on verse le milieu réactionnel sur un mélange glace/eau/acide chlorhydrique. On agite durant 15 minutes puis on extrait avec du dichlorométhane. On lave ensuite avec de l'eau jusqu'à pH neutre puis avec du chlorure de sodium.

De cette manière, on obtient 20,6 g de composé désiré.
Rendement : 83 %
P.E. : 118-126°C sous 0,05 mm Hg

### B. Acide 4-(4-chlorobutyl)benzoïque

On dissout 32,2 g (0,8 mole) d'hydroxyde de sodium dans 252 ml d'eau et on ajoute 178 ml de dioxane. On additionne ensuite, goutte à goutte et à 0°C, 15 ml (0,293 mole) de brome puis, entre -5°C et 0°C, on ajoute 20,6 g (0,097 mole) de composé obtenu à l'étape précédente. On maintient le milieu réactionnel à 0°C durant 2 heures puis on ajoute de l'acide chlorhydrique jusqu'à pH acide. On extrait avec du dichlorométhane puis on lave avec de l'eau et une solution de chlorure de sodium. On cristallise ensuite dans l'éther diéthylique.

De cette manière, on obtient 16,1 g de composé désiré.
Rendement : 68 %

### C. Chlorure de 4-(4-chlorobutyl)benzoyle

On dissout 14,65 g (0,0688 mole) de composé obtenu à l'étape précédente dans 60 ml de 1,2-dichloréthane. On ajoute alors 15 ml de chlorure de thionyle, on porte à reflux durant 3 heures et on évapore.

De cette manière, on obtient 7,45 g de composé désiré.
Rendement : 47 %
P.E. : 129-130°C sous 0,05 mm Hg

### D. 2-Bulyl-3-[4-(4-bromobulyl)benzoyl]-1-benzofurane-5-carboxylate de méthyle

On introduit 5,23 g de chlorure ferrique dans 122 ml de 1,2-dichloréthane et on ajoute, à une température inférieure ou égale à 15°C, 7,45 g (0,0322 mole) de 2-butyl-1-benzofurane-5-carboxylate de méthyle dans 61 ml de 1,2-dichloréthane. On additionne ensuite 5 g (0,0217 mole) de composé obtenu à l'étape précédente dans 61 ml de dichlorométhane. On maintient le milieu réactionnel à température ambiante durant 18 heures puis on verse sur un mélange glace/eau. On extrait avec du dichlorométhane puis on lave avec de l'eau, de l'acide chlorhydrique dilué, une solution d'hydrogénocarbonate de sodium, de l'eau et une solution saturée de chlorure de sodium. On purifie ensuite par chromatographie sur silice (éluant : dichlorométhane).

De cette manière, on obtient 7 g de composé désiré.
Rendement : 76 %

### E. Acide 2-butyl-3-[4-(4-bromobutyl)benzoyl]-1-benzofurane-5-carboxylique

Dans 127 ml de dioxane, on dissout 6,7 g (0,0157 mole) de composé obtenu à l'étape précédente et on ajoute 1,256 g (0,0314 mole) d'hydroxyde de sodium dissous dans 30 ml d'eau. On ajoute 30 ml de méthanol et on maintient le milieu réactionnel à température ambiante durant 18 heures. On évapore et on acidifie avec de l'acide chlorhydrique jusqu'à pH=2. On extrait avec de l'acétate d'éthyle. On lave ensuite avec de l'eau puis une solution saturée de chlorure de sodium.

De cette manière, on obtient 7,06 g de composé désiré.
Rendement: 100 %

### F. 2-Butyl-3-[4-(4-bromobutyl)benzoyl]-1-benzofurane-5-carboxylate d'isopropyle

Dans 90 ml de toluène, on dissout 7,06 g (0,0175 mole) de composé obtenu à l'étape précédente et on ajoute goutte à goutte, à température ambiante, 6 ml de chlorure d'oxalyle dissous dans 18 ml de toluène. On porte à reflux puis on évapore. On reprend dans l'éther diéthylique et on évapore. On reprend à nouveau dans 100 ml d'isopropanol et on porte à reflux. On évapore et on purifie par chromatographie sur silice (éluant : dichlorométhane).

De cette manière, on obtient 3,94 g de composé désiré.
Rendement : 50 %

### G. Oxalate de 2-butyl-3-[4-[4-(dibutylamino)butyl]benzoyl]-1-benzofurane-5-carboxylate d'isopropyle

Dans 80 ml d'acétonitrile, on dissout 3,7 g (0,0081 mole) de composé obtenu à l'étape précédente puis on ajoute 3,14 g (0,0243 mole) de dibutylamine, 1,21 g (0,0081 mole) d'iodure de sodium et 3,36 g (0,0243 mole) de carbonate de postassium. On porte à reflux puis on évapore. On reprend dans l'éther diéthylique puis on lave avec de l'eau et une solution de chlorure de sodium. On purifie ensuite par chromatographie sur silice (éluant : dichlorométhane/méthanol/ammoniaque 98/2/0,1), ce qui fournit 2 g (rendement : 45 %) de composé désiré sous forme basique.
Dans de l'éthanol absolu, on dissout alors 1,59 g (0,0029 mole) du composé basique ainsi obtenu et on ajoute une solution de 0,261 g (0,0029 mole) d'acide oxalique. On évapore et on reprend dans l'éther diéthylique. On cristallise ensuite dans l'éther diéthylique.

De cette manière, on obtient 1,55 g de composé désiré.
Rendement: 84 %
P.F. : 79-80°C

### EXEMPLE 59

### Oxalate de 2-Butyl-3-[4-(3-dibutylamino-propyl)-benzoyl]benzofuran-5-carboxylic acid methoxy-methyl-amide

### A. Chlorure d'acide 3-[4-(3-Bromo-propyl)-benzoyl]-2-butyl-benzofuran-5-carboxylique

On mélange 2,8 g d'acide 3-[4-(3-Bromo-propyl)-benzoyl]-2-butyl-benzofuran-5-carboxylique, 2,8 ml de SOCl₂ (chlorure de thionyle) et 30 ml de DCM (dichlorométhane) que l'on porte au reflux pendant 3 heures. On évapore et on reprend plusieurs fois à l'éther pour éliminer le SOCl₂ en excès. On évapore à sec.

De cette manière, on obtient 3 g du composé désiré.

### B. 3-[4-(3-Bromo-propyl)-benzoyl]-2-butyl-benzofuran-5-carboxylic acid methoxy-methyl-amide

On ajoute à froid 1,9 g de diméthylhydroxylamine à 3 g du composé obtenu à l'étape A précédente dans du DCM. On ajoute goutte à goutte 1,9 g de diisopropyléthylamine (DIPEA) dans du DCM, en 20 minutes. On laisse revenir à température ambiante. On évapore le DCM puis on reprend avec de l'eau et de l'acétate d'éthyle. On extrait trois fois à l'acétate d'éthyle puis on lave deux fois à l'eau puis avec une solution aqueuse de NaCl. On sèche sur Na₂SO₄, on concentre, puis on purifie par chromatographie sur silice (éluant : acétate d'éthyle/DCM).

De cette manière, on obtient 2,2 g du composé désiré.

### C. 2-Butyl-3-[4-(3-dibutylamino-propyl)-benzoyl]-benzofuran-5-carboxylic acid methoxy-methyl-amide

On mélange 2,2 g du composé obtenu à l'étape B précédente, 3 équivalents molaire de dibutylamine, 750 mg d'iodure de sodium, 2,3 g de carbonate de potassium et 50 ml d'acétonitrile. On porte au reflux pendant 16 heures. La réaction n'étant pas terminée, on ajoute un équivalent de dibutylamine (3ml au total pour cette réaction) et on laisse réagir 16 heures au reflux. On évapore à sec et on reprend avec de l'eau et de l'éther. On extrait trois fois à l'éther et on lave à l'eau et une solution aqueuse de NaCl saturée. On sèche sur Na₂So₄ et on concentre, puis on purifie par chromatographie sur silice (éluant : acétate d'éthyle/DCM, jusqu'à 30 % d'acétate d'éthyle puis : méthanol/DCM).

De cette manière, on obtient 1,977 g de composé désiré.

### E. Oxalate de 2-Butyl-3-[4-(3-dibutylamino-propyl)-benzoyl]-benzofuran-5 carboxylic acid methoxy-methyl-amide

On mélange 1,977 g du composé obtenu à l'étape précédente C avec un équivalent molaire d'acide oxalique dans du méthanol. On évapore ensuite le méthanol et on triture dans l'éther. On filtre.

De cette manière, on obtient 1,3 g du composé désiré.

### EXEMPLE 74

### Oxalate de 2-Butyl-3-[4(3-dibutylamino-2-hydroxy-propyl)-benzoyl]-benzofuran-5-carboxylate d'isopropyle

### A. Acide 3-(4-Allyl-benzoyl)-2-butyl-benzofuran-5-carboxylique

On ajoute 1,7 g de NaOH en pastilles à 8,1 g de 3-(4-Allyl-benzoyl)-2-butyl-benzofuran-5-carboxylate de méthyle dans 40 ml de méthanol, 200 ml de dioxane et 40 ml d'eau. On agite à température ambiante pendant 24 heures. On neutralise à pH 7 avec de l'acide chlorhydrique concentré puis on évapore. On ajoute de l'eau et on régénère l'acide avec de l'acide chlorhydrique concentré. On extrait trois fois à l'éther, on lave à l'eau puis avec une solution aqueuse de NaCl. On sèche sur Na₂SO₄ puis on concentre.

On obtient ainsi 7,5 g de produit brut utilisé tel quel pour l'étape suivante.

### B. 3-(4-Allyl-benzoyl)-2-butyl-benzofuran-5-carboxylate d'isopropyle

On ajoute 7,5 ml de SOCl₂ à 7,5 g du produit obtenu à l'étape A précédente dans 100 ml de DCM. On chauffe 3 heures à 45° C puis on évapore. On reprend à l'éther puis on évapore à sec. On ajoute ensuite 150 ml d'isopropanol et on porte au reflux à 100° C pendant 2 heures. On évapore. On reprend dans de l'eau et du DCM. On extrait au DCM et on lave à l'eau. On sèche sur Na₂SO₄ puis on concentre et on purifie par chromatographie sur silice (éluant : hexane/DCM).

De cette manière, on obtient 5,55 g de produit brut utilisé tel quel pour l'étape suivante.

### C. 2-Butyl-3-(4-oxiranylmethyl-benzoyl)-benzofuran-5-carboxylate d'isopropyle

A température ambiante, on ajoute 3,3 g d'acide 3-chloroperbenzoïque à 5,55 g du produit obtenu à l'étape B précédente dans 50 ml de DCM. On agite à température ambiante pendant 16 heures. On ajoute encore 660 mg (20 %) d'acide 3-chloroperbenzoïque et on agite à température ambiante pendant 60 heures. On filtre l'insoluble puis on lave à l'eau le filtrat. On sèche sur Na₂SO₄, on concentre puis on purifie par chromatographie sur silice (éluant : hexane/DCM).

De cette manière, on a obtenu 1,5 g du composé désiré.

### D. 2-Butyl-3-[4-(3-dibutylamino-2-hydroxy-propyl)-benzoyl]-benzofuran-5]-carboxylate d'isopropyle

On mélange 1,5 g du composé obtenu à l'étape C précédente avec 1,5 g de dibutylamine et 30 ml d'acétonitrile, que l'on porte à 60° C pendant 60 heures. On évapore et on met directement sur colonne silice pour purification (éluant : DCM/Acétate d'éthyle puis DCM/méthanol).

De cette manière, on obtient 899 mg de composé désiré.

### E. Oxalate de 2-Butyl-3-[4(3-dibutylamino-2-hydroxy-propyl)-benzoyl]-benzofuran-5-carboxylate d'isopropyle

On dissout 134 mg d'acide oxalique et 899 mg du composé obtenu à l'étape précédente D dans de l'isopropanol. On mélange et on évapore. On reprend et on triture dans l'éther. On filtre.

De cette manière, on obtient 771 mg du composé désiré.

### EXEMPLE 89

### Oxalate de 2-Butyl-3-[4-(2-dibutylamino-ethoxymetyl)-benzoyl]-benzofuran-5-carboxylate d'isopropyle

### A. 3-(4-Bromomethyl-benzoyl)-2-butyl-benzofuran-5-carboxylate de méthyle

A 25 g d'acide 4-bromométhyl-benzoïque, on ajoute 60 ml de SOCl₂ (chlorure de thionyle) et 390 ml de DCM (dichlorométhane). On chauffe à 50° C pendant 4 heures et 30 minutes. On évapore et on reprend à l'éther diéthylique. On obtient ainsi : 27,5 g du chlorure d'acide auquel on ajoute à froid 20 ml de SnCl₄, 400 ml d'EtCl₂ (1,2-dichloroéthane) et 14 g de 2-butyl-benzofurane-5-carboxylate de méthyle. On chauffe à 49° C pendant 18 heures. On verse sur de la glace. On extrait au DCM à trois reprise. On lave à l'eau et au NaHCO₃. On sèche sur Na₂SO₄. On concentre puis on purifie par chromatographie sur silice (éluant : hexane/DCM).

De cette manière, on obtient 16,85 g de composé désiré.

### B. 2-Butyl-3-[4-(2-chloro-ethoxymethyl)-benzoyl]-benzofuran-5-carboxylate de méthyle et 2-Butyl-3-[4-(2-chloro-ethoxymethyl)-benzoyl]-benzofuran-5-carboxylate de 2-chloroéthyle

On mélange 3,5 g du composé obtenu à l'étape précédente A, 3,35 g de AgClO₄ et 25 ml de 2-chloroéthanol, que l'on chauffe à 90° C pendant 1 heure. On ajoute ensuite du DCM puis on filtre. On lave à l'eau puis on sèche sur Na₂SO₄. On concentre puis on purifie par chromatographie sur silice (éluant : hexane/DCM).

On obtient ainsi 2,5 g d'un produit dont le composé désiré.

### C. Acide 2-Butyl-3-[4-(2-chloro-ethoxymethyl)-benzoyl]-benzofuran-5-carboxylique

On mélange 2,5 g du produit obtenu à l'étape précédente B, 10 ml de méthanol, 50 ml de dioxane, 10 ml d'eau et 466 mg de NaOH. On agite 16 heures à température ambiante. On ajoute de l'eau puis on extrait les impuretés à l'éther. On régénère l'acide avec de l'acide chlohydrique concentré jusqu'à obtenir un pH acide. On extrait ensuite trois fois à l'acétate d'éthyle, on lave à l'eau puis avec une solution aqueuse de NaCl saturée. On sèche sur Na₂SO₄ puis on concentre.

De cette manière, on obtient 2,2 g du composé désiré.

### D. 2-Butyl-3-[4-(2-chloro-ethoxymethyl)-benzoyl]-benzofuran-5-carboxylate d'isopropyle

On mélange 2,2 g du composé obtenu à l'étape précédente C, 3 ml de SOCl₂, 40 ml de DCM que l'on chauffe à 45° C pendant 1 heure 30 minutes. On évapore, on reprend à l'éther puis on concentre à sec. On obtient ainsi 2,1 g de produit que l'on mélange avec 50 ml d'isopropanol. On porte au reflux pendant 2 heures puis on évapore et on purifie par chromatographie sur silice (éluant : hexane/DCM).

De cette manière, on obtient 1,68 g du composé désiré.

### E. 2-Butyl-3-[4-(2-dibutylamino-ethoxymethyl)-benzoyl]-benzofuran-5-carboxylate d'isopropyle

On mélange 1,68 g du composé obtenu à l'étape précédente D, 551 mg d'iodure de sodium, 1,85 ml de dibutylamine, 1,52 g de carbonate de potassium et 40 ml d'acétonitrile. On chauffe à 110° C pendant 48 heures. On reprend dans de l'eau et de l'acétate d'éthyle. On extrait trois fois à l'acétate d'éthyle et on lave à l'eau. On sèche sur Na₂SO₄ puis on concentre et on purifie par chromatographie sur silice (éluant : méthanol/DCM).

De cette manière, on obtient 1,6 g du composé désiré.

### F. Oxalate de 2-Butyl-3-[4-(2-dibutylamino-ethoxymethyl)-benzoyl]-benzofuran-5-carboxylate d'isopropyle

On mélange 1,6 g du composé obtenu à l'étape précédente E avec 262 mg d'acide oxalique dans le méthanol. On évapore le solvant, on triture dans l'éther puis on filtre.

On obtient ainsi 1,39 g du composé désiré.

### EXEMPLE 97

### Oxalate de 3-[4-(3-dibutylamino-propyl)-benzoyl]-2-(4-hydroxy-butyl)-benzofurane-5-carboxylate d'isopropyle

### A. tert-Butvl-hex-5-ynyloxy-diphényl-silane

Dans 100 ml de DMF (*N*,*N*-diméthylformamide), 15,91 g (0,162 mole) d'Hex-5-yn-1-ol et 24,27 g (0,356 mole) d'imidazole on introduit, à température ambiante, 49,1g (0,178 mole) de chlorure de *tert*-Butyl-diphenyl-silane dans 100 ml de DMF. On agite à température ambiante pendant 4heures puis on verse dans l'eau et on extrait à l'acétate d'éthyle suivi d'un lavage à l'eau.

De cette manière, on obtient 59,19 g de composé désiré.

### B. 2-[4-(tert-Butyl-diphényl-silanyloxy)-butyl]-benzofurane-5-carboxylate de méthyle

On mélange 24,2 g (0,0719 mole) du composé obtenu à l'étape A précédente, 10 g (0,036 mole) de 4-hydroxy-3-iodo-benzoate de méthyle, 400 ml de DMF, 32,8 ml de pipéridine, 819 mg de dichloro bis-(triphénylphosphine)palladium et 210 mg d'iodure cuivreux. On chauffe ce mélange à 90° C pendant 4 heures. On concentre à sec, on extrait ensuite à l'acétate d'éthyle puis on lave à l'eau, une solution de chlorure de sodium et de l'acide chlorhydrique dilué, puis on purifie par chromatographie sur silice (éluant : DCM).

De cette manière, on obtient 13,50 g de composé désiré.
Rendement: 77,1%

### C. 3-[4-(3-Bromo-propyl)benzoyl]-2-{4-[4-(3-Bromo-propyl)benzoyloxy]-butyl}-benzofurane-5-carboxylate de méthyle

A un mélange de 6,62 g (0,0497 moles) de chlorure d'aluminium et de 100 ml de EtCl₂ (1,2-dichloroéthane), on additionne 8,06 g (0,0166 moles) du composé obtenu à l'étape précédente B dissous dans 200ml de EtCl₂, puis 13,06 g (0,0497 moles) de chlorure d'acide 4-(3-bromopropyl)-benzoïque et 100 ml de EtCl₂. On porte à 35 °C pendant 72 heures. On verse ensuite sur de l'eau et glace, puis on extrait au DCM et on lave à l'eau, puis on purifie par chromatographie sur silice (éluant : DCM et méthanol à 98/2).

De cette manière on obtient 3,3 g de composé souhaité.
Rendement : 28,46%

### D. Acide 3-[4-(3-bromo-propyl)benzoyl]-2-(4-hydroxy-butyl)-benzofurane-5-carboxylique

A un mélange de 3,30 g (0,0472 moles) de composé obtenu à l'étape précédente C, 50 ml de dioxane, et quelques ml de méthanol pour parfaire la dissolution, on ajoute 759 mg de NaOH et 10 ml d'eau. On agite à température ambiante pendant 4 heures. On concentre à sec. On reprend dans l'eau distillée. On acidifie avec de l'acide chlorhydrique dilué. On extrait ensuite à l'acétate d'éthyle et on lave à l'eau, puis on purifie par chromatographie sur silice (éluant : DCM/méthanol).

De cette manière on obtient 1,156 g de composé souhaité.
Rendement : 53,3%

### E. 3-[4-(3-Bromo-propyl)benzoyl]-2-(4-hydroxy-butyl)-benzofurane-5-carboxylate d'isopropyle

On mélange 1,094 g (0,0238 moles) du composé obtenu à l'étape D précédente, 50 ml d'acétonitrile, 1,156 g (0,0357 moles) de carbonate de césium et 4,06 g (0,0238 moles) d'iodure d'isopropyle que l'on porte au reflux pendant 2 heures et 30 minutes. On filtre sur fritté puis on concentre à sec le filtrat. On reprend dans de l'acétate d'éthyle le produit concentré. On lave à NaHCO₃ dilué, à l'eau et une solution de NaCl, puis on purifie par chromatographie sur silice (éluant: DCM/méthanol).

De cette manière, on obtient 699 mg de composé désiré.
Rendement : 58,6%

### F. 3-[4-(3-dibutylamino-propyl)-benzoyl]-2-(4-hydroxy-butyl)-benzofurane-5-carboxylate d'isopropyle

On mélange 869 mg (0,00173 mole) du composé obtenu à l'étape E précédente, 20 ml d'acétonitrile, 672 mg (0,0052 moles) de dibutylamine, 718 mg (0,0052 moles) de carbonate de potassium et 259 mg (0,0073 moles) d'iodure de sodium que l'on porte au reflux pendant 5 heures. On concentre à sec puis on extrait à l'acétate d'éthyle.On lave à l'eau et avec une solution saturée de NaCl, puis on purifie par chromatographie sur silice (éluant : DCM/méthanol/NH₄OH).

De cette manière, on obtient 482 mg de composé désiré.
Rendement: 50,67 %

### G. Oxalate de 3-[4-(3-(dibutylamino-propyl)-benzoyl]-2-(4-hydroxy-butyl)-benzofurane-5-carboxylate d'isopropyle

On réalise un mélange comprenant 443 mg (0,806 mmoles) du composé obtenu à l'étape F précédente, une quantité suffisante de méthanol pour une dissolution totale et 73 mg (0,806 mmoles) d'acide oxalique. On concentre à sec et on met sous éther isopropylique. On essore et on sèche.

De cette manière, on obtient 463 mg de composé désiré.
Rendement : 89,8 %
En utilisant les procédés décrits aux exemples précédents, on a préparé les composés listés ci-après. En ce qui concerne les composés des exemples 25, 32, 33, 42, 45, 46 et 58, on a listé les résultats des spectres R.M.N.

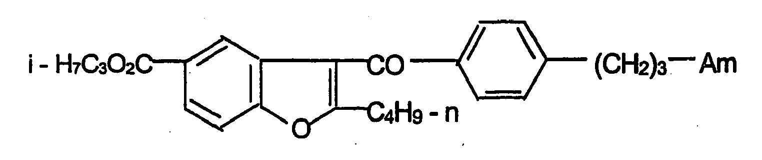

| **Am** | **Caractéristiques** | **Exemple** |
|---|---|---|
| - N(C₂H₅)₂ | Oxalate | 25 |
| | Solide amorphe | |
| - N(C₃H₇)₂ | Chlorhydrate | 26 |
| | solide blanc | |
| | P.F. : 121-122°C | |
| - N(C₅H₁₁)₂ | Oxalate | 27 |
| | P.F. : 123-125°C | |
| - N(i-C₄H₉)₂ | Oxalate | 28 |
| | solide blanc | |
| | P.F. : 123°C | |
| - N (i-C₅H₁₁)₂ | Oxalate | 29 |
| | solide blanc | |
| | P.F. : 126°C | |
| - NHC₄H₉ | Chlorhydrate | 30 |
| | P. F. : 122°C | |
| 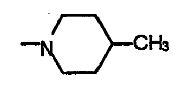 | Oxalate | 31 |
| | P.F. : 150°C | |
| 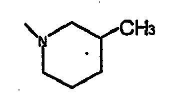 | Chlorhydrate | 32 |
| | Solide | |
| 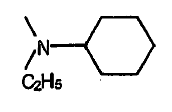 | Oxalate | 33 |
| | Poudre amorphe | |
| 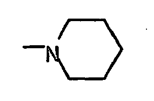 | Chlorhydrate | 34 |
| | P.F. : 149°C | |
| 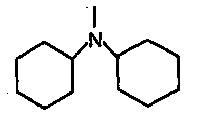 | Oxalate | 35 |
| | solide blanc | |
| | P.F. : 127°C | |
| 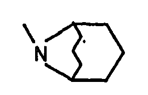 | Chlorhydrate | 36 |
| | solide blanc | |
| | P.F. : 209°C | |
| 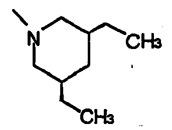 | Chlorhydrate | 37 |
| | solide blanc | |
| | P.F. : 135°C | |
| 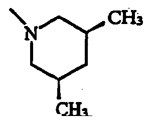 | Chlorhydrate | 38 |
| | Poudre blanche | |
| | P.F. : 167°C | |
| 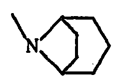 | Chlorhydrate | 39 |
| | P.F. : 146°C | |
| 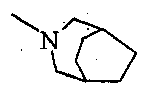 | Oxalate | 40 |
| | solide blanc | |
| | P.F. : 160°C | |
| 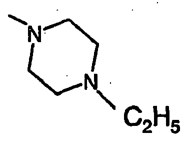 | Dioxalate | 48 |
| | Poudre blanche | |
| | P.F. : 227-228°C | |
| 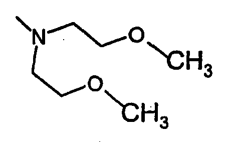 | Oxalate | 49 |
| | Poudre blanche | |
| | P.F. : 74-75°C | |
| 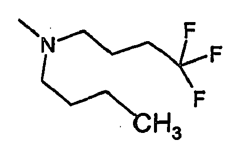 | Oxalate | 50 |
| | Poudre | |
| | P.F. : 146°C | |
| 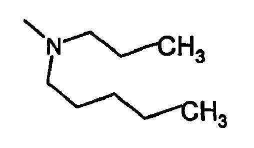 | Oxalate | 51 |
| | Solide blanc | |
| | P.F. :97-98°C | |
| 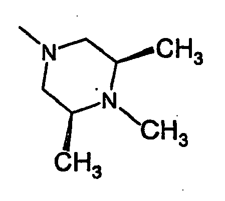 | Dioxalate | 52 |
| | Poudre | |
| | P.F. : 90-93°C | |
| 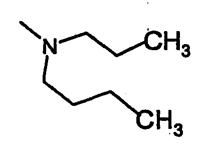 | Oxalate | 53 |
| | Solide | |
| | P.F. : 109-11°C | |
| 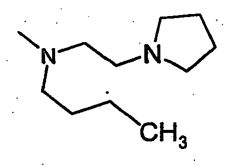 | Dioxalate | 54 |
| | Poudre | |
| 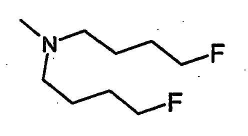 | Oxalate | 55 |
| | Poudre | |
| | P.F. : 90-93°C | |
| 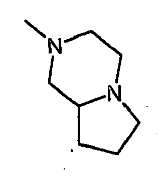 | Dioxalate | 56 |
| | Poudre | |
| | P.F. : 148°C | |

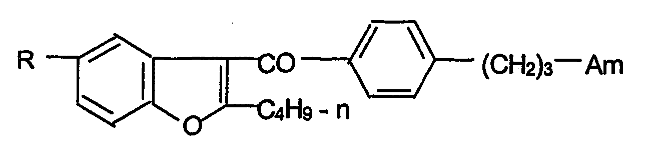

| **R** | **Am** | **Caractéristiques** | **Exemple** |
|---|---|---|---|
| H₃CO₂C - | 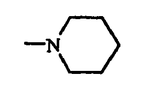 | Chlorhydrate | 41 |
| | | P.F. : 153°C | |
| H₅C₂O₂C - | - N(C₄H₉)₂ | Oxalate | 42 |
| | | solide | |
| H₇C₃O₂C - | - N(C₄H₉)₂ | Oxalate | 43 |
| | | Solide | |
| | | P.F. : 119°C | |
| 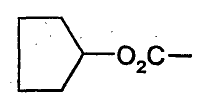 | - N(C₄H₉)₂ | Oxalate | 44 |
| | | P.F. : 132°C | |
| (H₅C₂)₂N - CO - | - N(C₄H₉)₂ | Oxalate | 45 |
| | | solide | |
| i - H₇C₃NH - CO - | - N(C₄H₉)₂ | Oxalate | 46 |
| | | solide amorphe | |
| 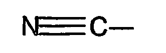 | - N(C₄H₉)₂ | Oxalate | 57 |
| | | Solide blanc | |
| | | P.F. : 129-131 °C | |
| 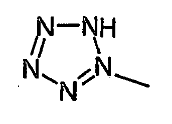 | - N(C₄H₉)₂ | Oxalate | 58 |
| | | Solide blanc | |
| | | amorphe | |
| 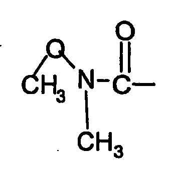 | - N(C₄H₉)₂ | Oxalate | 59 |
| | | Poudre | |
| | | P.F. : 82-83°C | |
| 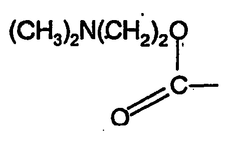 | - N(C₄H₉)₂ | Oxalate | 60 |
| | | Solide blanc | |
| | | P.F. : 138-141°C | |
| HOCH₂- | - N(C₄H₉)₂ | Oxalate | 61 |
| | | Solide | |
| | | P.F.: 117-119°C | |
| HOC - | - N(C₄H₉)₂ | Oxalate | 62 |
| | | Solide | |
| | | P.F. : 114-116°C | |
| 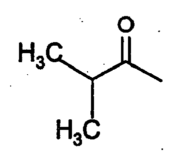 | - N(C₄H₉)₂ | Oxalate | 63 |
| | | Poudre | |
| | | P.F. : 94°C | |
| H₃CO₂C - | 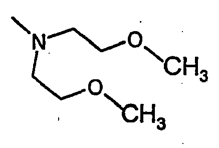 | Oxalate | 64 |
| | | Poudre | |
| | | P.F. : 107-109°C | |

On a également préparé les composés listés ci-après. En ce qui concerne les composés des exemples 81 et 92, on a également listé les résultats des spectres R.M.N.

| **Composé** | **Caractéristiques** | **Exemple** |
|---|---|---|
| 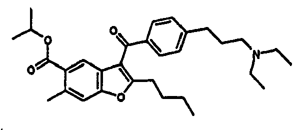 | Oxalate | 65 |
| | Solide blanc | |
| | P.F. : 155-157°C | |
| 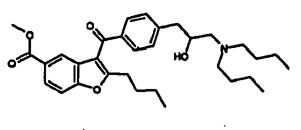 | Oxalate | 66 |
| | Poudre | |
| | P. F. : 128-130°C | |
| 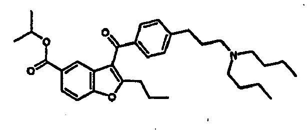 | Oxalate | 67 |
| | Solide | |
| | P.F. : 155-157°C | |
| 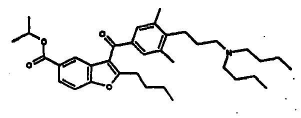 | Oxalate | 68 |
| | Solide | |
| | P.F. : 119-121°C | |
| 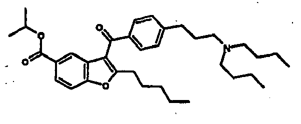 | Oxalate | 69 |
| | Solide blanc | |
| | P. F. : 124-126°C | |
| 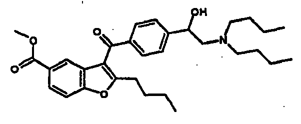 | Oxalate | 70 |
| | Solide blanc | |
| | P.F. : 124-126°C | |
| 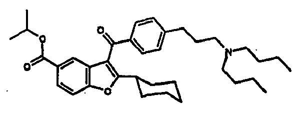 | Oxalate | 71 |
| | Solide blanc | |
| | P.F. : 124-126°C | |
| 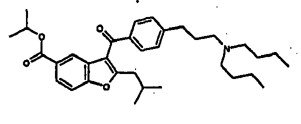 | Oxalate | 72 |
| | Solide blanc | |
| | P.F. : 105-107°C | |
| 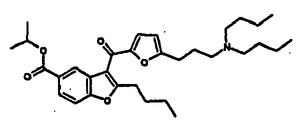 | Oxalate | 73 |
| | Solide | |
| | P.F. : 75-78°C | |
| 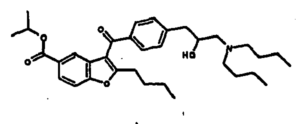 | Oxalate | 74 |
| | Solide blanc | |
| | P.F. : 90-91°C | |
| 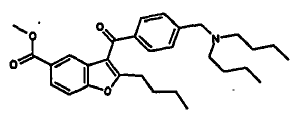 | Oxalate | 75 |
| | Solide blanc | |
| | P.F. : 96-97°C | |
| 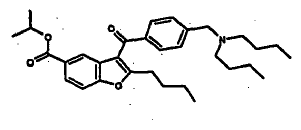 | Oxalate | 76 |
| | Solide blanc | |
| | P.F. : 107-108°C | |
| 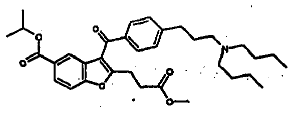 | Oxalate | 77 |
| | Poudre blanche | |
| | P.F. : 107-108°C | |
| 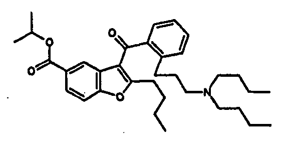 | Oxalate | 78 |
| | Poudre | |
| | P.F. : 85-88°C | |
| 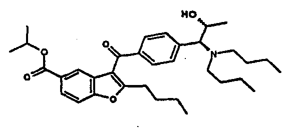 | Oxalate | 79 |
| | Poudre | |
| | P.F. : 150-151°C | |
| 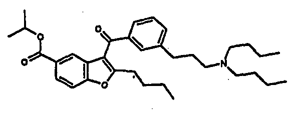 | Oxalate | 80 |
| | Solide | |
| | P.F. : 87-90°C | |
| 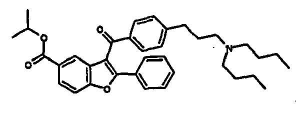 | Oxalate | 81 |
| | Solide amorphe | |
| 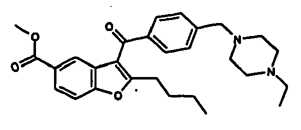 | Dioxalate | 82 |
| | Cristaux | |
| | P.F. : 220-221 °C | |
| 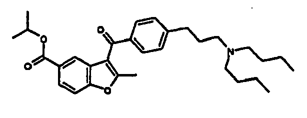 | Oxalate | 83 |
| | Solide blanc | |
| | P.F. : 107-108°C | |
| 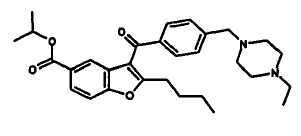 | Dioxalate | 84 |
| | Solide | |
| | P.F. : 222-224°C | |
| 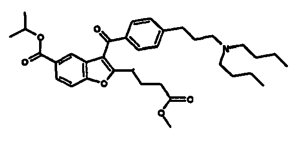 | Oxalate | 85 |
| | Poudre blanche | |
| | P.F. : 105°C | |
| 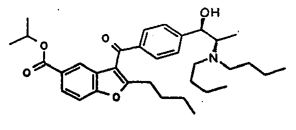 | Oxalate | 86 |
| | Solide blanc | |
| | P.F. : 107-108°C | |
| 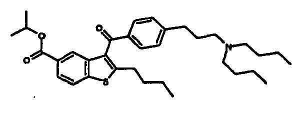 | Oxalate | 87 |
| | Solide | |
| | P.F. : 86-89°C | |
| 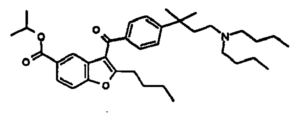 | Oxalate | 88 |
| | Solide | |
| | P.F. : 92-93°C | |
| 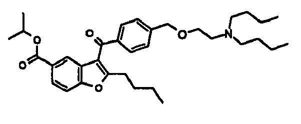 | Oxalate | 89 |
| | Poudre | |
| | P.F. : 73°C | |
| 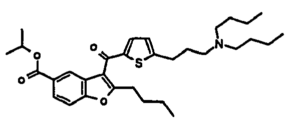 | Oxalate | 90 |
| | Poudre | |
| | P.F. : 100°C | |
| 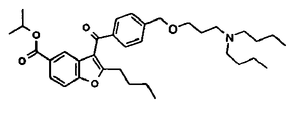 | Oxalate | 91 |
| | Poudre | |
| | P.F. : 73-74°C | |
| 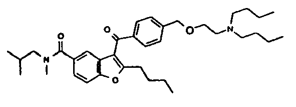 | Oxalate | 92 |
| | Poudre amorphe | |
| 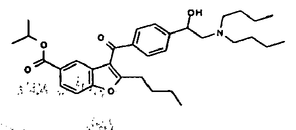 | Oxalate | 93 |
| | Poudre blanche | |
| | P.F. : 134-135°C | |
| 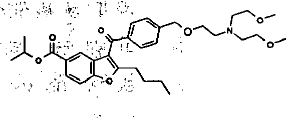 | Oxalate | 94 |
| | Poudre | |
| | P.F. : 93-94°C | |
| 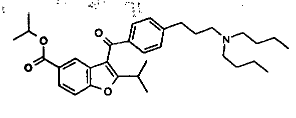 | Oxalate | 95 |
| | Solide blanc | |
| | P.F. : 106-108°C | |
| 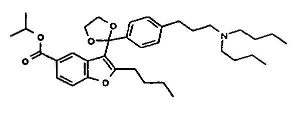 | Oxalate | 96 |
| | Solide | |
| | P.F. : 106-108°C | |
| 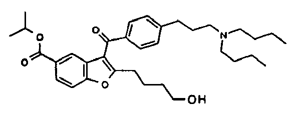 | Oxalate | 97 |
| | Solide amorphe | |
| 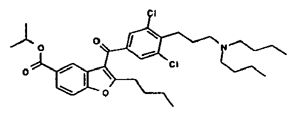 | Oxalate | 98 |
| | Solide | |
| | P.F. : 87-89°C | |

### Spectres R.M.N. ¹H à 200 MHz

### Exemple 25

Solvant :
   DMSO à 2,5 ppm
   DOH à 3,3 ppm
δ (ppm) :
   0,8 ; triplet ; 3H, 1CH₃
   1 à 1,4 ; triplet + doublet ; 12H, 4CH₃
   1,6 ; quintuplet ; 2H, 1CH₂
   2 ; multiplet ; 2H, 1CH₂
   2,8 ; multiplet ; 4H, CH₂ phényle, CH₂-CH=
   3,1 ; multiplet ; 6H ; 3NCH₂
   5,1 ; multiplet; 1H, O-CH
   7,4 ; doublet ; 2H, 2H aromatique
   7,8 ; doublet ; 3H, 3H aromatique
   8,0 ; doublet dédoublé ; 1H, 1H aromatique
   8,1 ; doublet ; 1H, 1H aromatique

### Exemple 32

Solvant :
   DMSO à 2,5 ppm
   DOH à 3,3 ppm
δ (ppm) :
   0,7 ; triplet + doublet ; 9H, 3CH₃
   1 ; multiplet ; 2H, 1CH₂
   1,1 ; doublet ; 6H, 2CH₃
   1,4 à 2 ; massif ; 9H, 4CH₂, 1 CH
   2,4 à 3,4 ; massif ; 10H, 3NCH₂, 1CH₂ phényle, 1CH₂-CH=
   5 ; 1 septuplet ; 1H, 10CH
   7,4 ; doublet ; 2H, 2H aromatique
   7,7 ; multiplet ; 3H, 3H aromatique
   7,9 ; doublet dédoublé ; 1H, 1H aromatique
   8 ; doublet; 1H, 1H aromatique

### Exemple 33

Solvant : DMSO à 2,5 ppm
δ (ppm) :
   0,85 ; triplet ; 3H, CH₃
   1 à 2,25 ; massif ; 25H, 3CH₃, 8CH₂
   2,85 ; multiplet ; 4H, 2CH₂ phényle
   3 à 3,45 ; massif ; 5H, NCH, 2NCH₂
   5,15 ; septuplet ; 1H, OCH
   7,4 à 8,2 ; massif ; 7H, 7H aromatique
   signal très élargi centré à 7,5 ; 2COOH+DOH

### Exemple 42

Solvant :
   DMSO à 2,5 ppm
   DOH à 3,3 ppm
δ (ppm) :
   0,8 à 1 ; triplet ; 9H, 3CH₃
   1 à 1,8 ; multiplet ; 12, 6CH₂
   2 ; multiplet ; 2H, 1CH₃
   2,7 ; multiplet ; 4H, 1CH₂ phényle, 1CH₂-CH=
   3 ; multiplet ; 6H, 3NCH₂
   4,2 ; quadruplet ; 2H, 10CH₂
   7,4 ; doublet ; 2H, 2H aromatique
   7,7 ; doublet ; 3H, 3H aromatique
   7,9 ; doublet dédoublé ; 1H, 1H aromatique
   8 ; doublet ; 1H, 1H aromatique

### Exemple 45

Solvant : DMSO à 2,5 ppm
δ (ppm) :
   0,6 à 1,7 ; massif ; 27H, 5CH₃, 6CH₂
   1,9 ; multiplet ; 2H, CH₂
   2,6 à 2,8 ; massif ; 4H, 2CH₂
   2,8 à 3,5; massif ; 10H, 5NCH₂
   6,8 ; signal très élargi ; 2COOH, DOH
   7,1 à 7,8 ; massif ; 7H, ¹H aromatique

### Exemple 46

Solvant : DMSO à 2,5 ppm
δ (ppm) :
   0,8 ; triplet ; 3H, CH₃
   0,9 ; triplet ; 6H, 2CH₃
   1 à 1,8 ; massif ; 18H, 2CH₃, 6CH₂
   1,95 ; multiplet ; 2H, CH₂
   2,75 ; triplet ; 4H, 2CH₂
   3 ; multiplet ; 6H ; 3NCH₂
   5,9 ; signal très élargi ; 2COOH+DOH
   7,3 à 8,1 ; massif ; 7H, ¹H aromatique
   8,3 ; doublet ; 1H, NH

### Exemple 58

Solvant : DMSO
δ (ppm): 0,8 (triplet, 3H) ; 0,9 (triplet, 6H); 1,1 à 2,2 (massif, 14H); 2,6 à 2,9 (massif, 4H) ; 2,9 à 3,2 (massif, 6H) ; 7,2 à 8,2 (massif ; 7H)

### Exemple 81

Solvant : DMSO
δ (ppm) : 0,8 (triplet, 3H) ; 1,1 à 2,0 (massif, 6H) ; 2,6 (triplet, 2H) ; 2,9 (triplet, 6H) ; 5,2 (septuplet ; 1H) ; 7,1 à 8,1 (massif ; 12H)

### Exemple 92

Solvant : DMSO
δ (ppm) : 0,3 à 2,0 (massif, 28H) ; 2,6 à 3,4 (massif, 13H) ; 3,7 (triplet élargi, 2H) ; 4,6 (singulet, 2H) ; 7,0 à 7,9 (massif ; 7H)

### EXEMPLE 47

Selon des techniques pharmaceutiques connues, on a préparé une capsule contenant les ingrédients suivants :

| **Ingrédient** | **mg** |
|---|---|
| Composé de l'invention | 100,0 |
| Amidon | 99,5 |
| Silice colloïdale | 0,5 |

## Revendications

1. Dérivés de benzofurane ou de benzothiophène de formule générale : 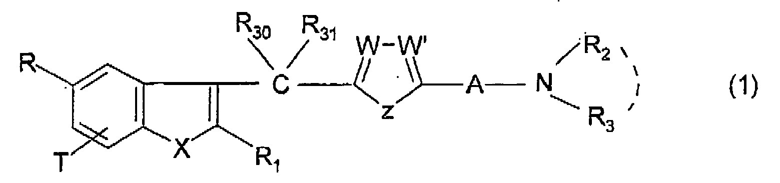 ainsi que leurs sels pharmaceutiquement acceptables, dans laquelle :
A représente un groupement alkylène, linéaire ou ramifié, en C₁-C₅, éventuellement substitué par un groupement hydroxyle, ou A représente un groupement de formule générale :
-R₁₉-O-R₂₀- (h)
dans laquelle R₁₉ et R₂₀, identiques ou différents, représentent chacun un groupement alkylène en C₁-C₄, linéaire ou ramifié,
R₃₀ et R₃₁, pris ensemble, représentent un groupement carbonyle avec le carbone sur lequel ils sont fixés, ou représentent un groupement de formule générale : 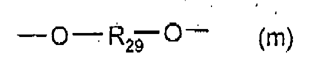 dans laquelle R₂₉ représente un groupement alkylène en C₁-C₄,
T représente l'hydrogène ou un radical alkyle en C₁-C₄,
R représente :
• le groupement cyano, formyle ou tétrazolyle,
• un groupement ester de formule générale: 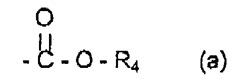 dans laquelle R₄ représente un groupement alkyle en C₁-C₅ , linéaire ou ramifié, ou cycloalkyle en C₃-C₆,
• un groupement carboxyle de formule générale : 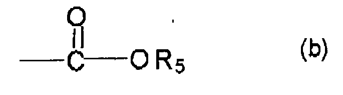 dans laquelle R₅ représente l'hydrogène ou un atome de métal alcalin,
• un groupement amide de formule générale : 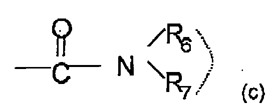 dans laquelle R₆ et R₇, identiques ou différents, représentent l'hydrogène ou un radical alkyle, linéaire ou ramifié, en C₁-C₄ ou R₆ et R₇, lorsqu'ils sont pris ensemble, représentent une chaîne alkylène en C₂-C₆,
• un groupement cétone de formule générale : 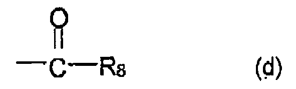 dans laquelle R₈ représente un groupement alkyle en C₁-C₄,
• un groupement oxime de formule générale :
- CH = N - OR₉ (e)
dans laquelle R₉ représente l'hydrogène ou un radical alkyle en C₁-C₄,
• un groupement carboxyle de formule générale : 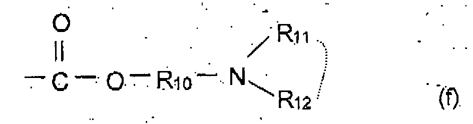 dans laquelle R₁₀ représente un groupement alkylène, linéaire ou ramifié, en C₁-C₄, R₁₁ représente l'hydrogène ou un radical alkyle en C₁-C₄, R₁₂ représente un radical alkyle en C₁-C₄ ou R₁₁ et R₁₂, lorsqu'ils sont pris ensemble, représentent une chaîne alkylène en C₂-C₆,
• un groupement de formule générale : 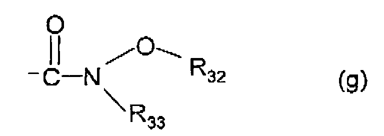 dans laquelle R₃₂ et R₃₃, identiques ou différents, représentent un groupement alkyle en C₁-C₄, linéaire ou ramifié,
R₁ représente un groupement alkyle en C₁-C₆ linéaire ou ramifié ou R₁ représente un groupement de formule générale : 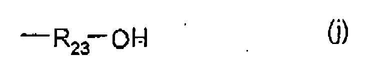 dans laquelle R₂₃ représente un groupement alkylène en C₁-C₆, linéaire ou ramifié,
R₂ et R₃, identiques ou différents, représentent l'hydrogène, un groupement alkyle linéaire ou ramifié en C₁-C₆ , éventuellement substitué par un ou plusieurs atomes d'halogène ou par un groupement pyrrolidinyle, un groupement cycloalkyle en C₃-C₆, un groupement de formule générale :
-R₂₄-O-R₂₅ (k)
dans laquelle R₂₄ représente un groupement alkylène en C₁-C₄, linéaire ou ramifié, et R₂₅ représente un groupement alkyle en C₁-C₄, linéaire ou ramifié,
ou R₂ et R₃, lorsqu'ils sont pris ensemble, représentent un groupement alkylène en C₃-C₁₀, linéaire ou ramifié, ou représentent avec l'atome d'azote sur lequel ils sont fixés un groupement de formule générale : 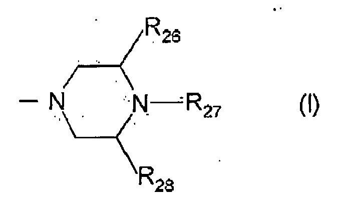 dans laquelle :
- R₂₆, R₂₇, et R₂₈, identiques ou différents, représentent l'hydrogène ou un groupement alkyle en C₁-C₄, linéaire ou ramifié, ou
- R₂₆ représente l'hydrogène ou un groupement alkyle en C₁-C₄ , linéaire ou ramifié, et R₂₇ et R₂₈ , lorsqu'ils sont pris ensemble, représentent un groupement alkylène en C₁-C₄, linéaire ou ramifié,
W, W' et Z sont tels que :
- lorsque W et W', identiques représentent CH, Z représente -O- ou -S-
- lorsque W représente CH et W' représente C-R₁₃, Z représente -CH=C(R₁₄)-, R₁₃ et R₁₄ étant identiques ou différents et représentant l'hydrogène, un atome d'halogène, un radical alkyle en C₁-C₄, ou un radical alkoxy en C₁-C₄,
X représente -O- ou -S-,
ces dérivés de benzofurane ou de benzothiophène étant sous forme d'isomères optiques individuels ou de mélanges de ceux-ci.

2. Dérivés de benzofurane ou de benzothiophène selon la revendication 1, **caractérisés en ce que** A représente un groupement alkylène, linéaire ou ramifié, en C₁-C₅ éventuellement substitué par un groupement hydroxyle.

3. Dérivés de benzofurane ou de benzothiophène selon la revendication 1 ou 2, **caractérisés en ce que** R représente le groupement isopropoxycarbonyle.

4. Dérivés de benzofurane ou de benzothiophène selon l'une quelconque des revendications précédentes, **caractérisés en ce que** R₁ et/ou R₂ et/ou R₃ représentent le groupement n-butyle.

5. Dérivés de benzofurane ou de benzothiophène selon l'une quelconque des revendications précédentes, **caractérisés en ce que** X représente - O-.

6. Dérivés de benzofurane ou de benzothiophène selon l'une quelconque des revendications précédentes, dans laquelle, lorsque R₃₀ et R₃₁ pris ensemble, représentent un groupement carbonyle avec le carbone sur lequel ils sont fixés 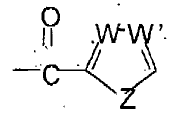 représente le groupement benzoyle.

7. Dérivés de benzofurane ou de benzothiophène selon l'une quelconque des revendications précédentes, **caractérisés en ce que** R₁ représente le groupement n-butyle, A représente le groupement propylène et R₂ et R₃ identiques représentent le groupement n-butyle.

8. 2-Butyl-3-[4-[3-(dibutylamino)propyl]benzoyl]-1-benzofurane-5-carboxylate d'isopropyle et ses sels pharmaceutiquement acceptables.

9. 2-Butyl-6-méthyl-3-[4-[3-(dlbutylamino)propyl]benzoyl]-1-benzofurane-5-carboxylate d'isopropyle et ses sels pharmaceutiquement acceptables.

10. Dérivés de benzofurane ou de benzothiophène selon l'une quelconque des revendications précédentes, dans lesquels le sel pharmaceutiquement acceptable est choisi parmi les maléate, fumarate, méthanesulfonate, benzoate, ascorbate, pamoate, succinate, hexamate, bisméthylènesalicylate, acétate, propionate, tartrate, salicylate, citrate, gluconate, lactate, malate, cinnamate, mandélate, citraconate, aspartate, palmitate, stéarate, itaconate, glycolate, p-aminobenzoate, glutamate, benzènesulfonate, p-toluènesulfonate et théophylline acétate ainsi que les sels formés à partir d'un acide aminé tel que le sel de lysine ou l'histidine.

11. Fumarate de 2-butyl-3-[4-[3-(dibutylamino)propyl]benzoyl]-1-benzofurane-5-carboxylate d'isopropyle.

12. Dérivés de benzofurane ou de benzothiophène selon l'une quelconque des revendications 1 à 9, dans lesquels le sel pharmaceutiquement acceptable est choisi parmi les chlorhydrate, bromhydrate, sulfate, sulfamate, phosphate et nitrate.

13. Sulfate de 2-butyl-3-[4-[3-(dibutylamino)propyl]benzoyl]-1-bonzofurane-5-carboxylate d'isopropyle.

14. Procédé de préparation de dérivés de benzofurane ou de benzothiophène selon la revendication 1 dans laquelle R représente le groupement cyano, le groupement formyle, un groupement (a) un groupement (d), un groupement (e) ou un groupement (f), **caractérisé en ce que** l'on fait, réagir un composé de formule générale : 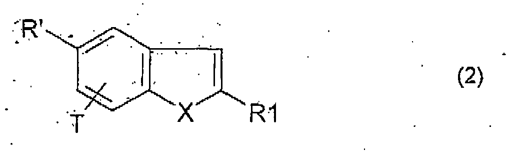 dans laquelle R' représente le groupement cyano, le groupement formyle, un groupement (a), un groupement (d), un groupement (e) ou un groupement (f), et R₁, T et X ont la même signification que dans la Revendication 1, avec un halogénure de formule générale : 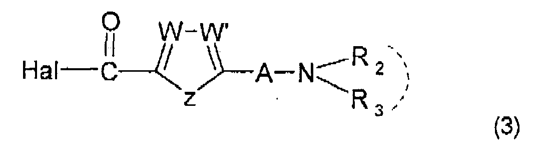 dans laquelle A, R₂, R₃, W, W' et Z ont la même signification que dans la Revendication 1 et Hal représente un atome d'halogène, la réaction ayant lieu en présence d'un acide de Lewis, ce qui fournit les composés désirés, sous forme de base libre, que l'on peut faire réagir, si nécessaire, avec un acide organique ou inorganique pour former un sel pharmaceutiquement acceptable.

15. Procédé de préparation de dérivés de benzofurane ou de benzothiophène selon la Revendication 1, dans laquelle R représente un groupement (b), **caractérisé en ce que** l'on saponifie en présence d'un hydroxyde de métal alcalin, un composé de formule générale : 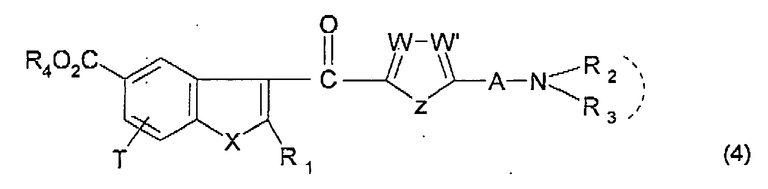 dans laquelle A, R₁, R₂, R₃, R₄, T, W, W', X et Z ont la même signification que dans la Revendication 1, ce qui fournit, sous forme de base libre, les composés désirés de formule (1) dans laquelle R₅ représente un atome de métal alcalin, composés que l'on traite, si nécessaire, avec un acide fort, ce qui fournit sous forme de base libre, les composés désirés de formule (1) dans laquelle R₅ représente l'hydrogène, la base libre ainsi formée pouvant, si nécessaire, être traitée avec un acide organique ou inorganique approprié pour obtenir un sel pharmaceutiquement acceptable.

16. Procédé de préparation de dérivés de benzofurane ou de benzothiophène selon la Revendication 1, dans laquelle R représente le groupement hydroxyméthyle, **caractérisé en ce que** l'on déprotège un cétal de formule générale : 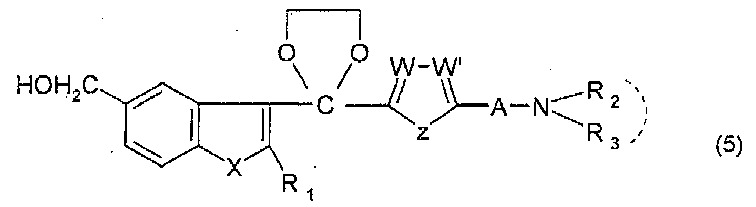 dans laquelle A, R₁, R₂, R₃, T, X, W, W' et Z ont la même signification que dans la Revendication 1 et ce, au moyen de pyridine p-toluènesulfonate, ce qui fournit les composés dérivés sous forme de base libre que l'on peut faire réagir, si nécessaire, avec un acide organique ou inorganique pour former un sel pharmaceutiquement acceptable.

17. Procédé de préparation de dérivés de benzofurane ou de benzothiophène selon la Revendication 1 dans laquelle R représente le groupement cyano, le groupement formyle, un groupement (a), un groupement (d), un groupement (e) ou un groupement (f), et R₂ et R₃, qui sont identiques, représentent chacun l'hydrogène, **caractérisé en ce que** l'on traite, avec la triphénylphosphine, un azide de formule générale : 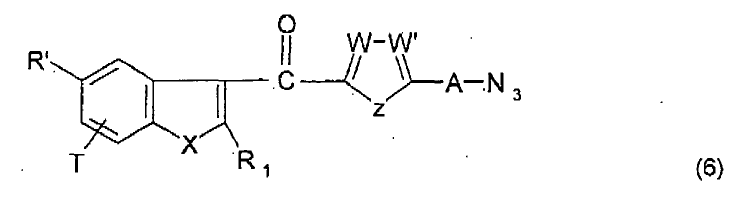 dans laquelle R' représente le groupement cyano, le groupement formyle, un groupement (a), un groupement (d), un groupement (e) ou un groupement (f), et A, R₁, T, X, W, W' et Z ont la même signification que dans la Revendication 1, ce qui fournit les composés désirés sous forme de base libre que l'on peut faire réagir, si nécessaire, avec un acide organique ou inorganique pour former un sel pharmaceutiquement acceptable.

18. Procédé de préparation de dérivés de benzofurane ou de benzothiophène selon la Revendication 1 dans laquelle R représente le groupement cyano, le groupement formyle, un groupement (a), un groupement (d), un groupement (e) ou un groupement (f), et A représente un groupement alkylène, linéaire ou ramifié, en C₃-C₅ ou un groupement (h), **caractérisé en ce que** l'on fait réagir, un composé cétonique de formule générale : 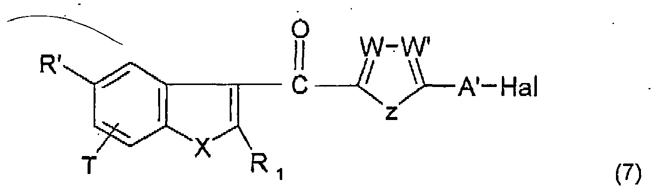 dans laquelle R' représente le groupement cyano, le groupement formyle, un groupement (a), un groupement (d), un groupement (e) ou un groupement (f), A' représente un groupement alkylène, linéaire ou ramifié, en C₃-C₅, Hal représente un atome d'halogène et R₁, T, W, W', X et Z ont la même signification que dans la Revendication 1, avec un composé de formule générale : 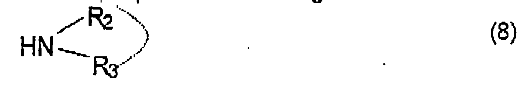 dans laquelle R₂ et R₃ ont la même signification que dans la Revendication 1, la réaction ayant lieu en présence d'un agent basique ou un excès de composé de formule (8), ce qui fournit les composés désirés sous forme de base libre que l'on peut faire réagir, si nécessaire, avec un acide organique ou inorganique pour former un sel pharmaceutiquement acceptable.

19. Procédé de préparation de dérivés de benzofurane ou de benzothiophène selon la Revendication 1 dans laquelle R représente le groupement cyano, le groupement formyle, un groupement (a), un groupement (d), un groupement (e) ou un groupement (f), R₂ représente l'hydrogène ou un groupement alkyle, linéaire ou ramifié, en C₁-C₆ et R₃ représente un groupement alkyle, linéaire ou ramifié, en C₁-C₆, **caractérisé en ce que** l'on fait réagir une amine de formule générale : 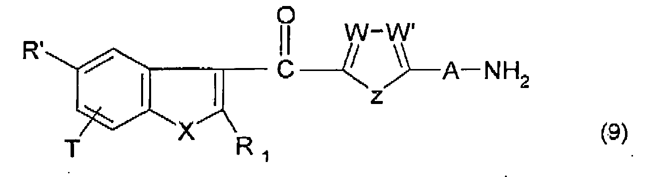 dans laquelle R' représente le groupement cyan, le groupement formyle, un groupement (a), un groupement (d), un groupement (e) ou un groupement (f), et A, R₁, T, X, W, W' et Z ont la même signification que dans ta Revendication 1, avec une aldéhyde dé formule générale 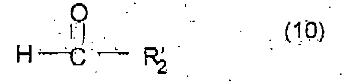 dans laquelle R'₂ représente l'hydrogène ou un radical alkyle en C₁-C₅ et ce, en présence de triacétoxyborohydrure de sodium, pour fournir les composés désirés, sous forme de base libre, que l'on peut faire réagir, si nécessaire, avec un acide organique ou inorganique pour former un sel pharmaceutiquement acceptable.

20. Procédé de préparation de dérivés de benzofurane ou de benzothiophène selon la Revendication 1 dans laquelle R représente un groupement (c), **caractérisé en ce que** l'on fait réagir un composé de formule générale : 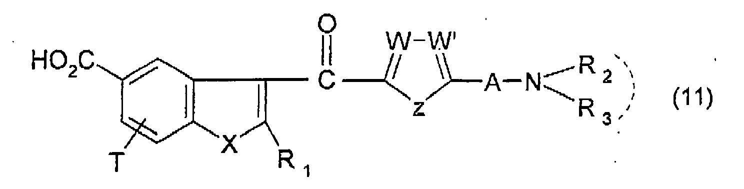 dans laquelle A, R₁, R₂, R₃, T, W, W', X et Z ont la même signification que dans la Revendication 1, et après protection de la fonction amine lorsque R₂ et/ou R₃ représentent l'hydrogène, la réaction ayant lieu avec un agent halogénant pour obtenir un halogénure d'acyle que l'on traite ensuite avec un composé de formule générale : 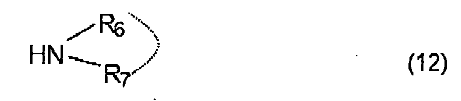 dans laquelle R₆ et R₇ ont la même signification que dans la Revendication 1, puis on déprotège, si nécessaire, le composé formé, ce qui fournit les composés désirés sous forme de base libre, la base libre ainsi formée pouvant, si nécessaire, être traitée avec un acide organique ou inorganique approprié pour obtenir un sel pharmaceutiquement acceptable.

21. Procédé de préparation de dérivés de benzofurane ou de benzothiophène selon la Revendication 1 dans laquelle R représente un groupement (a), **caractérisé en ce que** l'on fait réagir un composé de formule générale : 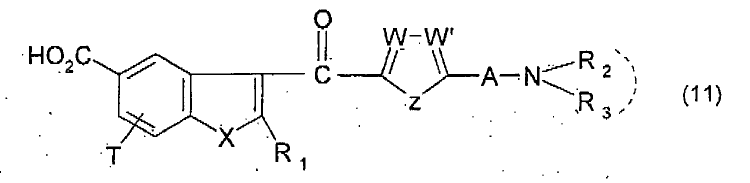 dans laquelle A, R₁, R₂, R₃, T, W, W', X et Z ont la même signification que dans la Revendication 1, et après protection de la fonction amine lorsque R₂ et/ou R₃ représentent l'hydrogène, la réaction ayant lieu avec une agent halogénant pour obtenir un halogénure d'acyle que l'on traite ensuite avec un alcool de formule générale:
R₄ - OH (13)
dans laquelle R₄ a la même signification que dans la Revendication 1, puis on déprotège, si nécessaire, le composé formé, ce qui fournit les composés désirés sous forme de base libre, la base libre ainsi formée pouvant, si nécessaire, être traitée avec un acide organique ou inorganique approprié pour obtenir un sel pharmaceutiquement acceptable.

22. Procédé de préparation de dérivés de benzofurane ou de benzothiophène selon la Revendication 1 dans laquelle R représente un groupement (c) dans lequel R₆ et R₇ représentent chacun l'hydrogène, **caractérisé en ce que** l'on hydrolyse en présence d'un acide fort un composé de formule générale : 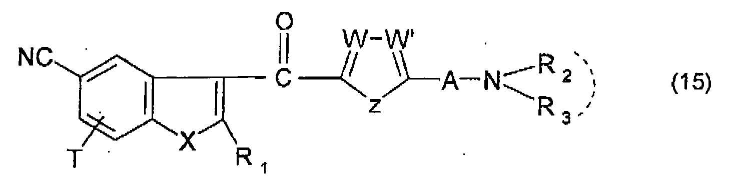 dans laquelle A, R₁, R₂, R₃, T, W, W', X et Z ont la même signification que dans la Revendication 1, ce qui fournit les composés désirés sous forme de base libre, la base libre ainsi formée pouvant, si nécessaire, être traitée avec un acide organique ou inorganique approprié pour obtenir un sel pharmaceutiquement acceptable.

23. Procédé de préparation de dérivés de benzofurane ou de benzothiophène selon la Revendication 1 dans laquelle R représente un groupement (f) du type dialkylaminoalkyle primaire, **caractérisé en ce que** l'on fait réagir un composé de formule générale : 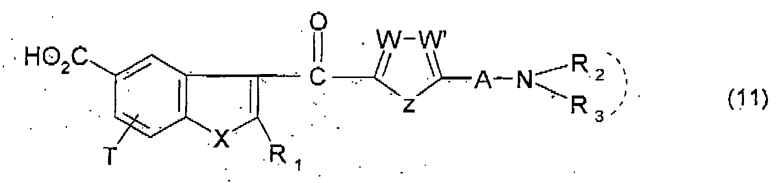 dans laquelle A, R₁, R₂, R₃, T, W, W', X et Z ont la même signification que dans la Revendication 1 et après protection de la fonction amine lorsque R₂ et/ou R₃ représentent l'hydrogène et ce, avec un alcool de formule générale : 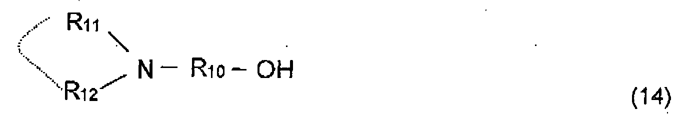 dans laquelle R₁₁ et R₁₂ ont la même signification que dans la Revendication 1, et R₁₀ représente un groupement alkylène linéaire en C₁-C₄, la réaction ayant lieu en présence de carbonyl diimidazole et de 1,8-diazabicyclo [5.4.0]undec-7ène, puis on déprotège, si nécessaire, le composé formé, ce qui fournit les composés désirés sous forme de base libre que l'on peut faire réagir, si nécessaire, avec un acide organique ou inorganique pour former un sel pharmaceutiquement acceptable.

24. Procédé de préparation de dérivés de benzofurane ou de benzothiophène selon la Revendication 1 dans laquelle R représente un groupement (f) du type dialkylaminoalkyle secondaire ou tertiaire, **caractérisé en ce que** l'on fait réagir un composé de formule générale : 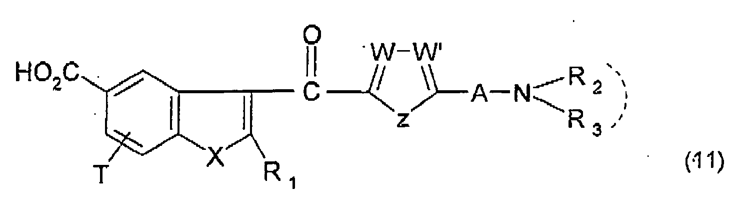 dans laquelle A, R₁. R₂, R₃, T, W, W', X et Z ont la même signification que dans la Revendication 1 et après protection de la fonction amine lorsque R₂ et/ou R₃ représentent l'hydrogène et ce, avec un agent halogénant, pour obtenir un halogénure d'acyle que l'on traite ensuite avec un alcool de formule générale : 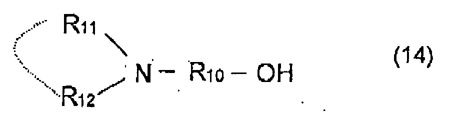 dans laquelle R₁₁ et R₁₂ ont la même signification que dans la Revendication 1, et R₁₀ représente un groupement alkylène secondaire ou tertiaire en C₂-C₄, puis on déprotège, si nécessaire, le composé formé, ce qui fournit les composés de formule (1) sous forme d'halohydrate ou sous forme de base libre, halohydrate que l'on peut traiter, si nécessaire, avec un agent basique, pour obtenir les composés désirés sous forme de base libre que l'on peut faire réagir, si nécessaire, avec un acide organique ou inorganique pour former un sel pharmaceutiquement acceptable.

25. Procédé de préparation de dérivés de benzofurane ou de benzothiophène selon la Revendication 1 dans laquelle R représente le groupement tétrazolyle, **caractérisé en ce que** l'on fait réagir avec un (trialkyl en C₁-C₄) azido étain un composé de formule générale : 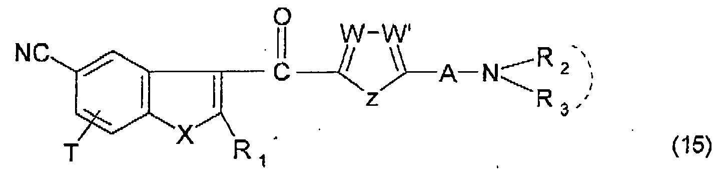 dans laquelle A, R₁, R₂, R₃, T, W, W', X et Z ont la même signification que dans la Revendication 1, ce qui fournit les composés désirés sous forme de base libre, la base libre ainsi formée pouvant, si nécessaire, être traitée avec un acide organique ou inorganique approprié pour obtenir un sel pharmaceutiquement acceptable.

26. Procédé de préparation de dérivés de benzofurane ou de benzothiophène selon la Revendication 1 dans laquelle R représente le groupement cyano, le groupement formyle, un groupement (a), un groupement (d), un groupement (e) ou un groupement (f), et dans laquelle R₂ et R₃, qui sont différents, représentent chacun un groupement alkyle, linéaire ou ramifié, en C₁-C₆ ou un groupement cycloalkyle en C₃-C₆, en faisant réagir un composé de formule générale : 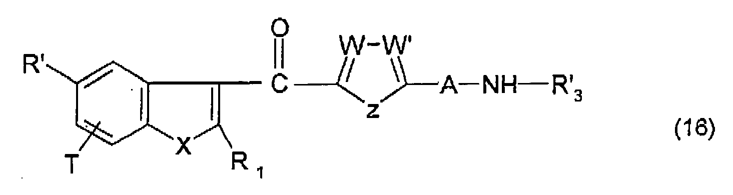 dans laquelle A, R₁, R₂, T, W, W', X et Z ont la même signification que dans Revendication 1 et R' représente le groupement cyano, le groupement formyle, un groupement (a), un groupement (d), un groupement (e) ou un groupement (f), et R'₃ représente un groupement alkyle, linéaire ou ramifié, en C₁-C₆ ou cycloalkyle en C₃-C₆, avec un halogénure de formule générale :
Hal - R"₂ (17)
dans laquelle Hal représente un atome d'halogène et R"₂ représente un groupement alkyle, linéaire ou ramifié, en C₁-C₆ ou un groupement cycloalkyle en C₃-C₆, la réaction ayant lieu en présence d'un agent basique, ce qui fournit les composés dérivés sous forme de base libre, la base libre ainsi formée pouvant, si nécessaire, être traitée avec un acide organique ou inorganique approprié pour obtenir un sel pharmaceutiquement acceptable.

27. Procédé de préparation de dérivés de benzofurane ou de benzothiophène selon la Revendication 14, 15, 17 ou 18, **caractérisé en ce que** la protection de la fonction amine s'effectue au moyen de 9-fluorénylméthylchloroformiate et la déprotection par traitement au moyen d'une amine secondaire.

28. Médicament, **caractérisé en ce qu'**il comprend un dérivé de benzofurane ou de benzothiophène, ou un sel pharmaceutiquement acceptable de ce dernier, selon l'une quelconque des revendications 1 à 13.

29. Composition pharmaceutique ou vétérinaire, **caractérisée en ce qu'**elle contient comme principe actif au moins un dérivé de benzofurane ou de benzothiophène, ou un sel pharmaceutiquement acceptable de ce dernier, selon l'une quelconque des revendications 1 à 13, en association avec un véhicule pharmaceutique ou excipient approprié.

30. Composition pharmaceutique ou vétérinaire selon la Revendication 29, pour le traitement de syndromes pathologiques du système cardio-vasculaire.

31. Composition pharmaceutique ou vétérinaire selon la Revendication 29 ou 30, pour le traitement de l'angine de poitrine, de l'hypertension, de l'arythmie atriale, ventriculaire ou supraventriculaire, de l'insuffisance circulatoire cérébrale, de l'insuffisance cardiaque, de l'infarctus du myocarde compliqué ou non d'insuffisance cardiaque ou pour la prévention de la mortalité post-infarctus.

32. Composition pharmaceutique ou vétérinaire selon l'une quelconque des Revendications 29 à 31, **caractérisée en ce qu'**elle contient de 50 à 500 mg de principe actif.

33. Utilisation d'au moins un dérivé de benzofurane ou de benzothiophène ou d'un sel pharmaceutiquement acceptable de ce dernier, selon l'une quelconque des Revendications 1 à 13, pour la fabrication d'un médicament destiné au traitement de syndromes pathologiques du système cardio-vasculaire.

## Claims

1. Benzofuran or benzothiophene derivatives of general formula: 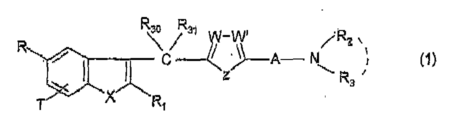 and their pharmaceutically acceptable salts, in which formula:
A represents a linear or branched C₁-C₅ alkylene group optionally substituted by a hydroxyl group or A represents a group of general formula:
-R₁₉-Q-R₂₀- (h)
in which R₁₉ and R₂₀, which are identical or different, each represent a linear or branched C₁-C₄ alkylene group,
R₃₀ and R₃₁, taken together, represent a carbonyl group with the carbon to which they are attached or represent a group of general formula: 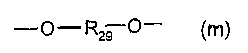 in which R₂₉ represents a C₁-C₄ alkylene group,
T represents hydrogen or a C₁-C₄ alkyl radical,
R represents:
• the cyano, formyl or tetrazolyl group,
• an ester group of general formula: 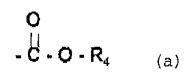 in which R₄ represents a linear or branched C₁-C₆ alkyl group or a C₃-C₆ cycloalkyl group,
• a carboxyl group of general formula: 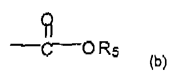 in which R₅ represents hydrogen or an alkali metal atom,
• an amide group of general formula: 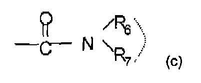 in which R₆ and R₇, which are identical or different, represent hydrogen or a linear or branched C₁-C₄ alkyl radical or R₆ and R₇, when they are taken together, represent a C₂-C₆ alkylene chain,
• a ketone group of general formula: 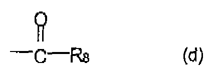 in which R₆ represents a C₁-C₄ alkyl group,
• an oxime group of general formula:
-CH=N-OR₉ (e)
in which R₉ represents hydrogen or a C₁-C₄ alkyl radical,
• a carboxyl group of general formula: 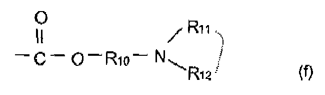 in which R₁₀ represents a linear or branched C₁-C₄ alkylene group, R₁₁ represents hydrogen or a C₁-C₄ alkyl radical, R₁₂ represents a C₁-C₄ alkyl radical or R₁₁ and R₁₂, when they are taken together, represent a C₂-C₆ alkylene chain,
• a group of general formula: 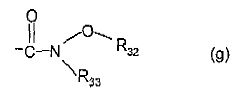 in which R₃₂ and R₃₂, which are identical or different, represent a linear or branched C₁-C₄ alkyl group,
R₁ represents a linear or branched C₁-C₆ alkyl group, or R₁ represents a group of general formula:
-R₂₃-OH (j)
in which R₂₃ represents a linear or branched C₁-C₆ alkylene group,
R₂ and R₃, which are identical or different, represent hydrogen, a linear or branched C₁-C₆ alkyl group optionally substituted by one or more halogen atoms or by a pyrrolidinyl group, a C₃-C₆ cycloalkyl group or a group of general formula:
-R₂₄-O-R₂₅ (k)
in which R₂₄ represents a linear or branched C₁-C₄ alkylene group and R₂₅ represents a linear or branched C₁-C₄ alkyl group,
or R₂ and R₃, when they are taken together, represent a linear or branched C₃-C₁₀ alkylene group or represent, with the nitrogen atom to which they are attached, a group of general formula: 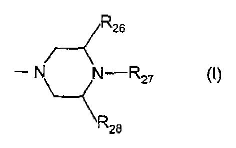 in which:
- R₂₆, R₂₇ and R₂₈, which are identical or different, represent hydrogen, or a linear or branched C₁-C₄ alkyl group, or
- R₂₆ represents hydrogen or a linear or branched C₁-C₄ alkyl group and R₂₇ and R₂₈, when they are taken together, represent a linear or branched C₁-C₄ alkylene group,
W, W' and Z are such that:
- when W and W', which are identical, represent CH, Z represents -O- or -S-,
- when W represents CH and W' represents C-R₁₃, Z represents 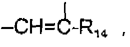 R₁₃ and R₁₄ being identical or different and representing hydrogen, a halogen atom, a C₁-C₄ alkyl radical or a C₁-C₄ alkoxy radical,
X represents -0- or -S-,
these benzofuran or benzothiophene derivatives being in the form of individual optical isomers or of mixtures of the latter.

2. Benzofuran or benzothiophene derivatives according to Claim 1, **characterized in that** A represents a linear or branched C₁-C₅ alkylene group optionally substituted by a hydroxyl group.

3. Benzofuran or benzothiophene derivatives according to Claim 1 or 2, **characterized in that** R represents the isopropoxycarbonyl group.

4. Benzofuran or benzothiophene derivatives according to any one of the preceding claims, **characterized in that** R₁ and/or R₂ and/or R₃ represent the n-butyl group.

5. Benzofuran or benzothiophene derivatives according to any one of the preceding claims, **characterized in that** X represents -O-.

6. Benzofuran or benzothiophene derivatives according to any one of the preceding claims, in which, when R₃₀ and R₃₁, taken together, represent a carbonyl group with the carbon to which they are attached, 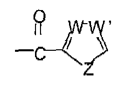 represents the benzoyl group.

7. Benzofuran or benzothiophene derivatives according to any one of the preceding claims, **characterized in that** R₁ represents the n-butyl group, A represents the propylene group and R₂ and R₃, which are identical, represent the n-butyl group.

8. Isopropyl 2-butyl-3-[4-[3-(dibutylamino)propyl]-benzoyl]-1-benzofuran-5-carboxylate and its pharmaceutically acceptable salts.

9. Isopropyl 2-butyl-6-methyl-3-[4-[3-(dibutylamino)propyl]benzoyl]-1-benzofuran-5-carboxylate and its pharmaceutically acceptable salts.

10. Benzofuran or benzothiophene derivatives according to any one of the preceding claims, in which the pharmaceutically acceptable salt is chosen from maleate, fumarate, methanesulphonate, benzoate, ascorbate, pamoate, succinate, hexamate, bismethylenesalicylate, acetate, propionate, tartrate, salicylate, citrate, gluconate, lactate, malate, cinnamate, mandelate, citraconate, aspartate, palmitate, stearate, itaconate, glycolate, p-aminobenzoate, glutamate, benzenesulphonate, p-toluenesulphonate and theophyllineacetate salts and the salts formed from an amino acid, such as the lysine or histidine salt.

11. Isopropyl 2-butyl-3-[4-[3-(dibutylamino)-propyl]benzoyl]-1-benzofuran-5-carboxylate fumarate.

12. Benzofuran or benzothiophene derivatives according to any one of Claims 1 to 9, in which the pharmaceutically acceptable salt is chosen from the hydrochloride, hydrobromide, sulphate, sulphamate, phosphate and nitrate salts.

13. Isopropyl 2-butyl-3-[4-[3-(dibutylamino)-propyl]benzoyl]-1-benzofuran-5-carboxylate sulphate.

14. Process for the preparation of benzofuran or benzothiophene derivatives according to Claim 1 in which R represents the cyano group, the formyl group, a group (a), a group (d), a group (e) or a group (f), **characterized in that** a compound of general formula: 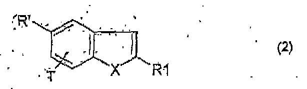 in which R' represents the cyano group, the formyl group, a group (a), a group (d), a group (e) or a group (f) and R₁, T and X have the same meaning as in Claim 1, is reacted with a halide of general formula: 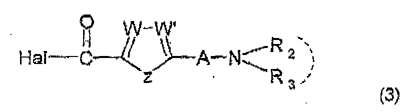 in which A, R₂, R₃, W, W' and Z have the same meaning as in Claim 1 and Hal represents a halogen atom, the reaction taking place in the presence of a Lewis acid, which gives the desired compounds in the free base form, which base can be reacted, if necessary, with an organic or inorganic acid to form a pharmaceutically acceptable salt.

15. Process for the preparation of benzofuran or benzothiophene derivatives according to Claim 1 in which R represents a group (b), **characterized in that** a compound of general formula: 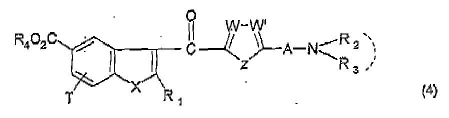 in which A, R₁, R₂, R₃, R₄, T, W, W', X and Z have the same meaning as in Claim 1, is saponified in the presence of an alkali metal hydroxide, which gives, in the free base form, the desired compounds of formula (1) in which R₅ represents an alkali metal atom, which compounds are treated, if necessary, with a strong acid, which gives, in the free base form, the desired compounds of formula (1) in which R₅ represents hydrogen, it being possible for the free base thus formed, if necessary, to be treated with an appropriate organic or inorganic acid to produce a pharmaceutically acceptable salt.

16. Process for the preparation of benzofuran or benzothiophene derivatives according to Claim 1 in which R represents the hydroxymethyl group, **characterized in that** a ketal of general formula: 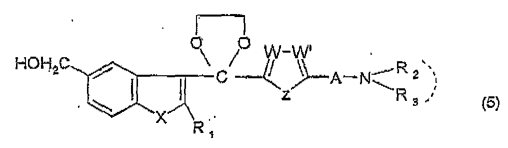 in which A, R₁, R₂, R₃, T, X, W, W' and Z have the same meaning as in Claim 1, is deprotected by means of pyridine p-toluenesulphonate, which gives the desired compounds in the free base form, which base can be reacted, if necessary, with an organic or inorganic acid to form a pharmaceutically acceptable salt.

17. Process for the preparation of benzofuran or benzothiophene derivatives according to Claim 1 in which R represents the cyano group, the formyl group, a group (a), a group (d), a group (e) or a group (f) and R₂ and R₃, which are identical, each represent hydrogen, **characterized in that** an azide of general formula: 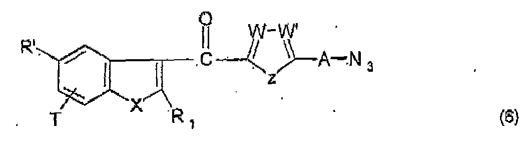 in which R' represents the cyano group, the formyl group, a group (a), a group (d), a group (e) or a group (f) and A, R₁, T, X, W, W' and Z have the same meaning as in Claim 1, is treated with triphenylphosphine, which gives the desired compounds in the free base form, which base can be reacted, if necessary, with an organic or inorganic acid to form a pharmaceutically acceptable salt.

18. Process for the preparation of benzofuran or benzothiophene derivatives according to Claim 1 in which R represents the cyano group, the formyl group, a group (a), a group (d), a group (e) or a group (f) and A represents a linear or branched C₃-C₅ alkylene group or a group (h), **characterized in that** a ketone compound of general formula: 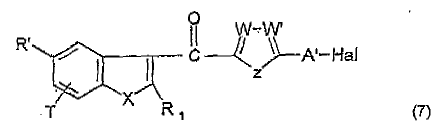 in which R' represents the cyano group, the formyl group, a group (a), a group (d), a group (e) or a group (f), A' represents a linear or branched C₃-C₅ alkylene group, Hal represents a halogen atom and R₁, T, W, W', X and Z have the same meaning as in Claim 1, is reacted with a compound of general formula: 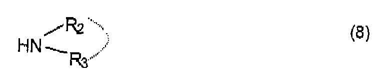 in which R₂ and R₃ have the same meaning as in Claim 1, the reaction taking place in the presence of a basic agent or an excess of compound of formula (8), which gives the desired compounds in the free base form, which base can be reacted, if necessary, with an organic or inorganic acid to form a pharmaceutically acceptable salt.

19. Process for the preparation of benzofuran or benzothiophene derivatives according to Claim 1 in which R represents the cyano group, the formyl group, a group (a), a group (d), a group (e) or a group (f), R₂ represents hydrogen or a linear or branched C₁-C₆ alkyl group and R₃ represents a linear or branched C₁-C₆ alkyl group, **characterized in that** an amine of general formula: 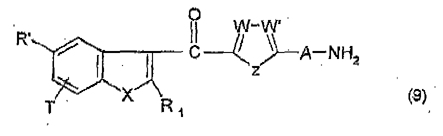 in which R' represents the cyano group, the formyl group, a group (a), a group (d), a group (e) or a group (f) and A, R₁, T, X, W, W' and Z have the same meaning as in Claim 1, is reacted with an aldehyde of general formula: 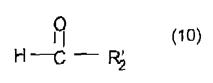 in which R'₂ represents hydrogen or a C₁-C₅ alkyl radical, in the presence of sodium triacetoxyborohydride, to give the desired compounds in the free base form, which base can be reacted, if necessary, with an organic or inorganic acid to form a pharmaceutically acceptable salt.

20. Process for the preparation of benzofuran or benzothiophene derivatives according to Claim 1 in which R represents a group (c), **characterized in that** a compound of general formula: 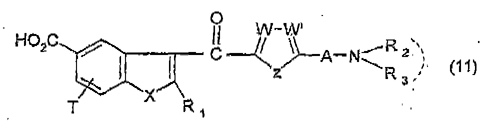 in which A, R₁, R₂, R₃, T, W, W', X and Z have the same meaning as in Claim 1, is reacted, after protection of the amine functional group when R₂ and/or R₂ represent hydrogen, the reaction taking place with a halogenating agent to produce an acyl halide, which halide is subsequently treated with a compound of general formula: 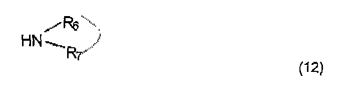 in which R₆ and R₇ have the same meaning as in Claim 1, and then, if necessary, the compound formed is deprotected, which gives the desired compounds in the free base form, it being possible for the free base thus formed, if necessary, to be treated with an appropriate organic or inorganic acid to produce a pharmaceutically acceptable salt.

21. Process for the preparation of benzofuran or benzothiophene derivatives according to Claim 1 in which R represents a group (a), **characterized in that** a compound of general formula: 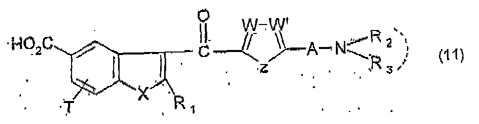 in which A, R₁, R₂, R₂, T, W, W', X and Z have the same meaning as in Claim 1, is reacted, after protection of the amine functional group when R₂ and/or R₃ represent hydrogen, the reaction taking place with a halogenating agent to produce an acyl halide, which halide is subsequently treated with an alcohol of general formula:
R₄-OH (13)
in which R₄ has the same meaning as in Claim 1, and then, if necessary, the compound formed is deprotected, which gives the desired compounds in the free base form, it being possible for the free base thus formed, if necessary, to be treated with an appropriate organic or inorganic acid to produce a pharmaceutically acceptable salt,

22. Process for the preparation of benzofuran or benzothiophene derivatives according to Claim 1 in which R represents a group (c) in which R₆ and R₇ each represent hydrogen, **characterized in that** a compound of general formula: 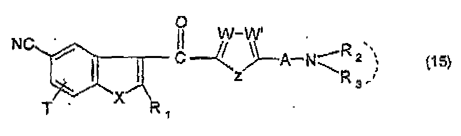 in which A, R₁, R₂, R₃, T, W, W', X and Z have the same meaning as in Claim 1, is hydrolysed in the presence of a strong acid, which gives the desired compounds in the free base form, it being possible for the free base thus formed, if necessary, to be treated with an appropriate organic or inorganic acid to produce a pharmaceutically acceptable salt.

23. Process for the preparation of benzofuran or benzothiophene derivatives according to Claim 1 in which R represents a group (f) of the primary dialkylaminoalkyl type, **characterized in that** a compound of general formula: 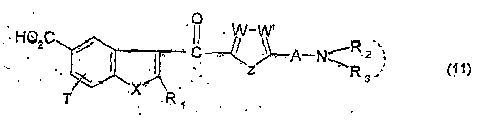 in which A, R₁, R₂, R₃, T, W, W', X and Z have the same meaning as in Claim 1, is reacted, after protection of the amine functional group when R₂ and/or R₃ represent hydrogen, with an alcohol of general formula: 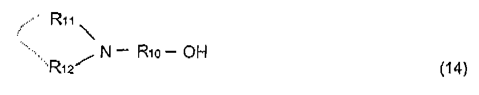 in which R₁₁ and R₁₂ have the same meaning as in Claim 1 and R₁₀ represents a linear C₁-C₄ alkylene group, the reaction taking place in the presence of carbonyldiimidazole and of 1,8-diazabicyclo[5.4.0]undec-7-ene, and then, if necessary, the compound formed is deprotected, which gives the desired compounds in the free base form, which base can be reacted, if necessary, with an organic or inorganic acid to form a pharmaceutically acceptable salt.

24. Process for the preparation of benzofuran or benzothiophene derivatives according to Claim 1 in which R represents a group (f) of the secondary or tertiary dialkylaminoalkyl type, **characterized in that** a compound of general formula: 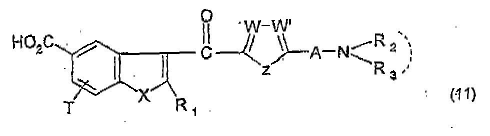 in which A, R₁, R₂, R₃, T, W, W', X and Z have the same meaning as in Claim 1, is reacted, after protection of the amine functional group when R₂ and/or R₃ represent hydrogen, with a halogenating agent to produce an acyl halide, which halide is subsequently treated with an alcohol of general formula: 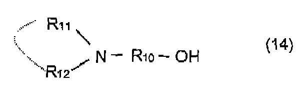 in which R₁₁ and R₁₂ have the same meaning as in Claim 1 and R₁₀ represents a secondary or tertiary C₂-C₄ alkylene group, and then, if necessary, the compound formed is deprotected, which gives the compounds of formula (1) in the hydrohalide form or in the free base form, which hydrohalide can be treated, if necessary, with a basic agent to produce the desired compounds in the free base form, which base can be reacted, if necessary, with an organic or inorganic acid to form a pharmaceutically acceptable salt.

25. Process for the preparation of benzofuran or benzothiophene derivatives according to Claim 1 in which R represents the tetrazolyl group, **characterized in that** a compound of general formula: 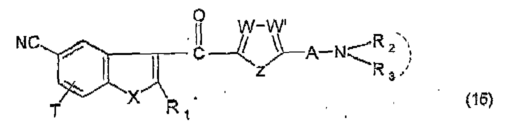 in which A, R₁, R₂, R₃, T, W, W', X and Z have the same meaning as in Claim 1, is reacted with a tri(C₁-C₄ alkyl)azidotin, which gives the desired compounds in the free base form, it being possible for the free base thus formed, if necessary, to be treated with an appropriate organic or inorganic acid to produce a pharmaceutically acceptable salt.

26. Process for the preparation of benzofuran or benzothiophene derivatives according to Claim 1 in which R represents the cyano group, the formyl group, a group (a), a group (d), a group (e) or a group (f) and
in which R₂ and R₃, which are different, each represent a linear or branched C₁-C₆ alkyl group or a C₃-C₆ cycloalkyl group, by reacting a compound of general formula; 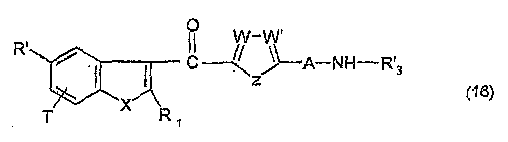 in which A, R₁, R₂, T, W, W', X and Z have the same meaning as in Claim 1 and R' represents the cyano group, the formyl group, a group (a), a group (d), a group (e) or a group (f) and R'₃ represents a linear or branched C₁-C₆ alkyl group or a C₂-C₆ cycloalkyl group, with a halide of general formula:
Hal-R"₂ (17)
in which Hal represents a halogen atom and R"₂ represents a linear or branched C₁-C₆ alkyl group or a C₃-C₆ cycloalkyl group, the reaction taking place in the presence of a basic agent, which gives the desired compounds in the free base form, it being possible for the free base thus formed, if necessary, to be treated with an appropriate organic or inorganic acid to produce a pharmaceutically acceptable salt.

27. Process for the preparation of benzofuran or benzothiophene derivatives according to Claim 14, 15, 17 or 18, **characterized in that** the protection of the amine functional group is carried out by means of 9-fluorenylmethyl chloroformate and the deprotection is carried out by treatment by means of a secondary amine.

28. Medicament, **characterized in that** it comprises a benzofuran or benzothiophene derivative or a pharmaceutically acceptable salt of the latter according to any one of Claims 1 to 13.

29. Pharmaceutical or veterinary composition, **characterized in that** it comprises, as active principle, at least one benzofuran or benzothiophene derivative or a pharmaceutically acceptable salt of the latter according to any one of Claims 1 to 13, in combination with an appropriate excipient or pharmaceutical vehicle.

30. Pharmaceutical or veterinary composition according to Claim 29 for the treatment of pathological syndromes of the cardiovascular system.

31. Pharmaceutical or veterinary composition according to Claim 29 or 30 for the treatment of angina pectoris, hypertension, atrial, ventricular or supraventricular arrhythmia, cerebral circulatory insufficiency, cardiac insufficiency or myocardial infarction, complicated or not complicated by cardiac insufficiency, or for the prevention of post-infarction mortality.

32. Pharmaceutical or veterinary composition according to any one of Claims 29 to 31, **characterized in that** it comprises from 50 to 500 mg of active principle.

33. Use of at least one benzofuran or benzothiophene derivative or of a pharmaceutically acceptable salt of the latter according to any one of Claims 1 to 13 for the manufacture of a medicament for use in the treatment of pathological syndromes of the cardiovascular system.

## Patentansprüche

1. Benzofuran- oder Benzothiophenderivate der allgemeinen Formel: 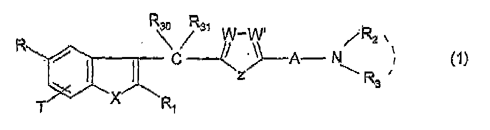 sowie pharmazeutisch unbedenkliche Salze davon, wobei:
A für eine lineare oder verzweigte C₁-C₅-Alkylengruppe, die gegebenenfalls durch eine Hydroxylgruppe substituiert ist, oder für eine Gruppe der allgemeinen Formel:
-R₁₉-O-R₂₀- (h)
worin R₁₉ und R₂₀ gleich oder verschieden sind und jeweils für eine lineare oder verzweigte C₁-C₄-Alkylengruppe stehen, steht,
R₃₀ und R₃₁ zusammengenommen mit dem Kohlenstoff, an den sie gebunden sind, für eine Carbonylgruppe stehen oder für eine Gruppe der allgemeinen Formel:
-O-R₂₉-O- (m)
worin R₂₉ für eine C₁-C₄-Alkylengruppe steht, stehen,
T für Wasserstoff oder einen C₁-C₄-Alkylrest steht, R für:
• eine Cyano-, Formyl- oder Tetrazolylgruppe,
• eine Estergruppe der allgemeinen Formel: 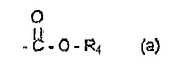 worin R₄ für eine lineare oder verzweigte C₁-C₆-Alkylgruppe oder eine C₃-C₆-Cycloalkylgruppe steht,
• eine Carboxylgruppe der allgemeinen Formel: 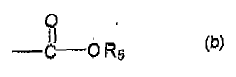 worin R₅ für Wasserstoff oder ein Alkalimetallatom steht,
• eine Amidgruppe der allgemeinen Formel: 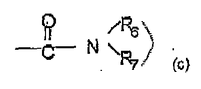 worin R₆ und R₇ gleich oder verschieden sind und für Wasserstoff oder einen linearen oder verzweigten C₁-C₄-Alkylrest stehen oder R₆ und R₇ zusammengenommen für eine C₂-C₆-Alkylenkette stehen,
• eine Ketongruppe der allgemeinen Formel: 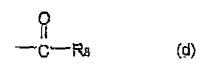 worin R₆ für eine C₁-C₄-Alkylgruppe steht,
• eine Oximgruppe der allgemeinen Formel:
-CH=N-OR₉ (e)
worin R₉ für Wasserstoff oder einen C₁-C₄-Alkylrest steht,
• eine Carboxylgruppe der allgemeinen Formel: 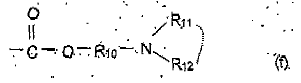 worin R₁₀ für eine lineare oder verzweigte C₁-C₄-Alkylengruppe steht, R₁₁ für Wasserstoff oder einen C₁-C₄-Alkylrast steht, R₁₂ für einen C₂-C₄-Alkylrest steht oder R₁₁ und R₁₂ zusammengenommen für eine C₂-C₆-Alkylenkette stehen,
• eine Gruppe der allgemeinen Formel: 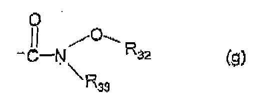 worin R₃₂ und R₃₃ gleich oder verschieden sind und für eine lineare oder verzweigte C₁-C₄-Alkylgruppe stehen,
steht,
R₁ für eine lineare oder verzweigte C₁-C₆-Alkylgruppe oder eine Gruppe der allgemeinen Formel:
-R₂₃-OH (j)
worin R₂₂ für eine lineare oder verzweigte C₁-C₆-Alkylengruppe steht, steht,
R₂ und R₃ gleich oder verschieden sind und für Wasserstoff, eine lineare oder verzweigte C₁-C₆-Alkylgruppe, die gegebenenfalls durch ein oder mehrere Halogenatome oder eine Pyrrolidingruppe substituiert ist, eine C₃-C₆-Cycloalkylgruppe, eine Gruppe der allgemeinen Formel:
-R₂₄-O-R₂₅ (k)
worin R₃₄ für eine lineare oder verzweigte C₁-C₄-Alkylengruppe steht und R₂₅ für eine lineare oder verzweigte C₁-C₄-Alkylgruppe steht, stehen oder R₂ und R₃ zusammengenommen für eine lineare oder verzweigte C₃-C₁₀-Alkylengruppe stehen oder mit dem Stickstoffatom, an das sie gebunden sind, für eine Gruppe der allgemeinen Formel: 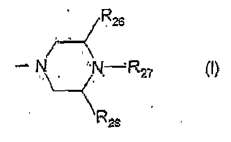 worin:
- R₂₆, R₂₇ und P₂₈ gleich oder verschieden sind und für Wasserstoff oder eine lineare oder verzweigte C₁-C₄-Alkylgruppe stehen oder
- R₂₆ für Wasserstoff oder eine lineare oder verzweigte C₁-C₄-Alkylgruppe steht und R₂₇ und R₂₈ zusammengenommen für eine lineare oder verzweigte C₁-C₄-Alkylengruppe stehen,
W, W' und z so beschaffen sind, daß:
- dann, wenn W und W' gleich sind und für CH stehen, Z für -O- oder -S- steht,
- dann, wenn W für CH und W' für C-R₁₃ steht, Z für
- CH=C(R₁₄)- steht, wobei R₁₃ und R₁₄ gleich oder verschieden sind und für Wasserstoff, ein Halogenatom, einen C₁-C₄-Alkylrest oder einen C₁-C₄-Alkoxyrest stehen,
X für -O- oder -S- steht,
stehen,
wobei diese Benzofuran- oder Benzothiophenderivate in Form von einzelnen optischen Isomeren oder Gemischen davon vorliegen.

2. Benzofuran- oder Benzothiophenderivate nach Anspruch 1, **dadurch gekennzeichnet, daß** A für eine lineare oder verzweigte C₁-C₅-Alkylengruppe, die gegebenenfalls durch eine Hydroxylgruppe substituiert ist, steht.

3. Benzofuran- oder Benzothiophenderivate nach Anspruch 1 oder 2, **dadurch gekennzeichnet, daß** R für eine Isopropoxycarbonylgruppe steht.

4. Benzofuran- oder Benzothiophenderivate nach einem der vorhergehenden Ansprüche, **dadurch gekennzeichnet, daß** R₁ und/oder R₂ und/oder R₃ für eine n-Butylgruppe stehen.

5. Benzofuran- oder Benzothiophenderivate nach einem der vorhergehenden Ansprüche, **dadurch gekennzeichnet, daß** X für -O- steht.

6. Benzofuran- oder Benzothiophenderivate nach einem der vorhergehenden Ansprüche, worin dann, wenn R₃₀ und R₃₁ zusammengenommen mit dem Kohlenstoff, an den sie gebunden sind, für eine Carbonylguppe stehen, 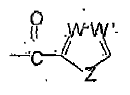 für eine Benzoylgruppe steht.

7. Benzofuran- oder Benzothiophenderivate nach einem der vorhergehenden Ansprüche, **dadurch gekennzeichnet, daß** R₁ für eine n-Butylgruppe steht, A für eine Propylengruppe steht und R₂ und R₃ gleich sind und für eine n-Butylgruppe stehen.

8. 2-Butyl-3-[4-[3-(dibutylamino)propyl]benzoyl]-1-benzofuran-5-carbonsäureisopropylester und pharmazeutisch unbedenkliche Salze davon.

9. 2-Butyl-6-methyl-3-[4-[3-(dibutylamino)propyl]-benzoyl]-1-benzofuran-5-carbonsäurezsopropylestar und pharmazeutisch unbedenkliche Salze davon.

10. Benzofuran- oder Benzothiophenderivate nach einem der vorhergehenden Ansprüche, worin das pharmazeutisch unbedenkliche Salz unter Maleat-, Fumarat-, Methansulfonat-, Benzoat-, Ascorbat-, Pamoat-, Succinat-, Hexamat-, Bismethylensalicylat-, Acetat-, Propionat-, Tartrat-, Salicylat-, Citrat-, Gluconat-, Lactat-, Malat-, Cinnamat-, Mandelat-, Citraconat-, Aspartat-, Palmitat-, Stearat-, Itaconat-, Glykolat-, p-Aminobenzoat-, Glutamat-, Benzolsulfonat-, p-Toluolsulfonat- und Theophyllinacetatsalzen sowie Aminosäuresalzen, wie einem Lysin- oder Histidinsalz, ausgewählt ist.

11. 2-Butyl-3-[4-[3-(dibutylamlno)propyl]benzoyl]-1-benzofuran-5-carbonsäureisopropylester-fumarat.

12. Benzofuran- oder Benzothiophenderivate nach einem der Ansprüche 1 bis 9, worin das pharmazeutisch unbedenkliche Salz unter Hydrochlorid-, Hydrobromid-, Sulfat-, Sulfamat-, Phosphat- und Nitratsalzen ausgewählt ist.

13. 2-Butyl-3-[4-[3-(dibutylamino)propyl]benzoyl]-1-benzofuran-5-carbonsäureisopropylester-sulfat.

14. Verfahren zur Herstellung von Benzofuran- oder Benzothiophenderivaten nach Anspruch 1, worin R für eine Cyanogruppe, eine Formylgruppe, eine Gruppe (a), eine Gruppe (d), eine Gruppe (e) oder eine Gruppe (f) steht, **dadurch gekennzeichnet, daß** man eine Verbindung der allgemeinen Formel: 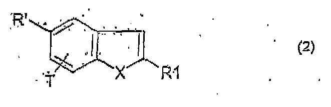 worin R' für eine Cyanogruppe, eine Formylgruppe, eine Gruppe (a), eine Gruppe (d), eine Gruppe (e) oder eine Gruppe (f) steht und R₁, T und X die gleiche Bedeutung wie in Anspruch 1 besitzen, mit einem Halogenid der allgemeinen Formel: 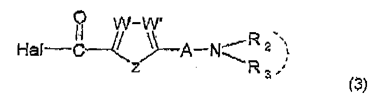 worin A, R₂, R₃, W, W' und Z die gleiche Bedeutung wie in Anspruch 1 besitzen und Hal für ein Halogenatom steht, umsetzt, wobei man die Umsetzung in Gegenwart einer Lewis-Säure durchführt, wodurch man die gewünschten Verbindungen in Form der freien Base erhält, welche man gegebenenfalls zur Bildung eines pharmazeutisch unbedenklichen Salzes mit einer organischen oder anorganischen Säure umsetzen kann.

15. Verfahren zur Herstellung von Benzofuran- oder Benzothiophenderivaten nach Anspruch 1, worin R für eine Gruppe (b) steht, **dadurch gekennzeichnet, daß** man eine Verbindung der allgemeinen Formel: 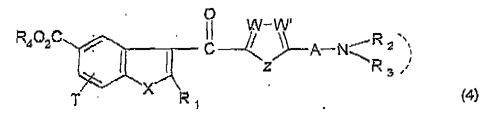 worin A, R₁, R₂, R₃, R₄, T, W, W', X und Z die gleiche Bedeutung wie in Anspruch 1 besitzen, in Gegenwart eines Alkalimetallhydroxids verseift, wodurch man die gewünschten Verbindungen der Formel (1), worin R₅ für ein Alkalimetallatom steht, in Form der freien Base erhält, welche man gegebenenfalls mit einer starken Säure behandelt, wodurch man die gewünschten Verbindungen der Formel (1), worin R₅ für Wasserstoff steht, in Form der freien Base erhält, wobei man die so gebildete freie Base gegebenenfalls zur Bildung eines pharmazeutisch unbedenklichen Salzes mit einer geeigneten organischen oder anorganischen Säure behandeln kann.

16. Verfahren zur Herstellung von Benzofuran- oder Benzothiophenderivaten nach Anspruch 1, worin R für eine Hydroxymethylgruppe steht, **dadurch gekennzeichnet, daß** man ein Ketal der allgemeinen Formel: 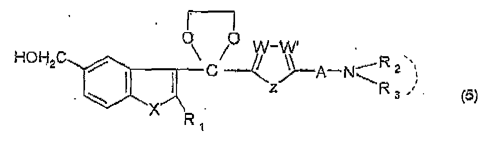 worin A, R₁, R₂, R₃, T, X, W, W' und Z die gleiche Bedeutung wie in Anspruch 1 besitzen, mit Pyridinp-toluolsulfonat entschützt, wodurch man die gewünschten Verbindungen in Form der freien Base erhält, welche man gegebenenfalls zur Bildung eines pharmazeutisch unbedenklichen Salzes mit einer organischen oder anorganischen Säure behandeln kann.

17. Verfahren zur Herstellung von Benzofuran- oder Benzothiophendsrivaten nach Anspruch 1, worin R für eine Cyanogruppe, eine Formylgruppe, eine Gruppe (a), eine Gruppe (d), eine Gruppe (e) oder eine Gruppe (f) steht und R₂ und R₃ gleich sind und jeweils für Wasserstoff stehen, **dadurch gekennzeichnet, daß** main ein Azid der allgemeinen Formel: 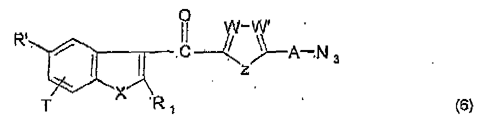 worin R' für eine Cyanogruppe, eine Formylgruppe, eine Gruppe (a), eine Gruppe (d), eine Gruppe (e) oder eine Gruppe (f) steht und A, R₁, T, X, W, W' und Z die gleiche Bedeutung wie in Anspruch 1 besitzen, mit Triphenylphosphin behandelt, wodurch man die gewünschten Verbindungen in Form der freien Base erhält, welche man gegebenenfalls zur Bildung eines pharmazeutisch unbedenklichen Salzes mit einer organischen oder anorganischen Säure umsetzen kann.

18. Verfahren zur Herstellung von Benzofuran- oder Benzothiophenderivaten nach Anspruch 1, worin R für eine Cyanogruppe, eine Formylgruppe, eine Gruppe (a), eine Gruppe (d), eine Gruppe (e) oder eine Gruppe (f) steht und A für eine lineare oder verzweigte C₃-C₅-Alkylengruppe oder eine Gruppe (h) steht, **dadurch gekennzeichnet, daß** man eine Ketonverbindung der allgemeinen Formel: 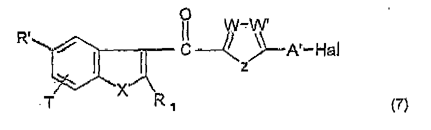 worin R' für eine Cyanogruppe, eine Formylgruppe, eine Gruppe (a), eine Gruppe (d), eine Gruppe (e) oder eine Gruppe (f) steht, A' für eine lineare oder verzweigte C₃-C₅-Alkylengruppe steht, Hal für ein Halogenatom steht und R₁, T, W, W', X und Z die gleiche Bedeutung wie in Anspruch 1 besitzen, mit einer Verbindung der allgemeinen Formel: 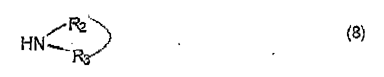 worin R₂ und R₃ die gleiche Bedeutung wie in Anspruch 1 besitzen, umsetzt, wobei man die Umsetzung in Gegenwart eines basischen Agens oder eines Überschusses der Verbindung der Formel (8) durchführt, wodurch man die gewünschten Verbindungen in Form der freien Base erhält, welche man gegebenenfalls zur Bildung eines pharmazeutisch unbedenklichen Salzes mit einer organischen oder anorganischen Säure umsetzen kann.

19. Verfahren zur Herstellung von Benzofuran- oder Benzothiophenderivaten nach Anspruch 1, worin R für eine Cyanogruppe, eine Formylgruppe, eine Gruppe (a), eine Gruppe (d), eine Gruppe (e) oder eine Gruppe (f) steht, R₂ für Wasserstoff oder eine lineare oder verzweigte C₁-C₆-Alkylgruppe steht und R₃ für eine lineare oder verzweigte C₁-C₆-Alkylgruppe steht, **dadurch gekennzeichnet, daß** man ein Amin der allgemeinen Formel: 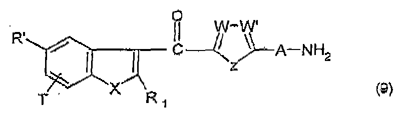 worin R' für eine Cyanogruppe, eine Formylgruppe, eine Gruppe (a), eine Gruppe (d), eine Gruppe (e) oder eine Gruppe (f) steht und A, R₁, T, X, w, W' und Z die gleiche Bedeutung wie in Anspruch 1 besitzen, in Gegenwart von Natriumtriacetoxyborhydrid mit einem Aldehyd der allgemeinen Formel: 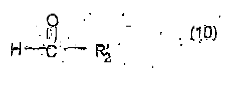 worin R'₂ für Wasserstoff oder einen C₁-C₅-Alkylrest steht, umsetzt, wodurch man die gewünschten Verbindungen in Form der freien Base erhält, welche man gegebenenfalls zur Bildung eines pharmazeutisch unbedenklichen Salzes mit einer organischen oder anorganischen Säure umsetzen kann.

20. Verfahren zur Herstellung von Benzofuran- oder Benzothiophenderivaten nach Anspruch 1, worin R für eine Gruppe (c) steht, **dadurch gekennzeichnet, daß** man eine Verbindung der allgemeinen Formel: 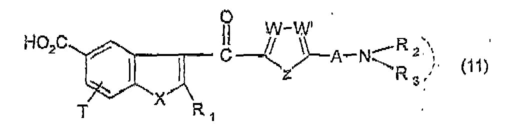 worin A, R₁, R₂, R₃, T, W, W', X und Z die gleiche Bedeutung wie in Anspruch 1 besitzen, nach Schützung der Aminfunktion, wenn R₂ und/oder R₃ für Wasserstoff stehen, mit einem Halogenierungsmittel zu einem Acylhalogenid umsetzt, welches man dann mit einer Verbindung der allgemeinen Formel: 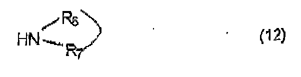 worin R₆ und R₇ die gleiche Bedeutung wie in Anspruch 1 besitzen, behandelt, wonach man gegebenenfalls die gebildete Verbindung entschützt, wodurch man die gewünschten Verbindungen in Form der freien Base erhält, wobei man die so gebildete freie Base gegebenenfalls zur Herstellung eines pharmazeutisch unbedenklichen Salzes mit einer geeigneten organischen oder anorganischen Säure behandeln kann.

21. Verfahren zur Herstellung von Benzofuran- oder Benzothiophenderivaten nach Anspruch 1, worin R für eine Gruppe (a) steht, **dadurch gekennzeichnet, daß** man eine Verbindung der allgemeinen Formel: 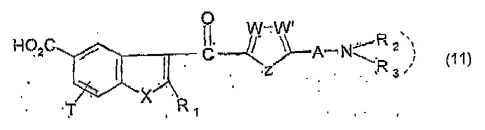 worin A, R₁, R₂, R₃, T, W, W', X und Z die gleiche Bedeutung wie in Anspruch 1 besitzen, nach Schützung der Aminfunktion, wenn R₂ und/oder R₃ für Wasserstoff stehen, mit einem Halogenierungsmittel zu einem Acylhalogenid umsetzt, welches man dann mit einem Alkohol der allgemeinen Formel:
R₄-OH (13)
worin R₄ die gleiche Bedeutung wie in Anspruch 1 besitzt, behandelt, wonach man gegebenenfalls die gebildete Verbindung entschützt, wodurch man die gewünschten Verbindungen in Form der freien Base erhält, wobei man die so gebildete freie Base gegebenenfalls zur Herstellung eines pharmazeutisch unbedenklichen Salzes mit einer geeigneten organischen oder anorganischen Säure behandeln kann.

22. Verfahren zur Herstellung von Benzofuran- oder Benzothiophenderivaten nach Anspruch 1, worin R für eine Gruppe (c), worin R₆ und R₇ jeweils für Wasserstoff stehen, steht, **dadurch gekennzeichnet, daß** man eine Verbindung der allgemeinen Formel: 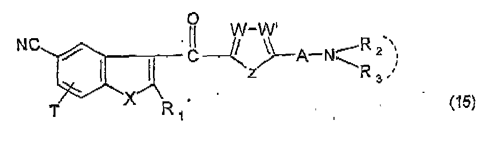 worin A, R₁, R₂, R₃, T, W, W', X und Z die gleiche Bedeutung wie in Anspruch 1 besitzen, in Gegenwart einer starken Säure hydrolysiert, wodurch man die gewünschten Verbindungen in Form der freien Base erhält, wobei man die so gebildete freie Base gegebenenfalls zur Herstellung eines pharmazeutisch unbedenklichen Salzes mit einer geeigneten organischen oder anorganischen Säure behandeln kann.

23. Verfahren zur Herstellung von Benzofuran- oder Benzothiophenderivaten nach Anspruch 1, worin R für eine Gruppe (f) vom Typ primäres Dialkylaminoalkyl steht, **dadurch gekennzeichnet, daß** man eine Verbindung der allgemeinen Formel: 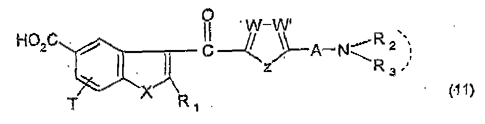 worin A, R₁, R₂, R₃, T, W, W', X und Z die gleiche Bedeutung wie in Anspruch 1 besitzen, nach schützung der Aminfunktion, wenn R₂ und/oder R₃ für Wasserstoff stehen, mit einem Alkohol der allgemeinen Formel: 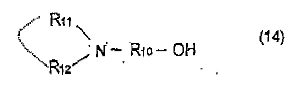 worin R₁₁ und R₁₂ die gleiche Bedeutung wie in Anspruch 1 besitzen und R₁₀ für eine lineare C₁-C₄-Alkylengruppe steht, umsetzt, wobei man die Umsetzung in Gegenwart von Carbonyldiimidazol und 1,8-Diazabicyclo[5.4.0]undec-7-en durchführt, wonach man gegebenenfalls die gebildete Verbindung entschützt, wodurch man die gewünschten Verbindungen in Form der freien Base erhält, welche man gegebenenfalls zur Bildung eines pharmazeutisch unbedenklichen Salzes mit einer organischen oder anorganischen Säure behandeln kann.

24. Verfahren zur Herstellung von Benzofuran- oder Hanzothiophenderivaten nach Anspruch 1, worin R für eine Gruppe (f) vom Typ sekundäres oder tertiäres Dialkylaminoalkyl steht, **dadurch gekennzeichnet, daß** man eine Verbindung der allgemeinen Formel: 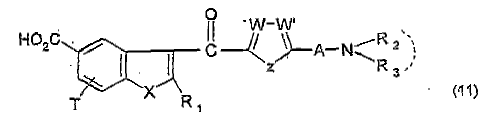 worin A, R₁, R₂, R₃, T, W, W', X und Z die gleiche Bedeutung wie in Anspruch 1 besitzen, nach Schützung der Aminfunktion, wenn R₂ und/oder R₃ für Wasserstoff stehen, mit einem Halogenierungsmittel zu einem Acylhalogenid umsetzt, welches man dann mit einem Alkohol der allgemeinen Formel: 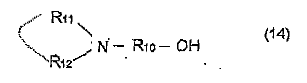 worin R₁₁ und R₁₂ die gleiche Bedeutung wie in Anspruch 1 besitzen und R₁₀ für eine sekundäre oder tertiäre C₂-C₄-Alkylengruppe steht, behandelt und danach gegebenenfalls die gebildete Verbindung entschützt, wodurch man die Verbindungen der Formel (1) in Hydrohalogenidform oder in Form der freien Base erhält, wobei man das Hydrohalogenid gegebenenfalls mit einem basischen Agens behandeln kann, wodurch man die gewünschten Verbindungen in Form der freien Base erhält, welche man gegebenenfalls zur Bildung eines pharmazeutisch unbedenklichen Salzes mit einer organischen oder anorganischen Säure behandeln kann.

25. Verfahren zur Herstellung von Benzofuran- oder Benzothiophenderivaten nach Anspruch 1, worin R für eine Tetrazolylgruppe steht, **dadurch gekennzeichnet, daß** man eine Verbindung der allgemeinen Formel: 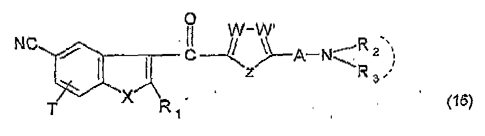 worin A, R₁, R₂, R₃, T, W, W, X und Z die gleiche Bedeutung wie in Anspruch 1 besitzen, mit einem (Tri-C₁-C₄-alkyl)azidozinn umsetzt, wodurch man die gewünschten Verbindungen in Form der freien Base erhält, wobei man die so gebildete freie Base gegebenenfalls zur Herstellung eines phaxmazeutisch unbedenklichen Salzes mit einer geeigneten organischen oder anorganischen Säure behandeln kann.

26. Verfahren zur Herstellung von Benzofuran- oder Benzothiophenderivaten nach Anspruch 1, worin R für eine Cyanogruppe, eine Formylgruppe, eine Gruppe (a), eine Gruppe (d), eine Gruppe (e) oder eine Gruppe (f) steht und R₂ und R₃ verschieden sind und jeweils für eine lineare oder verzweigte C₁-C₆-Alkylgruppe oder eine C₃-C₆-Cycloalkylgruppe stehen, durch Umsetzung einer Verbindung der allgemeinen Formel: 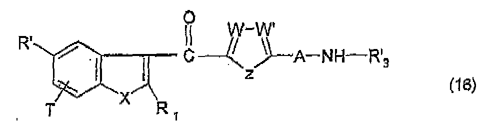 worin A, R₁, R₂, T, W, W', X und Z die gleiche Bedeutung wie in Anspruch 1 besitzen und R' für eine Cyanogruppe, eine Formylgruppe, eine Gruppe (a), eine Gruppe (d), eine Gruppe (e) oder eine Gruppe (f) steht und R'₃ für eine lineare oder verzweigte C₁-C₆-Alkylgruppe oder eine C₃-C₆-Cycloalkylgruppe steht, mit einem Halogenid der allgemeinen Formel;
Hal-R"₂ (17)
worin Hal für ein Halogenatom steht und R"₂ für eine lineare oder verzweigte C₁-C₆-Alkylgruppe oder eine C₃-C₆-Cycloalkylgruppe steht, wobei man die Umsetzung in Gegenwart eines basischen Agens durchführt, wodurch man die gewünschten Verbindungen in Form der freien Base erhält, wobei man die so gebildete freie Base gegebenenfalls zur Herstellung eines pharmazeutisch unbedenklichen Salzes mit einer geeigneten organischen oder anorganischen Säure behandeln kann.

27. Verfahren zur Herstellung von Benzofuran- oder Benzothiophenderivaten nach Anspruch 14, 15, 17 oder 18, **dadurch gekennzeichnet, daß** man die Schützung der Aminfunktion mit Chlorameisensäure-9-fluorenylmethylester und die Entschützung durch Behandlung mit einem sekundären Amin durchführt.

28. Arzneimittel, **dadurch gekennzeichnet, daß** es ein Benzofuran- oder Benzothiophenderivat oder ein pharmazeutisch unbedenkliches Salz davon gemäß einem der Ansprüche 1 bis 13 umfaßt.

29. Pharmazeutische oder veterinärmedizinische Zusammensetzung, **dadurch gekennzeichnet, daß** sie als Wirkstoff mindestens ein Benzofuran- oder Benzothiophenderivat oder ein pharmazeutisch unbedenkliches Salz davon gemäß einem der Ansprüche 1 bis 13 in Kombination mit einem pharmazeutischen Vehikel oder einem geeigneten Hilfsstoff enthält,

30. Pharmazeutische oder veterinärmedizinische Zusammensetzung nach Anspruch 29 zur Behandlung von pathologischen Syndromen des Herz-Kreislauf-Systems.

31. Pharmazeutische oder veterinärmedizinische Zusammensetzung nach Anspruch 29 oder 30 zur Behandlung von Angina pectoris, Hypertonie, atrialer, ventrikulärer oder supraventrikulärer Arrhythmie, Hirnkreislaufinsuffizienz, Herzinsuffizienz und Myokardinfarkt mit oder ohne Komplikation durch Herzinsuffizienz oder zur Prävention von Postinfarkt-Mortalität.

32. Pharmazeutische oder veterinärmedizinische Zusammensetzung nach einem der Ansprüche 29 bis 31, **dadurch gekennzeichnet, daß** sie 50 bis 500 mg Wirkstoff enthält.

33. Verwendung mindestens eines Benzofuran- oder Benzothiophenderivats oder eines pharmazeutisch unbedenklichen Salzes davon gemäß einem der Ansprüche 1 bis 13 zur Herstellung eines Arzneimittels zur Behandlung von pathologischen Syndromen des Herz-Kreislauf-Systems.
